# EUROPEAN PATENT APPLICATION

(11) **EP 4 599 890 A2**
(43) Date of publication of application: **13.08.2025**
(21) Application number: 25185652.2
(22) Date of filing: 13.01.2016
(51) Int. Cl.: A61P 25/02

(54) **SUNITINIB FOR PREVENTING AND/OR TREATING AMYOTROPHIC LATERAL SCLEROSIS**

(30) Priority: 13.01.2015 JP 2015004589; 09.07.2015 JP 2015137909
(62) Divisional of application: 20201324.9
(71) Applicant: Kyoto University, Kyoto-shi, Kyoto 606-8501 (JP)
(72) Inventor: INOUE, Haruhisa, Kyoto, 606-8501 (JP); IMAMURA, Keiko, Kyoto, 606-8501 (JP)
(74) Representative: J A Kemp LLP

(57) **Abstract**

The present invention provides a compound for use in the prophylaxis and/or treatment of amyotrophic lateral sclerosis (ALS), wherein the compound is bosutinib and wherein the ALS is sporadic ALS, ALS having SOD1 mutation, ALS having TDP-43 mutation and/or ALS having C9orf72 mutation.

## Description

### [Technical Field]

The present invention relates to a prophylactic and/or therapeutic agent for amyotrophic lateral sclerosis.

### [Background Art]

Amyotrophic lateral sclerosis (hereinafter, ALS) is a motor neuron disease of poor prognosis, which develops at middle ages and thereafter and causes progressive paralysis of skeletal muscles. It is designated as a disease in the project for investigation and research into specific diseases sponsored by the Ministry of Health, Labor and Welfare of Japan. More than about 90% of cases of ALS are sporadic and the cause is unknown. As a component of ubiquitin-positive inclusion bodies found in the lower motor neuron of sporadic ALS patients, a 43-kDa TAR DNA-binding protein (TDP-43) has been identified in recent years, and is attracting attention as an etiology gene (non-patent document 1). On the other hand, the remaining 10% are familial cases, and point mutation of genes such as Cu/Zn superoxide dismutase (SOD1) gene, TDP-43 gene and the like has been reported. In this case, the gain-of-toxic function theory is likely wherein motor neuron death is caused by the cytotoxicity newly gained by mutated SOD1 (non-patent document 2) .

The only currently commercially available therapeutic drug for ALS is riluzole (Rilutek^{™}, Aventis), a glutamate receptor antagonist possessing glutamate suppressing action (patent document 1).

On the other hand, it is considered that the pathology can be reproduced in vitro by establishing an induced pluripotent stem cell (iPS cell) obtained from cells derived from a patient by using a reprogramming technique and inducing differentiation from this iPS cell into a pathogenic cell. The present inventors have demonstrated using atorvastatin known to have an anti-ALS action that a candidate substance of a therapeutic drug for ALS can be screened for by inducing differentiation of an iPS cell established from fibroblasts derived from ALS patients having a mutation in the SOD1 gene into astrocyte, and using, as an index, a decrease in the expression level of SOD1 in the obtained astrocyte (patent document 2). Furthermore, they have identified sorafenib, which is a multikinase inhibitor and used as a therapeutic drug for progressive renal cell carsinoma, as a candidate substance of a therapeutic drug for ALS, by using the screening system (patent document 3). Also, they have shown that a candidate substance of a therapeutic drug for ALS can be screened for by inducing differentiation of iPS cell, established from ALS patients having a mutation in TDP-43 gene, into motor neuron (MN), and screening using a decrease in the expression level of TDP-43 in the obtained motor neuron, improvement of fragility to stress, recovery of neurite length and the like as indices (patent document 4, non-patent document 3). Furthermore, they have shown that a motor neuron that reproduces pathology of patients well can be promptly and synchronically prepared by introducing 3 kinds of nerve cell lineage specific transcription factors into pluripotent stem cells (patent document 5).

Thus, while a screening and efficacy evaluation system of a therapeutic drug for ALS using nerve cell derived from iPS cell is being developed, and a promising candidate substance (therapeutic drug seeds) is being found, a considerable amount of way is still expected until practicalization as a pharmaceutical product.

Incidentally, as a method for overcoming the deadlock seen recently in the development and research of a new drug a new research concept called drug repositioning (DR) has become the subject of discussion. It aims to find a new medicinal effect from existing drugs showing safety and pharmacokinetics in human, which have already been confirmed from actual results, and achieve practicalization thereof. Since many existing data can be used, it is further advantageous in that the development cost can be suppressed low, accumulated know-how and materials (surrounding compounds and the like) exist and the like. As mentioned above, the present inventors have already found that sorafenib, which is used as a therapeutic drug for cancer, has an ALS treatment activity as novel efficacy.

### [Document List]

### [Patent documents]

patent document 1: AU 666150 B2
patent document 2: WO 2011/074690
patent document 3: WO 2012/029994
patent document 4: WO 2013/108926
patent document 5: WO 2014/148646

### [non-patent documents]

non-patent document 1: Neumann M, et al., Science. 2006, 314: 130-3.
non-patent document 2: Bruijn, L.I., et al., 2004. Annu. Rev. Neurosci. 27, 723-749.
non-patent document 3: Egawa N, et al., Sci. Transl. Med. 2012, 4: 145ra104

### [SUMMARY OF THE INVENTION]

### [Problems to be Solved by the Invention]

An object of the present invention is to provide a novel medicament candidate having a prophylactic and/or therapeutic activity for ALS, as well as accelerate the development of a prophylactic and/or therapeutic agent for ALS as a realistic pharmaceutical product.

### [Means of Solving the Problems]

In an attempt to solve the above-mentioned problem, the present inventors induced differentiation of iPS cell established from ALS patients having a mutation in SOD1 gene into motor neuron and, using the survival of the motor neuron as an index, screened for a compound having an anti-ALS activity from a library of known compounds including medicaments already on the market as pharmaceutical products. As a result, they have found that epithelial cell growth factor receptor (EGFR) inhibitor, fibroblast growth factor receptor (FGFR) inhibitor, Aurorakinase inhibitor, protein kinase A (PKA) inhibitor, protein kinase C (PKC) inhibitor, MEK inhibitor, Met inhibitor, JNK inhibitor, Syk inhibitor, JAK inhibitor, prostaglandin analogue, 3-hydroxybutyric acid and estrogen receptor antagonist remarkably improve survival of ALS motor neuron. In addition, compounds having an anti-ALS activity including Bosutinib, which is a drug in the market, were also found even from the compounds not belonging to the above-mentioned category.

Furthermore, 6 kinds of drug in the market (including Tivozanib which was not approved after filing request for approval and examination thereof was completed) confirmed to have an anti-ALS activity against mutated SOD1 ALS were subjected to comparison of the anti-ALS effects thereof by using ALS motor neurons derived from iPS cells established from patients having SOD1 mutation, patients having TDP-43 mutation, patients having C9orf72 mutation, and sporadic ALS patients, and improvement in the cell survival rate as an index. As a result, they showed varying anti-ALS activity spectra depending on the medicaments.

From the above results, the present inventors have found that any of the above-mentioned kinase inhibitor or other medicament is useful for the prophylaxis and/or treatment of ALS, and further that effective prophylaxis and/or treatment of ALS, which is suitable for individual patients or a preliminary group thereof, can be available by properly according to the causative gene of ALS, which resulted in the completion of the present invention.

Therefore, the present invention provides the following.
[1] A prophylactic and/or therapeutic agent for amyotrophic lateral sclerosis (ALS), which comprises one or more kinase inhibitors selected from the group consisting of an epithelial cell growth factor receptor (EGFR) inhibitor, a fibroblast growth factor receptor (FGFR) inhibitor, an Aurorakinase inhibitor, a protein kinase A (PKA) inhibitor, a protein kinase C (PKC) inhibitor, an MEK inhibitor, an Met inhibitor, a JNK inhibitor, a Syk inhibitor and a JAK inhibitor, and/or a prostaglandin analogue and/or an estrogen receptor antagonist.
[2] The agent of [1], wherein the aforementioned EGFR inhibitor is a PDGF Receptor Tyrosine Kinase Inhibitor III or BPIQ-I.
[3] The agent of [1], wherein the aforementioned FGFR inhibitor is Pazopanib or an analog thereof or PDGF Receptor Tyrosine Kinase Inhibitor III.
[4] The agent of [1], wherein the aforementioned Aurorakinase inhibitor is ZM-447439, VX-680, Aurora Kinase Inhibitor II, CYC116, KW2449, or AT9283.
[5] The agent of [1], wherein the aforementioned PKA inhibitor is PDGF Receptor Tyrosine Kinase Inhibitor III.
[6] The agent of [1], wherein the aforementioned PKC inhibitor is PDGF Receptor Tyrosine Kinase inhibitor III or Enzastaurin.
[7] The agent of [1], wherein the aforementioned MEK inhibitor is U0126-EtOH.
[8] The agent of [1], wherein the aforementioned Met inhibitor is MGCD-265, PF-2341066 (Crizotinib) or an analog thereof or BMS 777607.
[9] The agent of [1], wherein the aforementioned JNK inhibitor is SP600125.
[10] The agent of [1], wherein the aforementioned Syk inhibitor is a Syk Inhibitor.
[11] The agent of [1], wherein the aforementioned JAK inhibitor is JAK Inhibitor I or AT9283.
[12] The agent of [1], wherein the aforementioned prostaglandin analogue is Bimatoprost.
[13] The agent of [1], wherein the aforementioned estrogen receptor antagonist is Raloxifene.
[14] A prophylactic and/or therapeutic agent for ALS, comprising one or more compounds selected from the group consisting of Tivozanib and an analog thereof, SB 216763, Cdk2 Inhibitor II, BUDESONIDE, RIBOFLAVIN, alpha-TOCHOPHEROL, AMODIAQUINE, SU9516, Sunitinib and an analog thereof, GSK-3 Inhibitor XIII, Bisindolylmaleimide I, HYDROQUINONE, FLUNISOLIDE, MGCD-265, Indirubin-3'-monoxime, HYDRASTINE (1R, 9S), PIPERINE, BUTAMBEN, Axitinib and an analog thereof, APOMORPHINE, FENBUFEN, Bosutinib (SKI-606) and an analog thereof, Wee1 Inhibitor, Cdk2 Inhibitor IV, NU6140, 3-hydroxybutyric acid, Imatinib, Nilotinib, Rebastinib, and Bafetinib.
[15] A method for the prophylaxis and/or treatment of ALS, comprising administering an effective amount of one or more kinase inhibitors selected from the group consisting of an EGFR inhibitor, an FGFR inhibitor, an Aurorakinase inhibitor, a PKA inhibitor, a PKC inhibitor, an MEK inhibitor, an Met inhibitor, a JNK inhibitor, a Syk inhibitor and a JAK inhibitor, and/or a prostaglandin analogue and/or an estrogen receptor antagonist.
[16] A method for the prophylaxis and/or treatment of ALS, comprising administering an effective amount of one or more kinase inhibitors selected from the group consisting of Tivozanib and an analog thereof, SB 216763, Cdk2 Inhibitor II, BUDESONIDE, RIBOFLAVIN, alpha-TOCHOPHEROL, AMODIAQUINE, SU9516, Sunitinib and an analog thereof, GSK-3 Inhibitor XIII, Bisindolylmaleimide I, HYDROQUINONE, FLUNISOLIDE, MGCD-265, Indirubin-3'-monoxime, HYDRASTINE (1R,9S), PIPERINE, BUTAMBEN, Axitinib and an analog thereof, APOMORPHINE, FENBUFEN, Bosutinib (SKI-606) and an analog thereof, a Wee1 Inhibitor, Cdk2 Inhibitor IV, NU6140, 3-hydroxybutyric acid, Imatinib, Nilotinib, Rebastinib, and Bafetinib.
[17] One or more kinase inhibitors selected from the group consisting of an EGFR inhibitor, an FGFR inhibitor, an Aurorakinase inhibitor, a PKA inhibitor, a PKC inhibitor, an MEK inhibitor, an Met inhibitor, a JNK inhibitor, a Syk inhibitor and a JAK inhibitor, and/or a prostaglandin analogue and/or an estrogen receptor for the prophylaxis and/or treatment of ALS.
[18] One or more kinase inhibitors selected from the group consisting of Tivozanib and an analog thereof, SB 216763, Cdk2 Inhibitor II, BUDESONIDE, RIBOFLAVIN, alpha-TOCHOPHEROL, AMODIAQUINE, SU9516, Sunitinib and an analog thereof, GSK-3 Inhibitor XIII, Bisindolylmaleimide I, HYDROQUINONE, FLUNISOLIDE, MGCD-265, Indirubin-3'-monoxime, HYDRASTINE (1R,9S), PIPERINE, BUTAMBEN, Axitinib and an analog thereof, APOMORPHINE, FENBUFEN, Bosutinib (SKI-606) and an analog thereof, a Wee1 Inhibitor, Cdk2 Inhibitor IV, NU6140, 3-hydroxybutyric acid, Imatinib, Nilotinib, Rebastinib, and Bafetinib for the prophylaxis and/or treatment of ALS.

### [Effect of the Invention]

Various kinase inhibitors of the present invention and other compound having an anti-ALS activity are useful for the prophylaxis and/or treatment of ALS. In addition, an effective prophylaxis and/or treatment of ALS, which is suitable for individual patients or a preliminary group thereof, can be available by properly using the kinase inhibitor of the present invention according to the causative gene of ALS.

### [Brief Description of the Drawings]

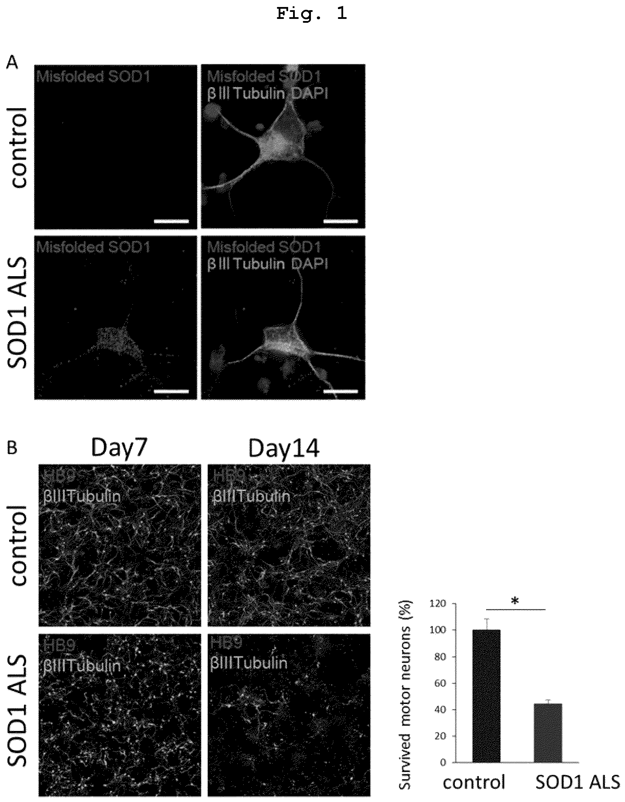

In Fig. 1, Fig. 1A shows immunostained images showing accumulation of misfolded SOD1 in motoneuron cell established iPS cell derived from ALS patients having SOD1 mutation (SOD1-L144FVX mutation) (SOD1 ALS). Control shows motoneuron cell established from iPS cell derived from healthy subject. The right panel shows the results of triple staining with βIII-tubulin and DAPI. Fig. 1B shows immunostained images showing fragility (decrease in the number of surviving cells) of motoneuron cell derived from SOD1 mutation. The right graph shows the survival rate, at 14 days after the start of differentiation induction, of motoneuron cell. *:p<0.05
Fig. 2 is a graph showing that Tivozanib, Bosutinib, Sunitinib, Crizotinib, Axitinib and Pazopanib improve survival rate of motoneuron cells having SOD1 mutation in a dose-dependent manner.
Fig. 3 shows that Tivozanib, Bosutinib and Sunitinib inhibit phosphorylation of Erk and c-abl in motoneuron cell having SOD1 mutation. Fig. 3A shows Western blot images using anti-Erk antibody and anti-phosphorylated Erk antibody, and anti-c-abl antibody and anti-phosphorylated c-abl antibody, Fig. 3B and C each show phosphorylated Erk/Erk (p-Erk/Erk) ratio and phosphorylated c-abl/c-abl (p-Abl/Abl) ratio obtained by image analysis of the band intensity.
Fig. 4 shows the survival rate, at 14 days after the start of induction, of motoneuron cell induced from iPS cell, derived from ALS patients having various gene mutations and sporadic ALS patients.
Fig. 5 shows a dose-dependent protection effect of Tivozanib, Bosutinib, Sunitinib, Crizotinib, Axitinib and Pazopanib on the survival of motoneuron cell induced from iPS cell derived from ALS patients having various gene mutations and sporadic ALS patients. In the Figure, SALS means sporadic ALS. The bar graph of the column of each motoneuron cell shows no addition (0 µM), 0.1 µM addition, 1 µM addition of medicament from the left.
Fig. 6 is a graph showing that Bimatoprost, Edaravone, 3-hydroxybutyric acid and Raloxifene improve survival rate of motoneuron cells having SOD1 mutation in a dose-dependent manner.
Fig. 7 is a drawing showing that Imatinib, Nilotinib, Rebastinib, AT9283 and Bafetinibin improve survival rate of familial ALS (mSOD1 ALS) motoneuron cells in a dose-dependent manner.
Fig. 8 is a drawing showing that Bosutinib decreases misfolded TDP43 even in familial ALS having TDP-43 mutation and sporadic ALS.

### [Description of Embodiments]

The present invention provides a prophylactic and/or therapeutic agent for amyotrophic lateral sclerosis (ALS) (hereinafter to be also referred to as an agent for the prophylaxis or treatment of ALS) containing one or more kinase inhibitors selected from the group consisting of an epithelial cell growth factor receptor (EGFR) inhibitor, a fibroblast growth factor receptor (FGFR) inhibitor, an Aurorakinase inhibitor, a protein kinase A (PKA) inhibitor, a protein kinase C (PKC) inhibitor, an MEK inhibitor, an Met inhibitor, a JNK inhibitor, a Syk inhibitor and a JAK inhibitor, and/or, one or more compounds selected from the group consisting of a prostaglandin analogue, 3-hydroxybutyric acid, an estrogen receptor antagonist, Tivozanib and an analog thereof, SB 216763, Cdk2 Inhibitor II, BUDESONIDE, RIBOFLAVIN, alpha-TOCHOPHEROL, AMODIAQUINE, SU9516, Sunitinib and an analog thereof, GSK-3 Inhibitor XIII, Bisindolylmaleimide I, HYDROQUINONE, FLUNISOLIDE, MGCD-265, Indirubin-3'-monoxime, HYDRASTINE (1R, 9S), PIPERINE, BUTAMBEN, Axitinib and an analog thereof, APOMORPHINE, FENBUFEN, Bosutinib (SKI-606) and an analog thereof, a Wee1 Inhibitor, Cdk2 Inhibitor IV, NU6140, AT9283, Imatinib, Nilotinib, Rebastinib, and Bafetinib.

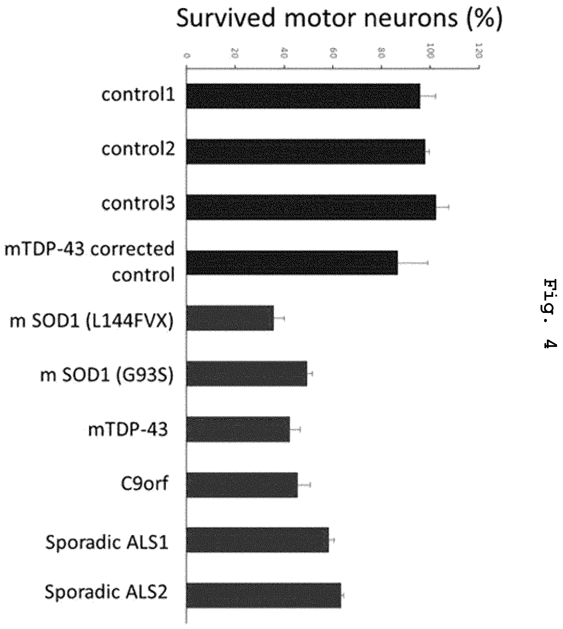

In the present invention, the amyotrophic lateral sclerosis (ALS) to be treated includes both sporadic and familial amyotrophic lateral sclerosis (ALS). In the case of familial ALS, the causative gene is not particularly limited, and may be any known causative gene such as SOD1, TDP-43, C9orf72, alsin, SETX, FUS/TLS, VAPB, ANG, FIG4, OPTN, ATXN2, DAO, UBQLN2, PFN1, DCTN1, CHPM2B, VCP and the like. In one embodiment, in the case of familial ALS having SOD1 mutation, examples of the SOD1 gene mutation include, but are not limited to, a mutation in which the 144th Leu of SOD1 protein is substituted by Phe-Val-Xaa (Xaa is any amino acid) (SOD1-L144FVX), a mutation in which the 93rd Gly is substituted by Ser (SOD1-G93S), a mutation in which the 106th Leu is substituted by Val and the like. In another embodiment, in the case of familial ALS having TDP-43 mutation, examples of the TDP-43 gene mutation include, but are not limited to, a mutation in which the 337th Met of TDP-43 protein is substituted by Val (TDP-43-M337V). In still another embodiment, in the case of familial ALS having C9orf72 mutation, examples of the C9orf72 SOD1 gene mutation include, but are not limited to, (GGGGCC)n repeats of abnormal elongation in intron 1.

An EGFR inhibitor as an active ingredient of the prophylactic or therapeutic agent for ALS in the present invention includes all of naturally-occurring substances derived from microorganism etc., semi-synthetic substances derived therefrom, and fully synthetic compounds. Examples thereof include, but are not limited to, PDGF Receptor Tyrosine Kinase Inhibitor III, BPIQ-I (see Table 4 for the above), gefitinib, erlotinib, afatinib, lapatinib, cetuximab, panitumumab, icotinib/BPI-2009-H, dacomitinib/PF-00299804, AZD8931, AC480/BMS-599626, varlitinib/ARRY-334543, JNJ-26483327, CUDC-101, TAK-285, ARRY-380, AZD4769, HKI-357, S-222611, canertinib/CI-1033/ PD-183805, pelitinib/EKB-569, AV-412, HM61713, HM781-36B, CO-1686, CP-724714, AP26113, PR610, AZD9291, AEE788/NVP-AEE788, epitinib/HMPL-813, theliatinib/HMPL-309, XL647/KD019, poziotinib/HM781-36B, neratinib/PF-05208767/HKI-272, mubritinib/TAK-165, ASP8273 and the like. Preferably, it may be PDGF Receptor Tyrosine Kinase Inhibitor III or BPIQ-I. In another embodiment, the EGFR inhibitor can be an EGFR selective inhibitor. Examples of the EGFR selective inhibitor include BPIQ-I.

An FGFR inhibitor as an active ingredient of the prophylaxis or therapeutic agent for ALS in the present invention includes all of naturally-occurring substance derived from microorganism etc., a semi-synthetic substance derived therefrom, and fully synthetic compounds. Examples thereof include, but are not limited to, Pazopanib or an analog thereof, PDGF Receptor Tyrosine Kinase Inhibitor III (see Table 4 for the above), lenvatinib, AZD4547, NVP-BGJ398, brivanib/BMS-582664, LY2874455, lucitanib/E-3810, CP-547632, masitinib/AB-1010, nintedanib/BIBF1120, sulfatinib/HMPL-012, dovitinib/TKI258/CHIR-258, XL228, orantinib/SU6668/TSU-68, ENMD-2076, S49076, JNJ-42756493, BAY 1163877, Debio1347/CH5183284 and the like. Preferably, it may be Pazopanib or an analog thereof, or PDGF Receptor Tyrosine Kinase Inhibitor III. In another embodiment, the FGFR inhibitor may be an FGFR selective inhibitor.

Here, the "analog of Pazopanib" is, for example, a compound represented by the following formula (I): 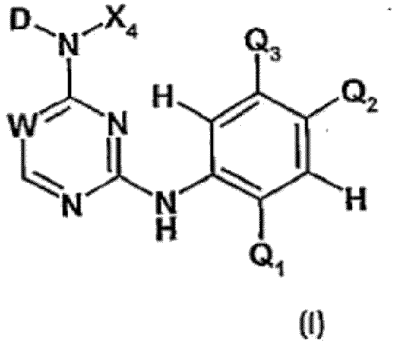 wherein
D is 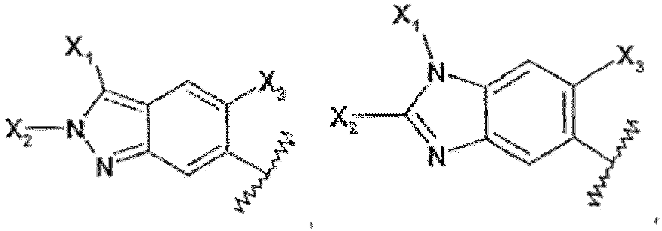 or 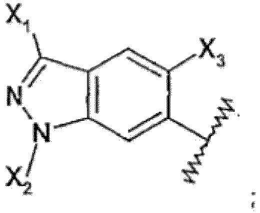
X₁ is hydrogen, C₁-C₄ alkyl, C₁-C₄ haloalkyl, or C₁-C₄ hydroxyalkyl;
X₂ is hydrogen, C₁-C₄ alkyl, C₁-C₄ haloalkyl, C(O)R¹, or aralkyl;
X₃ is hydrogen or halogen;
X₄ is hydrogen, C₁-C₄ alkyl, C₁-C₄ haloalkyl, heteroaralkyl, cyanoalkyl, -(CH₂)ₚC=CH(CH₂)ₜH, -(CH₂)ₚC≡C(CH₂)ₜH, or C₃-C₇ cycloalkyl;
p is 1, 2, or 3;
t is 0 or 1;
W is N or C-R (wherein R is hydrogen, halogen or cyano);
Q₁ is hydrogen, halogen, C₁-C₂ haloalkyl, C₁-C₂ alkyl, C₁-C₂ alkoxy, or C₁-C₂ haloalkoxy;
Q₂ is A¹ or A²;
Q₃ is A¹ when Q₂ is A², and A² when Q₂ is A¹; wherein
A¹ is hydrogen, halogen, C₁-C₃ alkyl, C₁-C₃ haloalkyl, or -OR¹;
A² is a group defined by - (Z)ₘ-(Z¹)-(Z²) (wherein
Z is CH₂, m is 0, 1, 2, or 3, or
Z is NR², m is 0 or 1, or
Z is oxygen, m is 0 or 1, or
Z is CH₂NR², m is 0 or 1;
Z¹ is S(O)₂, S(O), or C(O);
Z² is C₁-C₄ alkyl, NR³R⁴, aryl, arylamino, aralkyl, aralkoxy, or heteroaryl;
R¹ is C₁-C₄ alkyl;
R², R³, and R⁴ are each independently selected from hydrogen, C₁-C₄ alkyl, C₃-C₇ cycloalkyl, -S(O)₂R⁵, and -C(O)R⁵;
R⁵ is C₁-C₄ alkyl, or C₃-C₇ cycloalkyl; and
when Z is an oxygen, Z¹ is S(O)₂); and
when D is 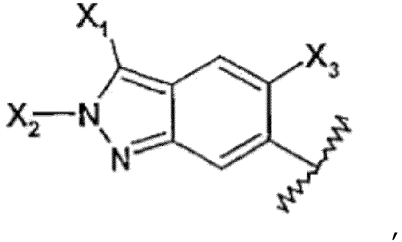
X₂ is C₁-C₄ alkyl, C₁-C₄ haloalkyl, C(O)R¹, or aralkyl.

The terms "C₁-C₄ alkyl", "C₁-C₄ haloalkyl", "C₁-C₄ hydroxyalkyl", "aralkyl", "halogen", "heteroaralkyl", "cyanoalkyl", "C₃-C₇ cycloalkyl", "C₁-C₂ haloalkyl", "C₁-C₂ alkyl", "C₁-C₂ alkoxy", "C₁-C₂ haloalkoxy", "C₁-C₃ alkyl", "C₁-C₃ haloalkyl", "aryl", "arylamino", "aralkoxy", and "heteroaryl" in the above-mentioned formula (I) are as defined in WO 2002/059110 (National Publication of International Patent Application No. 2004-517925).

Specific examples of the analog of Pazopanib include the following.
N²-[5-(ethylsulfonyl)-2-methoxyphenyl]-5-fluoro-N⁴-methyl-N⁴-(3-methyl-1H-indazol-6-yl)-2,4-pyrimidinediamine;
3-({5-fluoro-4-[methyl(3-methyl-1H-indazol-6-yl)amino]-2-pyrimidinyl}amino)-4-methoxy-N-methylbenzenesulfonamide;
5-fluoro-N⁴-methyl-N⁴-(3-methyl-1H-indazol-6-yl)-N²-{3-[(methylsulfonyl)methyl]phenyl}-2,4-pyrimidinediamine;
3-({5-fluoro-4-[methyl(3-methyl-1H-indazol-6-yl)amino]-2-pyrimidinyl}amino)-N-isopropylbenzenesulfonamide;
5-fluoro-N²-[5-(isopropylsulfonyl)-2-methoxyphenyl]-N⁴-methyl-N⁴-(3-methyl-1H-indazol-6-yl)-2,4-pyrimidinediamine;
N-[5-({5-fluoro-4-[methyl(3-methyl-1H-indazol-6-yl)amino]-2-pyrimidinyl}amino)-2-methylphenyl]methanesulfonamide;
5-fluoro-N⁴-methyl-N⁴-(3-methyl-1H-indazol-6-yl)-N²-[4-(methylsulfonyl)phenyl]-2,4-pyrimidinediamine;
N⁴-(3-ethyl-1H-indazol-6-yl)-5-fluoro-N⁴-methyl-N²-{3-[(methylsulfonyl)methyl]phenyl}-2,4-pyrimidinediamine;
4-({5-fluoro-4-[methyl(3-methyl-1H-indazol-6-yl)amino]-2-pyrimidinyl}amino)benzenesulfonamide;
N⁴-ethyl-5-fluoro-N²-[2-methoxy-5-(methylsulfonyl)phenyl]-N⁴-(3-methyl-1H-indazol-6-yl)-2,4-pyrimidinediamine;
[4-({5-fluoro-4-[methyl(3-methyl-1H-indazol-6-yl)amino]-2-pyrimidinyl}amino)phenyl]-N-methylmethanesulfonamide; 5-fluoro-N²-{3-[(isopropylsulfonyl)methyl]phenyl}-N⁴-methyl-N⁴-(3-methyl-1H-indazol-6-yl)-2,4-pyrimidinediamine;
3-({5-fluoro-4-[methyl(3-methyl-1H-indazol-6-yl)amino]-2-pyrimidinyl}amino)-4-methoxybenzamido;
4-({5-fluoro-4-[methyl(3-methyl-1H-indazol-6-yl)amino]-2-pyrimidinyl}amino)-3-methoxybenzenesulfonamide;
N²-(3-methyl-1H-indazol-6-yl)-N⁴-{3-[(methylsulfonyl)methyl]phenyl}-1,3,5-triazine-2,4-diaminetrifluoroacetic acid;
N²-methyl-N²-(3-methyl-1H-indazol-6-yl)-N⁴-{3-[(methylsulfonyl)methyl]phenyl}-1,3,5-triazine-2,4-diamine;
N²-[5-(ethylsulfonyl)-2-methoxyphenyl]-N⁴-methyl-N⁴-(3-methyl-1H-indazol-6-yl)-1,3,5-triazine-2,4-diamine;
N-[2-methyl-5-({4-[methyl(3-methyl-1H-indazol-6-yl)amino]-1,3,5-triazin-2-yl}amino)phenyl]methanesulfonamide;
N²-methyl-N²-(3-methyl-1H-indazol-6-yl)-N⁴-[3-(methylsulfonyl)phenyl]-1,3,5-triazine-2,4-diamine;
N-[4-({4-[methyl(3-methyl-1H-indazol-6-yl)amino]-1,3,5-triazin-2-yl}amino)phenyl]acetamide;
3-({4-[methyl(3-methyl-1H-indazol-6-yl)amino]-2-pyrimidinyl}amino)benzenesulfonamide;
N²-[5-(ethylsulfonyl)-2-methoxyphenyl]-N⁴-methyl-N⁴-(3-methyl-1H-indazol-6-yl)-2,4-pyrimidinediamine;
N⁴-methyl-N⁴-(3-methyl-1H-indazol-6-yl)-N²-{3-[(methylsulfonyl)methyl]phenyl}-2,4-pyrimidinediamine;
N-isopropyl-3-({4-[methyl(3-methyl-1H-indazol-6-yl)amino]-2-pyrimidinyl}amino)benzenesulfonamide;
N-cyclopropyl-3-({4-[methyl(3-methyl-1H-indazol-6-yl)amino]-2-pyrimidinyl}amino)benzenesulfonamide;
N⁴-ethyl-N²-[5-(ethylsulfonyl)-2-methoxyphenyl]-N⁴-(3-methyl-1H-indazol-6-yl)-2,4-pyrimidinediamine;
N-[3-({4-[methyl(3-methyl-1H-indazol-6-yl)amino]-2-pyrimidinyl}amino)phenyl]methanesulfonamide;
N²-{3-[(isopropylsulfonyl)methyl]phenyl}-N⁴-methyl-N⁴-(3-methyl-1H-indazol-6-yl)-2,4-pyrimidinediamine;
N²-{4-[(isopropylsulfonyl)methyl]phenyl}-N⁴-methyl-N⁴-(3-methyl-1H-indazol-6-yl)-2,4-pyrimidinediamine;
N²-[5-(isobutylsulfonyl)-2-methoxyphenyl]-N⁴-(3-methyl-1H-indazol-6-yl)-2,4-pyrimidinediamine;
N-[3-({4-[methyl(3-methyl-1H-indazol-6-yl)amino]-2-pyrimidinyl}amino)phenyl]acetamide;
N-[3-({4-[ethyl(3-methyl-1H-indazol-6-yl)amino]-2-pyrimidinyl}amino)phenyl]acetamide;
N²-(2-methoxy-5-{[(5-methyl-3-isoxazolyl)methyl]sulfonyl}phenyl)-N⁴-(3-methyl-1H-indazol-6-yl)-2,4-pyrimidinediamine;
4-methoxy-3-({4-[(3-methyl-1H-indazol-6-yl)amino]-2-pyrimidinyl}amino)benzenesulfonamide;
N²-[5-(isopropylsulfonyl)-2-methoxyphenyl]-N⁴-methyl-N⁴-(3-methyl-1H-indazol-6-yl)-2,4-pyrimidinediamine;
N²-[5-(ethylsulfonyl)-2-methoxyphenyl]-N⁴-isopropyl-N⁴-(3-methyl-1H-indazol-6-yl)-2,4-pyrimidinediamine;
N⁴-(1H-indazol-6-yl)-N⁴-methyl-N²-{3-[(methylsulfonyl)methyl]phenyl}-2,4-pyrimidinediamine;
N⁴-(1,3-dimethyl-1H-indazol-6-yl)-N⁴-methyl-N²-{3-[(methylsulfonyl)methyl]phenyl}-2,4-pyrimidinediamine;
N⁴-(2,3-dimethyl-2H-indazol-6-yl)-N⁴-methyl-N²-{3-[(methylsulfonyl)methyl]phenyl}-2,4-pyrimidinediamine;
N⁴-(2,3-dimethyl-2H-indazol-6-yl)-N²-[5-(ethylsulfonyl)-2-methoxyphenyl]-N⁴-methyl-2,4-pyrimidinediamine;
1-[4-methoxy-3-({4-[(3-methyl-1H-indazol-6-yl)amino]-2-pyrimidinyl}amino)phenyl]-1-propanone;
4-methoxy-N-[3-({4-[methyl(3-methyl-1H-indazol-6-yl)amino]-2-pyrimidinyl}amino)phenyl]benzenesulfonamide;
4-methoxy-N-methyl-3-({4-[(3-methyl-1H-indazol-6-yl)amino]-2-pyrimidinyl}amino)benzenesulfonamide;
[(3-methyl-1H-indazol-6-yl)(2-{4-[(methylsulfonyl)methyl]anilino}-4-pyrimidinyl)amino]acetonitrile;
[{2-[5-(ethylsulfonyl)-2-methoxyanilino]-4-pyrimidinyl}(3-methyl-1H-indazol-6-yl)amino]acetonitrile;
[(3-methyl-1H-indazol-6-yl)(2-{3-[(methylsulfonyl)methyl]anilino}-4-pyrimidinyl)amino]acetonitrile;
4-methoxy-N-methyl-3-({4-[methyl(3-methyl-1H-indazol-6-yl)amino]-2-pyrimidinyl}amino)benzenesulfonamide; 4-({4-[methyl(3-methyl-1H-indazol-6-yl)amino]-2-pyrimidinyl}amino)benzamide;
3-methoxy-4-({4-[methyl(3-methyl-1H-indazol-6-yl)amino]-2-pyrimidinyl}amino)benzenesulfonamide;
N⁴-ethynyl-N⁴-(3-methyl-1H-indazol-6-yl)-N²-{3-[(methylsulfonyl)methyl]phenyl}-2,4-pyrimidinediamine; 3-({4-[(3-methyl-1H-indazol-6-yl)(2-propynyl)amino]-2-pyrimidinyl}amino)benzenesulfonamide;
4-({4-[methyl(3-methyl-1H-indazol-6-yl)amino]-2-pyrimidinyl}amino)benzenesulfonamide;
N⁴-methyl-N⁴-(3-methyl-1H-indazol-6-yl)-N²-[3-(methylsulfonyl)phenyl]-2,4-pyrimidinediamine;
4-methoxy-3-({4-[methyl(3-methyl-1H-indazol-6-yl)amino]-2-pyrimidinyl}amino)benzenesulfonamide;
N²-[5-(ethylsulfonyl)-2-methoxyphenyl]-N⁴-(3-methyl-1H-indazol-6-yl)-2,4-pyrimidinediamine;
3-({4-[methyl(3-methyl-1H-indazol-6-yl)amino]-2-pyrimidinyl}amino)benzamide;
N²-[4-(ethylsulfonyl)phenyl]-N⁴-methyl-N⁴-(3-methyl-1H-indazol-6-yl)-2,4-pyrimidinediamine;
N-[4-({4-[methyl(3-methyl-1H-indazol-6-yl)amino]-2-pyrimidinyl}amino)benzyl]ethanesulfonamide;
N-[3-({4-[methyl(3-methyl-1H-indazol-6-yl)amino]-2-pyrimidinyl}amino)benzyl]methanesulfonamide;
2-chloro-5-({4-[methyl(3-methyl-1H-indazol-6-yl)amino]-2-pyrimidinyl}amino)benzenesulfonamide;
2-chloro-4-({4-[methyl(3-methyl-1H-indazol-6-yl)amino]-2-pyrimidinyl}amino)benzenesulfonamide;
4-chloro-3-({4-[methyl(3-methyl-1H-indazol-6-yl)amino]-2-pyrimidinyl}amino)benzenesulfonamide;
3-methyl-4-({4-[methyl(3-methyl-1H-indazol-6-yl)amino]-2-pyrimidinyl}amino)benzenesulfonamide;
2-methyl-5-({4-[methyl(3-methyl-1H-indazol-6-yl)amino]-2-pyrimidinyl}amino)benzenesulfonamide;
4-methyl-3-({4-[methyl(3-methyl-1H-indazol-6-yl)amino]-2-pyrimidinyl}amino)benzenesulfonamide;
N⁴-methyl-N⁴-(3-methyl-1H-indazol-6-yl)-N²-[3-(methylsulfinyl)phenyl]-2,4-pyrimidinediamine;
N²-[2-fluoro-5-(methylsulfonyl)phenyl]-N⁴-methyl-N⁴-(3-methyl-1H-indazol-6-yl)-2,4-pyrimidinediamine;
N²-[2-methoxy-5-(methylsulfonyl)phenyl]-N⁴-methyl-N⁴-(3-methyl-1H-indazol-6-yl)-2,4-pyrimidinediamine;
3-({4-[(2,3-dimethyl-2H-indazol-6-yl) (methyl)amino]pyrimidin-2-yl}amino)benzenesulfonamide;
2-[4-({4-[(2,3-dimethyl-2H-indazol-6-yl) (methyl)amino]pyrimidin-2-yl}amino)phenyl]ethanesulfonamide;
N⁴-(2,3-dimethyl-2H-indazol-6-yl)-N⁴-methyl-N²-{4-[(methylsulfonyl)methyl]phenyl}pyrimidine-2,4-diamine;
3-({4-[[3-(hydroxymethyl)-2-methyl-2H-indazol-6-yl] (methyl)amino]pyrimidin-2-yl}amino)benzenesulfonamide;
3-({4-[(1,2-dimethyl-1H-benzimidazol-5-yl) (methyl)amino]pyrimidin-2-yl}amino)benzenesulfonamide;
3-({4-[(2-benzyl-1-methyl-1H-benzimidazol-5-yl) (methyl)amino]pyrimidin-2-yl}amino)benzenesulfonamide;
3-({4-[(2-ethyl-3-methyl-2H-indazol-6-yl) (methyl)amino]pyrimidin-2-yl}amino)benzenesulfonamide;
3-({4-[[2-(3-chlorobenzyl)-3-methyl-2H-indazol-6-yl] (methyl)amino]pyrimidin-2-yl}amino)benzenesulfonamide;
3-({4-[(2,3-dimethyl-2H-indazol-6-yl) (methyl)amino]-1,3,5-triazin-2-yl}amino)benzenesulfonamide; and
5-({4-[(2,3-dimethyl-2H-indazol-6-yl) (methyl)amino]-1,3,5-triazin-2-yl}amino)-2-methylbenzenesulfonamide

An Aurorakinase inhibitor as an active ingredient of the prophylactic or therapeutic agent for of ALS in the present invention includes all of naturally-occurring substances derived from microorganism etc., semi-synthetic substances derived therefrom, and fully synthetic compounds. Examples thereof include, but are not limited to, ZM-447439, VX-680 (tozasertib/MK-0457), Aurora Kinase Inhibitor II, CYC116, KW2449 (see Table 4 for the above), AT9283 (see Table 8), danusertib/PHA-739358, alisertib/MLN8237, ENMD-2076, PF-3814735, cenisertib/R763/AS703569, BI811283, AMG900, TTP607, GSK1070916A, AZD1152, TAK-901, MK5108/VX-689, BI 847325 and the like. Preferably, it may be ZM-447439, VX-680, Aurora Kinase Inhibitor II, CYC116, KW2449 or AT9283. In another embodiment, the Aurorakinase inhibitor may be an Aurorakinase selective inhibitor. Examples of the Aurorakinase selective inhibitor include ZM-447439, VX-680, Aurora Kinase Inhibitor II, CYC116 and the like.

A PKA inhibitor as an active ingredient of the prophylactic or therapeutic agent for ALS in the present invention includes all of naturally-occurring substances derived from microorganism etc., semi-synthetic substances derived therefrom, and fully synthetic compounds. Examples thereof include, but are not limited to, PDGF Receptor Tyrosine Kinase Inhibitor III (see Table 4), H89, KT5720 and the like. Preferably, it may be PDGF Receptor Tyrosine Kinase Inhibitor III. In another embodiment, the PKA inhibitor may be a PKA selective inhibitor.

A PKC inhibitor as an active ingredient of the prophylactic or therapeutic agent for ALS in the present invention includes all of naturally-occurring substances derived from microorganism etc., semi-synthetic substances derived therefrom, and fully synthetic compounds. Examples thereof include, but are not limited to, PDGF Receptor Tyrosine Kinase Inhibitor III, Enzastaurin (see Table 4 for the above), bryostatin 1, UCN-01, AEB071/sotrastaurin acetate, safingol, midostaurin/PKC₄ 12, sophoretin/quercetin and the like. Preferably, it may be PDGF Receptor Tyrosine Kinase Inhibitor III, Enzastaurin. In another embodiment, the PKC inhibitor may be a PKC selective inhibitor. Examples of the PKC selective inhibitor include Enzastaurin and the like.

An MEK inhibitor as an active ingredient of the prophylactic or therapeutic agent for ALS in the present invention includes all of naturally-occurring substances derived from microorganism etc., semi-synthetic substances derived therefrom, and fully synthetic compounds. Examples thereof include, but are not limited to, U0126-EtOH (see Table 4), selumetinib/AZD6244/ARRY-142886, refametinib/RDEA119/BAY869766, pimasertib/MSC1936369/AS703026, MEK162/ARRY-162, AZD8330/ARRY-424704, cobimetinib/GDC-0973/RG7420, GDC-0623/RG7421/XL518, CIF/RG7167/RO4987655, CKI27/RG7304/RO5126766/CH5126766, E6201, TAK-733, PD-0325901, AS703988/MSC₂ 015103B, WX-554, BVD-523, MK-8353/SCH9000353, BI 847325, CI-1040/PD184352 and the like. Preferably, it may be U0126-EtOH. In another embodiment, the MEK inhibitor may be an MEK selective inhibitor. Examples of the MEK selective inhibitor include U0126-EtOH.

An Met inhibitor as an active ingredient of the prophylactic or therapeutic agent for ALS in the present invention includes all of naturally-occurring substances derived from microorganism etc., semi-synthetic substances derived therefrom, and fully synthetic compounds. Examples thereof include, but are not limited to, MGCD-265, PF-2341066 (Crizotinib) or an analog thereof, BMS 777607 (see Table 4 for the above), INC₂ 80/INCB-028060, foretinib/GSK1363089/XL880, tivantinib/ARQ197, EMD-94283, MSC₂ 156119J/EMD1214063, golvatinib/E7050, JNJ-38877605, MK-2461, MK-8033, PF-4217903, AMG208, AMG 337, SAR125844, cabozantinib/BMS-907351/XL184, LY2801653, TAS-115, volitinib/HMPL-504, amuvatinib/MP-470/HPK-56, S49076 and the like. Preferably, it may be MGCD-265, PF-2341066 (Crizotinib) or an analog thereof, BMS 777607. In another embodiment, Met inhibitor may be a Met selective inhibitor. Examples of the Met selective inhibitor include PF-2341066(Crizotinib) or an analog thereof, BMS 777607 and the like.

Here, the analog of "PF-2341066 (Crizotinib)" is, for example, a compound represented by the following formula (II): 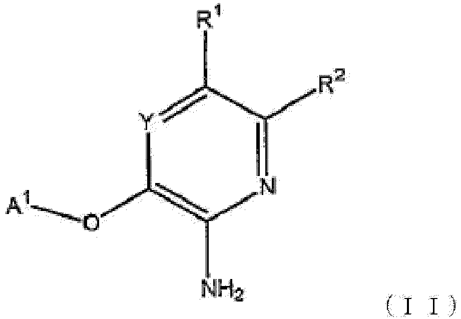 wherein
Y is N or CR¹²;
R¹ is selected from C₆₋₁₂ aryl, 5 - 12-membered heteroaryl, C₃₋₁₂ cycloalkyl, 3 - 12-membered heteroalicycle, -O(CR⁶R⁷)ₙR⁴, - C(O)R⁴, -C(O)OR⁴, -CN, -NO₂, -S(O)ₘR⁴, -SO₂NR⁴R⁵, -C(O)NR⁴R⁵, - NR⁴C(O)R⁵, -C(=NR⁶)NR⁴R⁵, C₁₋₈ alkyl, C₂₋₈ alkenyl, and C₂₋₈ alkynyl, each hydrogen in R¹ is optionally substituted by one or more R³ groups;
R² is hydrogen, halogen, C₁₋₁₂ alkyl, C₂₋₁₂ alkenyl, C₂₋₁₂ alkynyl, C₃₋₁₂ cycloalkyl, C₆₋₁₂ aryl, 3 - 12-membered heteroalicycle, 5 - 12-membered heteroaryl, -S(O)ₘR⁴, -SO₂NR⁴R⁵, -S(O)₂OR⁴, -NO₂, - NR⁴R⁵, - (CR⁶R⁷)ₙOR⁴, -CN, -C(O)R⁴, -OC(O)R⁴, -O(CR⁶R⁷)ₙ^{R4}, - NR⁴C(O)R⁵, -(CR⁶R⁷)ₙC(O)OR⁴, -(CR⁶R⁷)ₙNCR⁴R⁵, -C(=NR⁶)NR⁴R⁵, -
NR⁴C(O)NR⁵R⁶, -NR⁴S(O)ₚR⁵, or-C(O)NR⁴R⁵, each hydrogen in R² is optionally substituted by one or more R⁸ groups;
R³ is halogen, C₁₋₁₂ alkyl, C₂₋₁₂ alkenyl, C₂₋₁₂ alkynyl, C₃₋₁₂ cycloalkyl, C₆₋₁₂ aryl, 3 - 12-membered heteroalicycle, 5 - 12-membered heteroaryl, -S(O)ₘR⁴, -SO₂NR⁴R⁵, -S(O)₂OR⁴, -NO₂, -NR⁴R⁵, -(CR⁶R⁷)ₙOR⁴, -CN, -C(O)R⁴, -OC(O)R⁴, -O(CR⁶R⁷)ₙR⁴, -NR⁴C(O)R⁵, - (CR⁶R⁷)ₙC(O)OR⁴, - (CR⁶R⁷)ₙNCR⁴R⁵, -C(=NR⁶)NR⁴R⁵, -NR⁴C(O)NR⁵R⁶, - NR⁴S(O)ₚR⁵, or-C(O)NR⁴R⁵, each hydrogen in R³ is optionally substituted by one or more R⁸ groups, and R³ groups on the adjacent atoms may be joined to form C₆₋₁₂ aryl, 5 - 12-membered heteroaryl, C₃₋₁₂ cycloalkyl, or 3 - 12-membered heteroalicyclic group;
R⁴, R⁵, R⁶ and R⁷ are each independently hydrogen, halogen, C₁₋₁₂ alkyl, C₂₋₁₂ alkenyl, C₂₋₁₂ alkynyl, C₃₋₁₂ cycloalkyl, C₆₋₁₂ aryl, 3 - 12-membered heteroalicycle, or 5 - 12-membered heteroaryl, or any two of R⁴, R⁵, R⁶ and R⁷ bonded to the same nitrogen atom may be joined with the nitrogen bonded thereto to form 3 - 12-membered heteroalicyclic or 5 - 12-membered heteroaryl group containing 1 - 3 additional hetero atoms selected from N, O, and S; or any two of R⁴, R⁵, R⁶ and R⁷ bonded to the same carbon atom may be joined to form C₃₋₁₂ cycloalkyl, C₆₋₁₂ aryl, 3 - 12-membered heteroalicycle, or 5 - 12-membered heteroaryl group; and each hydrogen in R⁴, R⁵, R⁶ and R⁷ is optionally substituted by one or more R⁸ groups;
one or more R⁸ are each independently halogen, C₁₋₁₂ alkyl, C₂₋₁₂ alkenyl, C₂₋₁₂ alkynyl, C₃₋₁₂ cycloalkyl, C₆₋₁₂ aryl, 3 - 12-membered heteroalicycle, 5 - 12-membered heteroaryl, -CN, -O-C₁₋₁₂ alkyl, -O-(CH₂)ₙC₃₋₁₂ cycloalkyl, -O-(CH₂)ₙC₆₋₁₂ aryl, -O-(CH₂)ₙ(3 - 12-membered heteroalicyclic) or -O-(CH₂)ₙ(5 - 12-membered heteroaryl); each hydrogen in R⁸ is optionally substituted by one or more R¹¹ groups;
A¹ is -(CR⁹R¹⁰)ₙ-A² {provided:
   (i) when Y is N, and R¹ is substituted or unsubstituted aryl, or substituted or unsubstituted heteroaryl, A¹ is -(CR⁹R¹⁰)ₙ-A², and n is not 0; and
   (ii) when Y is N, R² is H, and A¹ is m-chlorobenzyl, R¹ is not unsubstituted piperazine};
R⁹ and R¹⁰ are each independently hydrogen, halogen, C₁₋₁₂ alkyl, C₃₋₁₂ cycloalkyl, C₆₋₁₂ aryl, 3 - 12-membered heteroalicycle, 5 - 12-membered heteroaryl, -S(O)ₘR⁴, -SO₂NR⁴R⁵, -S(O)₂OR⁴, -NO₂, - NR⁴R⁵, -(CR⁶R⁷)ₙOR⁴, -CN, -C(O)R⁴, -OC(O)R⁴, -NR⁴C(O)R⁵, - (CR⁶R⁷)ₙC(O)OR⁴, -(CR⁶R⁷)ₙNCR⁴R⁵, -NR⁴C(O)NR⁵R⁶, -NR⁴S(O)ₚR⁵, or-C(O)NR⁴R⁵; R⁹ and R¹⁰ may be joined to form C₃₋₁₂ cycloalkyl, 3 - 12-membered heteroalicycle, C₆₋₁₂ aryl, or 5 - 12-membered heteroaryl ring; each hydrogen in R⁹ and R¹⁰ is optionally substituted by one or more R³ groups;
A² is C₆₋₁₂ aryl, 5 - 12-membered heteroaryl, C₃₋₁₂ cycloalkyl, or 3 - 12-membered heteroalicycle, A² is optionally substituted by one or more R³ groups;
one or more R¹¹ are each independently halogen, C₁₋₁₂ alkyl, C₁₋₁₂ alkoxy, C₃₋₁₂ cycloalkyl, C₆₋₁₂ aryl, 3 - 12-membered heteroalicycle, 5 - 12-membered heteroaryl, -O-C₁₋₁₂ alkyl, -O-(CH₂)ₙC₃₋₁₂ cycloalkyl, -O-(CH₂)ₙC₆₋₁₂ aryl, -O-(CH₂)ₙ(3 - 12-membered heteroalicyclic), -O-(CH₂)ₙ(5 - 12-membered heteroaryl), or-CN, each hydrogen in R¹¹ is optionally substituted by one or more groups selected from halogen, -OH, - CN, -C₁₋₁₂ alkyl optionally halogenated partly or completely, - O-C₁₋₁₂ alkyl optionally halogenated partly or completely, -CO, -SO, and -SO₂;
R¹² is hydrogen, halogen, C₁₋₁₂ alkyl, C₂₋₁₂ alkenyl, C₂₋₁₂ alkynyl, C₃₋₁₂ cycloalkyl, C₆₋₁₂ aryl, 3 - 12-membered heteroalicycle, 5 - 12-membered heteroaryl, -S(O)ₘR⁴, -SO₂NR⁴R⁵, -S(O)₂OR⁴, -NO₂, - NR⁴R⁵, -(CR⁶R⁷)ₙOR⁴, -CN, -C(O)R⁴, -OC(O)R⁴, -O(CR⁶R⁷)ₙR⁴, - NRC(O)R⁵, -(CR⁶R⁷)ₙC(O)OR⁴, -(CR⁶R⁷)ₙNCR⁴R⁵, -C(=NR⁶)NR⁴R⁵, - NR⁴C(O)NR⁵R⁶, -NR⁴S(O)ₚR⁵, or -C(O)NR⁴R⁵, each hydrogen in R¹² is optionally substituted by one or more R³ groups;
R¹ and R², or R¹ and R¹² may be joined to form C₆₋₁₂ aryl, 5 - 12-membered heteroaryl, C₃₋₁₂ cycloalkyl, or 3 - 12-membered heteroalicyclic group;
m is 0, 1 or 2;
n is 0, 1, 2, 3 or 4; and
p is 1 or 2.

Each term described as higher concept in the explanation of each group in the above-mentioned formula (II) (e.g., "C₆₋₁₂ aryl", "5 - 12-membered heteroaryl", "C₃₋₁₂ cycloalkyl", "3 - 12-membered heteroalicycle", "C₁₋₈ alkyl", "C₂₋₈ alkenyl", "C₂₋₈ alkynyl" etc. for R¹) is as defined in WO 2004/076412 (National Publication of International Patent Application No. 2006-519232).

Specific examples of the analog of PF-2341066 (Crizotinib) include the following.
4-[6-amino-5-(2,6-dichloro-benzyloxy)-pyridin-3-yl]-phenol; 3-(2,6-dichloro-benzyloxy)-5-[4-(2-morpholin-4-yl-ethoxy)-phenyl]-pyridin-2-ylamine;
3-(2,6-dichloro-benzyloxy)-5-[3-(2-morpholin-4-yl-ethoxy)-phenyl]-pyridin-2-ylamine;
3-(2,6-dichloro-benzyloxy)-5-(1H-indol-4-yl)-pyridin-2-ylamine; 3-[2-chloro-6-(1H-indol-4-yl)-benzyloxy]-5-(1H-indol-4-yl)-pyridin-2-ylamine;
2-[6-amino-5-(2,6-dichloro-benzyloxy)-pyridin-3-yl]-pyrrole-1-carboxylic acid tert-butyl ester;
3-(2,6-dichloro-benzyloxy)-5-(1H-pyrrol-2-yl)-pyridin-2-ylamine;
3-(2,6-dichloro-benzyloxy)-5-(4-fluoro-phenyl)-pyridin-2-ylamine;
3-(2,6-dichloro-benzyloxy)-5-phenyl-pyridin-2-ylamine; 3-(2,6-dichloro-benzyloxy)-5-(2-fluoro-phenyl)-pyridin-2-ylamine;
3-(2,6-dichloro-benzyloxy)-5-(3-fluoro-phenyl)-pyridin-2-ylamine;
5-(4-amino-phenyl)-3-(2,6-dichloro-benzyloxy)-pyridin-2-ylamine;
N-{4-[6-amino-5-(2,6-dichloro-benzyloxy)-pyridin-3-yl]-phenyl}-methanesulfonamide;
N-{4-[6-amino-5-(2,6-dichloro-benzyloxy)-pyridin-3-yl]-phenyl}-acetamide;
3-[6-amino-5-(2,6-dichloro-benzyloxy)-pyridin-3-yl]-phenol; 3-(2,6-dichloro-benzyloxy)-5-(4-methoxy-phenyl)-pyridin-2-ylamine;
5-(3-amino-phenyl)-3-(2,6-dichloro-benzyloxy)-pyridin-2-ylamine;
3-(2,6-dichloro-benzyloxy)-5-(3-trifluoromethoxy-phenyl)-pyridin-2-ylamine;
2-[6-amino-5-(2,6-dichloro-benzyloxy)-pyridin-3-yl]-phenol; 3-(2,6-dichloro-benzyloxy)-5-(2-phenoxy-phenyl)-pyridin-2-ylamine;
3-(2,6-dichloro-benzyloxy)-5-(3,4-difluoro-phenyl)-pyridin-2-ylamine;
3-(2,6-dichloro-benzyloxy)-5-(3-isopropyl-phenyl)-pyridin-2-ylamine;
3-(2,6-dichloro-benzyloxy)-5-(2-trifluoromethyl-phenyl)-pyridin-2-ylamine;
3-(2,6-dichloro-benzyloxy)-5-(2-methoxy-phenyl)-pyridin-2-ylamine;
3-(2,6-dichloro-benzyloxy)-5-(4-trifluoromethyl-phenyl)-pyridin-2-ylamine;
N-{2-[6-amino-5-(2,6-dichloro-benzyloxy)-pyridin-3-yl]-phenyl}-methanesulfonamide;
{4-[6-amino-5-(2,6-dichloro-benzyloxy)-pyridin-3-yl]-phenyl}-methanol;
5-benzo[1,3]dioxol-5-yl-3-(2,6-dichloro-benzyloxy)-pyridin-2-ylamine;
3-(2,6-dichloro-benzyloxy)-5-(2-trifluoromethoxy-phenyl)-pyridin-2-ylamine;
3-(2,6-dichloro-benzyloxy)-5-(4-methyl-thiophen-2-yl)-pyridin-2-ylamine;
5-(2-benzyloxy-phenyl)-3-(2,6-dichloro-benzyloxy)-pyridin-2-ylamine;
3-(2,6-dichloro-benzyloxy)-5-(3-methoxy-phenyl)-pyridin-2-ylamine;
3-(2,6-dichloro-benzyloxy)-5-(1H-indol-2-yl)-pyridin-2-ylamine; 5-(4-benzyloxy-3-fluoro-phenyl)-3-(2,6-dichloro-benzyloxy)-pyridin-2-ylamine;
4-[6-amino-5-(2,6-dichloro-benzyloxy)-pyridin-3-yl]-benzoic acid;
4-[6-amino-5-(2,6-dichloro-benzyloxy)-pyridin-3-yl]-N-(2-diethylamino-ethyl)-benzamide;
4-[6-amino-5-(2,6-dichloro-benzyloxy)-pyridin-3-yl]-N-(3-diethylamino-propyl)-benzamide;
{4-[6-amino-5-(2,6-dichloro-benzyloxy)-pyridin-3-yl]-phenyl}-(4-methyl-piperazin-1-yl)-methanone;
{4-[6-amino-5-(2,6-dichloro-benzyloxy)-pyridin-3-yl]-phenyl}-[(2R)-2-pyrrolidin-1-ylmethyl-pyrrolidin-1-yl]-methanone;
{4-[6-amino-5-(2,6-dichloro-benzyloxy)-pyridin-3-yl]-phenyl}-[(2S)-2-pyrrolidin-1-ylmethyl-pyrrolidin-1-yl]-methanone;
{4-[6-amino-5-(2,6-dichloro-benzyloxy)-pyridin-3-yl]-phenyl}-(4-pyrrolidin-1-yl-piperidin-1-yl]-methanone;
{4-[6-amino-5-(2,6-dichloro-benzyloxy)-pyridin-3-yl]-phenyl}-[4-(2-hydroxy-ethyl)-piperidin-1-yl]-methanone;
{4-[6-amino-5-(2,6-dichloro-benzyloxy)-pyridin-3-yl]-phenyl}-[(3S)-3-dimethylamino-pyrrolidin-1-yl]-methanone;
{4-[6-amino-5-(2,6-dichloro-benzyloxy)-pyridin-3-yl]-phenyl}-[(3R)-3-dimethylamino-pyrrolidin-1-yl]-methanone;
{4-[6-amino-5-(2,6-dichloro-benzyloxy)-pyridin-3-yl]-phenyl}-[(3S)-3-cyclopropylaminomethyl-piperidin-1-yl]-methanone;
4-[6-amino-5-(2,6-dichloro-benzyloxy)-pyridin-3-yl]-N-(2-hydroxy-3-pyrrolidin-1-yl-propyl)-benzamide;
{4-[6-amino-5-(2,6-dichloro-benzyloxy)-pyridin-3-yl]-phenyl}-[(2S)-2-(3-fluoro-piperidin-1-ylmethyl)-pyrrolidin-1-yl]-methanone;
{4-[6-amino-5-(2,6-dichloro-benzyloxy)-pyridin-3-yl]-phenyl}-(4-cyclopropyl-piperazin-1-yl)-methanone;
{4-[6-amino-5-(2,6-dichloro-benzyloxy)-pyridin-3-yl]-phenyl}-{(2R)-2-[(cyclopropylmethyl-amino)-methyl]-pyrrolidin-1-yl}-methanone;
4-[6-amino-5-(2,6-dichloro-benzyloxy)-pyridin-3-yl]-N-cyclopropylmethyl-N-(2R)-pyrrolidin-2-ylmethyl-benzamide;
4-[6-amino-5-(2,6-dichloro-benzyloxy)-pyridin-3-yl]-N-(2-hydroxy-3-pyrrolidin-1-yl-propyl)-N-methyl-benzamide;
{4-[6-amino-5-(2,6-dichloro-benzyloxy)-pyridin-3-yl]-phenyl}-{ (2S)-2-[(3R)-3-hydroxy-pyrrolidin-1-ylmethyl]-pyrrolidin-1-yl}-methanone;
3-[6-amino-5-(2,6-dichloro-benzyloxy)-pyridin-3-yl]-benzoic acid;
{3-[6-amino-5-(2,6-dichloro-benzyloxy)-pyridin-3-yl]-phenyl}-[(2R)-2-pyrrolidin-1-ylmethyl-pyrrolidin-1-yl]-methanone;
{4-[6-amino-5-(2,6-dichloro-benzyloxy)-pyridin-3-yl]-phenoxy}-acetic acid;
2-{4-[6-amino-5-(2,6-dichloro-benzyloxy)-pyridin-3-yl]-phenoxy}-1-[(2R)-2-pyrrolidin-1-ylmethyl-pyrrolidin-1-yl]-ethanone;
2-{4-[6-amino-5-(2,6-dichloro-benzyloxy)-pyridin-3-yl]-phenoxy}-1-[(2S)-2-pyrrolidin-1-ylmethyl-pyrrolidin-1-yl]-ethanone;
3-(2,6-dichloro-benzyloxy)-5-(1H-indol-5-yl)-pyridin-2-ylamine; 3-(2,6-dichloro-benzyloxy)-5-[3-(1-methyl-1,2,3,6-tetrahydro-pyridin-4-yl)-1H-indol-5-yl]-pyridin-2-ylamine;
3-(2,6-dichloro-benzyloxy)-5-[3-(1-methyl-piperidin-4-yl)-1H-indol-5-yl]-pyridin-2-ylamine;
3-(2,6-dichloro-benzyloxy)-5-(3-morpholin-4-ylmethyl-1H-indol-5-yl)-pyridin-2-ylamine;
3-(2,6-dichloro-benzyloxy)-5-(3-piperidin-1-ylmethyl-1H-indol-5-yl)-pyridin-2-ylamine;
3-(2,6-dichloro-benzyloxy)-5-(3-pyrrolidin-1-ylmethyl-1H-indol-5-yl)-pyridin-2-ylamine;
3-(2,6-dichloro-benzyloxy)-5-(3-diethylaminomethyl-1H-indol-5-yl)-pyridin-2-ylamine;
(1-{5-[6-amino-5-(2,6-dichloro-benzyloxy)-pyridin-3-yl]-1H-indol-3-ylmethyl}-(3R)-pyrrolidin-3-yl)-carbamic acid tert-butyl ester;
3-(2,6-dichloro-benzyloxy)-5-{3-(2,6-dimethyl-morpholin-4-ylmethyl)-1H-indol-5-yl}-pyridin-2-ylamine;
N-(1-{5-[6-amino-5-(2,6-dichloro-benzyloxy)-pyridin-3-yl]-1H-indol-3-ylmethyl}-(3R)-pyrrolidin-3-yl)-acetamide;
1-(4-{5-[6-amino-5-(2,6-dichloro-benzyloxy)-pyridin-3-yl]-1H-indol-3-ylmethyl}-piperazin-1-yl)-ethanone;
3-(2-chloro-3,6-difluoro-benzyloxy)-5-(1H-indole-5-yl)-pyridin-2-ylamine;
1-(4-{5-[6-amino-5-(2-chloro-3,6-difluoro-benzyloxy)-pyridin-3-yl]-1H-indol-3-ylmethyl}-piperazin-1-yl)-ethanone;
3-(2-chloro-3,6-difluoro-benzyloxy)-5-[3-(2,6-dimethyl-morpholin-4-ylmethyl)-1H-indol-5-yl]-pyridin-2-ylamine;
N-(1-{5-[6-amino-5-(2-chloro-3,6-difluoro-benzyloxy)-pyridin-3-yl]-1H-indol-3-ylmethyl}-(3S)-pyrrolidin-3-yl)-acetamide;
3-(2-chloro-3,6-difluoro-benzyloxy)-5-(3-piperidin-1-ylmethyl-1H-indol-5-yl)-pyridin-2-ylamine;
3-(2-chloro-3,6-difluoro-benzyloxy)-5-(3-morpholin-4-ylmethyl-1H-indol-5-yl)-pyridin-2-ylamine;
3-(2-chloro-3,6-difluoro-benzyloxy)-5-(3-pyrrolidin-1-ylmethyl-1H-indol-5-yl)-pyridin-2-ylamine;
5-[6-amino-5-(2,6-dichloro-benzyloxy)-pyridin-3-yl]-1H-indole-2-carboxylic acid ethyl ester;
5-[6-amino-5-(2,6-dichloro-benzyloxy)-pyridin-3-yl]-1H-indole-2-carboxylic acid;
{5-[6-amino-5-(2,6-dichloro-benzyloxy)-pyridin-3-yl]-1H-indol-2-yl}-(4-methyl-piperazin-1-yl)-methanone;
{5-[6-amino-5-(2,6-dichloro-benzyloxy)-pyridin-3-yl]-1H-indol-2-yl}-[(3R)-3-dimethylamino-pyrrolidin-1-yl]-methanone;
{5-[6-amino-5-(2,6-dichloro-benzyloxy)-pyridin-3-yl]-1H-indol-2-yl}-[(2R)-2-pyrrolidin-1-ylmethyl-pyrrolidin-1-yl]-methanone; 5-[6-amino-5-(2,6-dichloro-benzyloxy)-pyridin-3-yl]-1H-indole-2-carboxylic acid (2-pyrrolidin-1-yl-ethyl)-amide;
5-[6-amino-5-(2,6-dichloro-benzyloxy)-pyridin-3-yl]-1H-indole-2-carboxylic acid (2-morpholin-4-yl-ethyl)-amide;
(1-{5-[6-amino-5-(2,6-dichloro-benzyloxy)-pyridin-3-yl]-1H-indole-2-carbonyl}-(3S)-pyrrolidin-3-yl)-carbamic acid tert-butyl ester;
{5-[6-amino-5-(2,6-dichloro-benzyloxy)-pyridin-3-yl]-1H-indol-2-yl}-[(3S)-3-amino-pyrrolidin-1-yl]-methanone;
5-[6-amino-5-(2,6-dichloro-benzyloxy)-pyridin-3-yl]-1H-indole-2-carboxylic acid (2-hydroxy-3-pyrrolidin-1-yl-propyl)-amide; 4-(6-amino-5-benzyloxy-pyridin-3-yl)-phenol;
3-benzyloxy-5-phenyl-pyridin-2-ylamine;
3-(3-methoxy-benzyloxy)-5-phenyl-pyridin-2-ylamine;
3-(2-chloro-4-fluoro-benzyloxy)-5-phenyl-pyridin-2-ylamine; 3-(2-chloro-benzyloxy)-5-phenyl-pyridin-2-ylamine;
3-(2,5-dichloro-benzyloxy)-5-phenyl-pyridin-2-ylamine;
3-(2-chloro-5-trifluoromethyl-benzyloxy)-5-phenyl-pyridin-2-ylamine;
3-(2,4-dichloro-5-fluoro-benzyloxy)-5-phenyl-pyridin-2-ylamine; 3-(2-chloro-3-trifluoromethyl-benzyloxy)-5-phenyl-pyridin-2-ylamine;
3-(2-chloro-3,6-difluoro-benzyloxy)-5-phenyl-pyridin-2-ylamine;
3-(3,4-dichloro-benzyloxy)-5-phenyl-pyridin-2-ylamine;
2-(2-amino-5-phenyl-pyridin-3-yloxymethyl)-benzonitrile;
3-(2-chloro-6-fluoro-3-methyl-benzyloxy)-5-phenyl-pyridin-2-ylamine;
5-phenyl-3-(2,3,6-trifluoro-benzyloxy)-pyridin-2-ylamine; 3-(2,6-difluoro-benzyloxy)-5-phenyl-pyridin-2-ylamine;
3-(2,6-difluoro-3-methyl-benzyloxy)-5-phenyl-pyridin-2-ylamine; 3-(3-chloro-2,6-difluoro-benzyloxy)-5-phenyl-pyridin-2-ylamine; 3-(2-chloro-6-fluoro-benzyloxy)-5-phenyl-pyridin-2-ylamine;
3-(3-fluoro-4-methoxy-benzyloxy)-5-phenyl-pyridin-2-ylamine; N-[3-(2-amino-5-phenyl-pyridin-3-yloxymethyl)-phenyl]-methanesulfonamide;
5-[4-(2-morpholin-4-yl-ethoxy)-phenyl]-3-(3-nitro-benzyloxy)-pyridin-2-ylamine;
5-[4-(2-morpholin-4-yl-ethoxy)-phenyl]-3-(naphthalen-1-ylmethoxy)-pyridin-2-ylamine; 3-(2-chloro-3,6-difluoro-benzyloxy)-5-[4-(2-morpholin-4-yl-ethoxy)-phenyl]-pyridin-2-ylamine;
2-{2-amino-5-[4-(2-morpholin-4-yl-ethoxy)-phenyl]-pyridin-3-yloxy}-N-(4-isopropyl-phenyl)-2-phenyl-acetamide;
3-(5-chloro-benzo[b]thiophen-3-ylmethoxy)-5-[4-(2-morpholin-4-yl-ethoxy)-phenyl]-pyridin-2-ylamine;
{4-[6-amino-5-(4-fluoro-2-trifluoromethyl-benzyloxy)-pyridin-3-yl]-phenyl}-[(2R)-2-pyrrolidin-1-ylmethyl-pyrrolidin-1-yl]-methanone;
{4-[6-amino-5-(2-fluoro-6-trifluoromethyl-benzyloxy)-pyridin-3-yl]-phenyl}-[(2R)-2-pyrrolidin-1-ylmethyl-pyrrolidin-1-yl]-methanone;
{4-[6-amino-5-(5-fluoro-2-trifluoromethyl-benzyloxy)-pyridin-3-yl]-phenyl}-[(2R)-2-pyrrolidin-1-ylmethyl-pyrrolidin-1-yl]-methanone;
(4-{6-amino-5-[1-(2-trifluoromethyl-phenyl)-ethoxy]-pyridin-3-yl}-phenyl)-[(2R)-2-pyrrolidin-1-ylmethyl-pyrrolidin-1-yl]-methanone;
{4-[6-amino-5-(2-bromo-benzyloxy)-pyridin-3-yl]-phenyl}-[(2R)-2-pyrrolidin-1-ylmethyl-pyrrolidin-1-yl]-methanone;
{4-[6-amino-5-(3-fluoro-2-trifluoromethyl-benzyloxy)-pyridin-3-yl]-phenyl}-[(2R)-2-pyrrolidin-1-ylmethyl-pyrrolidin-1-yl]-methanone;
{4-[6-amino-5-(2-chloro-3,6-difluoro-benzyloxy)-pyridin-3-yl]-phenyl}-[(2R)-2-pyrrolidin-1-ylmethyl-pyrrolidin-1-yl]-methanone;
4-[6-amino-5-(2,6-difluoro-benzyloxy)-pyridin-3-yl]-phenol;
3-(2,6-difluoro-benzyloxy)-5-(1H-indol-4-yl)-pyridin-2-ylamine; 3-(2,6-difluoro-benzyloxy)-5-[4-(2-morpholin-4-yl-ethoxy)-phenyl]-pyridin-2-ylamine;
4-[6-amino-5-(2,6-difluoro-benzyloxy)-pyridin-3-yl]-benzoic acid;
{4-[6-amino-5-(2,6-difluoro-benzyloxy)-pyridin-3-yl]-phenyl}-[(2R)-2-pyrrolidin-1-ylmethyl-pyrrolidin-1-yl]-methanone;
{4-[6-amino-5-(2,6-difluoro-benzyloxy)-pyridin-3-yl]-phenyl}-[(2S)-2-pyrrolidin-1-ylmethyl-pyrrolidin-1-yl]-methanone;
{4-[6-amino-5-(2,6-difluoro-benzyloxy)-pyridin-3-yl]-phenoxy}-acetic acid ester;
{4-[6-amino-5-(2,6-difluoro-benzyloxy)-pyridin-3-yl]-phenoxy}-acetic acid;
2-{4-[6-amino-5-(2,6-difluoro-benzyloxy)-pyridin-3-yl]-phenoxy}-1-[(2R)-2-pyrrolidin-1-ylmethyl-pyrrolidin-1-yl]-ethanone;
2-{4-[6-amino-5-(2,6-difluoro-benzyloxy)-pyridin-3-yl]-phenoxy}-1-[(2S)-2-pyrrolidin-1-ylmethyl-pyrrolidin-1-yl]-ethanone;
4-[6-amino-5-(2-chloro-6-fluoro-benzyloxy)-pyridin-3-yl]-phenol;
4-[6-amino-5-(2-chloro-4-fluoro-benzyloxy)-pyridin-3-yl]-phenol;
4-[6-amino-5-(2,4-dichloro-benzyloxy)-pyridin-3-yl]-phenol; 2-[2-amino-5-(4-hydroxy-phenyl)-pyridin-3-yloxymethyl]-benzonitrile;
4-[6-amino-5-(2-trifluoromethyl-benzyloxy)-pyridin-3-yl]-phenol;
4-[6-amino-5-(2-chloro-benzyloxy)-pyridin-3-yl]-phenol;
4-[6-amino-5-(4-tert-butyl-benzyloxy)-pyridin-3-yl]-phenol; N-{4-[6-amino-5-(2-cyano-benzyloxy)-pyridin-3-yl]-phenyl}-methanesulfonamide;
2-[2-amino-5-(4-methanesulfonylamino-phenyl)-pyridin-3-yloxymethyl]-benzamide;
2-[2-amino-5-(4-methanesulfonylamino-phenyl)-pyridin-3-yloxymethyl]-benzoic acid;
N-(4-{6-amino-5-[2-(4-methyl-piperazine-1-carbonyl)-benzyloxy]-pyridin-3-yl}-phenyl)-methanesulfonamide;
2-[2-amino-5-(4-methanesulfonylamino-phenyl)-pyridin-3-yloxymethyl]-N-(2-hydroxy-ethyl)-benzamide;
2-[2-amino-5-(4-methanesulfonylamino-phenyl)-pyridin-3-yloxymethyl]-N-isobutyl-benzamide;
4-[6-amino-5-(2-chloro-6-fluoro-benzyloxy)-pyridin-3-yl]-benzoic acid;
{4-[6-amino-5-(2-chloro-6-fluoro-benzyloxy)-pyridin-3-yl]-phenyl}-[(2R)-2-pyrrolidin-1-ylmethyl-pyrrolidin-1-yl]-methanone;
{4-[6-amino-5-(2-chloro-6-fluoro-benzyloxy)-pyridin-3-yl]-phenyl}-[(2S)-2-pyrrolidin-1-ylmethyl-pyrrolidin-1-yl]-methanone;
{4-[6-amino-5-(2-chloro-6-fluoro-benzyloxy)-pyridin-3-yl]-phenyl}-[(3S)-3-dimethylamino-pyrrolidin-1-yl]-methanone;
{4-[6-amino-5-(2-chloro-6-fluoro-benzyloxy)-pyridin-3-yl]-phenyl}-[(3S)-3-amino-pyrrolidin-1-yl]-methanone;
{4-[6-amino-5-(2-chloro-6-fluoro-benzyloxy)-pyridin-3-yl]-phenyl}-(4-methyl-piperazin-1-yl)-methanone;
1-(4-{4-[6-amino-5-(2-chloro-6-fluoro-benzyloxy)-pyridin-3-yl]-benzoyl}-piperazin-1-yl)-ethanone;
4-[6-amino-5-(2-chloro-6-fluoro-benzyloxy)-pyridin-3-yl]-N-(2-morpholin-4-yl-ethyl)-benzamide;
4-[6-amino-5-(2-chloro-6-fluoro-benzyloxy)-pyridin-3-yl]-N-(3-morpholin-4-yl-propyl)-benzamide; 4-[6-amino-5-(2-chloro-benzyloxy)-pyridin-3-yl]-benzoic acid;
{4-[6-amino-5-(2-chloro-benzyloxy)-pyridin-3-yl]-phenyl}-[(2R)-2-pyrrolidin-1-ylmethyl-pyrrolidin-1-yl]-methanone;
{4-[6-amino-5-(2-chloro-benzyloxy)-pyridin-3-yl]-phenyl}-[(2S)-2-pyrrolidin-1-ylmethyl-pyrrolidin-1-yl]-methanone;
{4-[6-amino-5-(2-chloro-benzyloxy)-pyridin-3-yl]-phenyl}-[(3S)-3-dimethylamino-pyrrolidin-1-yl]-methanone;
{4-[6-amino-5-(2-chloro-benzyloxy)-pyridin-3-yl]-phenyl}-[(3S)-3-amino-pyrrolidin-1-yl]-methanone;
{4-[6-amino-5-(2-chloro-benzyloxy)-pyridin-3-yl]-phenyl}-(4-pyrrolidin-1-yl-piperidin-1-yl)-methanone;
{4-[6-amino-5-(2-chloro-benzyloxy)-pyridin-3-yl]-phenyl}-(4-methyl-piperazin-1-yl)-methanone;
1-(4-{4-[6-amino-5-(2-chloro-benzyloxy)-pyridin-3-yl]-benzoyl}-piperazin-1-yl)-ethanone;
4-[6-amino-5-(2-chloro-benzyloxy)-pyridin-3-yl]-N-(2-morpholin-4-yl-ethyl)-benzamide;
4-[6-amino-5-(2-chloro-benzyloxy)-pyridin-3-yl]-N-(3-morpholin-4-yl-propyl)-benzamide;
4-[6-amino-5-(2-cyano-benzyloxy)-pyridin-3-yl]-benzoic acid; 2-{2-amino-5-[4-((2R)-2-pyrrolidin-1-ylmethyl-pyrrolidine-1-carbonyl)-phenyl]-pyridin-3-yloxymethyl}-benzonitrile;
2-{2-amino-5-[4-((2S)-2-pyrrolidin-1-ylmethyl-pyrrolidine-1-carbonyl)-phenyl]-pyridin-3-yloxymethyl}-benzonitrile;
2-{2-amino-5-[4-((3S)-3-dimethylamino-pyrrolidine-1-carbonyl)-phenyl]-pyridin-3-yloxymethyl}-benzonitrile;
2-{2-amino-5-[4-((3S)-3-amino-pyrrolidine-1-carbonyl)-phenyl]-pyridin-3-yloxymethyl}-benzonitrile;
2-{2-amino-5-[4-(4-pyrrolidin-1-yl-piperidine-1-carbonyl)-phenyl]-pyridin-3-yloxymethyl}-benzonitrile;
2-{2-amino-5-[4-(4-methyl-piperazine-1-carbonyl)-phenyl]-pyridin-3-yloxymethyl}-benzonitrile;
2-{5-[4-(4-acetyl-piperazine-1-carbonyl)-phenyl]-2-amino-pyridin-3-yloxymethyl}-benzonitrile;
4-[6-amino-5-(2-cyano-benzyloxy)-pyridin-3-yl]-N-(1-methyl-piperidin-4-yl)-benzamide;
4-[6-amino-5-(2-cyano-benzyloxy)-pyridin-3-yl]-N-(2-morpholin-4-yl-ethyl)-benzamide;
4-[6-amino-5-(2-cyano-benzyloxy)-pyridin-3-yl]-N-(3-morpholin-4-yl-propyl)-benzamide;
4-[6-amino-5-(2,4-dichloro-benzyloxy)-pyridin-3-yl]-benzoic acid;
{4-[6-amino-5-(2,4-dichloro-benzyloxy)-pyridin-3-yl]-phenyl}-[(2R)-2-pyrrolidin-1-ylmethyl-pyrrolidin-1-yl]-methanone;
{4-[6-amino-5-(2,4-dichloro-benzyloxy)-pyridin-3-yl]-phenyl}-[(2S)-2-pyrrolidin-1-ylmethyl-pyrrolidin-1-yl]-methanone;
{4-[6-amino-5-(2,4-dichloro-benzyloxy)-pyridin-3-yl]-phenyl}-[(3S)-3-dimethylamino-pyrrolidin-1-yl]-methanone;
{4-[6-amino-5-(2,4-dichloro-benzyloxy)-pyridin-3-yl]-phenyl}-[(3S)-3-amino-pyrrolidin-1-yl]-methanone;
{4-[6-amino-5-(2,4-dichloro-benzyloxy)-pyridin-3-yl]-phenyl}-(4-pyrrolidin-1-yl-piperidin-1-yl)-methanone;
{4-[6-amino-5-(2,4-dichloro-benzyloxy)-pyridin-3-yl]-phenyl}-(4-methyl-piperazin-1-yl)-methanone;
1-(4-{4-[6-amino-5-(2,4-dichloro-benzyloxy)-pyridin-3-yl]-benzoyl}-piperazin-1-yl)-ethanone;
4-[6-amino-5-(2,4-dichloro-benzyloxy)-pyridin-3-yl]-N-(1-methyl-piperidin-4-yl)-benzamide;
4-[6-amino-5-(2,4-dichloro-benzyloxy)-pyridin-3-yl]-N-(2-morpholin-4-yl-ethyl)-benzamide;
4-[6-amino-5-(2,4-dichloro-benzyloxy)-pyridin-3-yl]-N-(3-morpholin-4-yl-propyl)-benzamide;
4-[6-amino-5-(2-trifluoromethyl-benzyloxy)-pyridin-3-yl]-benzoic acid;
{4-[6-amino-5-(2-trifluoromethyl-benzyloxy)-pyridin-3-yl]-phenyl}-[(2R)-2-pyrrolidin-1-ylmethyl-pyrrolidin-1-yl]-methanone;
{4-[6-amino-5-(2-trifluoromethyl-benzyloxy)-pyridin-3-yl]-phenyl}-[(2S)-2-pyrrolidin-1-ylmethyl-pyrrolidin-1-yl]-methanone;
{4-[6-amino-5-(2-trifluoromethyl-benzyloxy)-pyridin-3-yl]-phenyl}-[(3S)-3-dimethylamino-pyrrolidin-1-yl]-methanone;
[(3S)-3-amino-pyrrolidin-1-yl]-{4-[6-amino-5-(2-trifluoromethyl-benzyloxy)-pyridin-3-yl]-phenyl}-methanone;
{4-[6-amino-5-(2-trifluoromethyl-benzyloxy)-pyridin-3-yl]-phenyl}-(4-pyrrolidin-1-yl-piperidin-1-yl)-methanone;
{4-[6-amino-5-(2-trifluoromethyl-benzyloxy)-pyridin-3-yl]-phenyl}-(4-methyl-piperazin-1-yl)-methanone;
1-(4-{4-[6-amino-5-(2-trifluoromethyl-benzyloxy)-pyridin-3-yl]-benzoyl}-piperazin-1-yl)-ethanone;
4-[6-amino-5-(2-trifluoromethyl-benzyloxy)-pyridin-3-yl]-N-(1-methyl-piperidin-4-yl)-benzamide;
4-[6-amino-5-(2-trifluoromethyl-benzyloxy)-pyridin-3-yl]-N-(2-morpholin-4-ylethyl)-benzamide;
4-[6-amino-5-(2-trifluoromethyl-benzyloxy)-pyridin-3-yl]-N-(3-morpholin-4-yl-propyl)-benzamide;
4-[6-amino-5-(4-tert-butyl-benzyloxy)-pyridin-3-yl]-benzoic acid;
{4-[6-amino-5-(4-tert-butyl-benzyloxy)-pyridin-3-yl]-phenyl}-[(2R)-2-pyrrolidin-1-ylmethyl-pyrrolidin-1-yl]-methanone;
{4-[6-amino-5-(4-tert-butyl-benzyloxy)-pyridin-3-yl]-phenyl}-[(2S)-2-pyrrolidin-1-ylmethyl-pyrrolidin-1-yl]-methanone;
{4-[6-amino-5-(4-tert-butyl-benzyloxy)-pyridin-3-yl]-phenyl}-[(3R)-3-dimethylamino-pyrrolidin-1-yl]-methanone;
{4-[6-amino-5-(4-tert-butyl-benzyloxy)-pyridin-3-yl]-phenyl}-(4-methyl-piperazin-1-yl)-methanone;
1-(4-{4-[6-amino-5-(4-tert-butyl-benzyloxy)-pyridin-3-yl]-benzoyl}-piperazin-1-yl)-ethanone;
4-[6-amino-5-(4-tert-butyl-benzyloxy)-pyridin-3-yl]-N-(1-methyl-piperidin-4-yl)-benzamide;
4-[6-amino-5-(4-tert-butyl-benzyloxy)-pyridin-3-yl]-N-(2-morpholin-4-yl-ethyl)-benzamide;
4-[6-amino-5-(4-tert-butyl-benzyloxy)-pyridin-3-yl]-N-(3-morpholin-4-yl-propyl)-benzamide;
4-[6-amino-5-(2-chloro-4-fluoro-benzyloxy)-pyridin-3-yl]-benzoic acid;
{4-[6-amino-5-(2-chloro-4-fluoro-benzyloxy)-pyridin-3-yl]-phenyl}-[(2R)-2-pyrrolidin-1-ylmethyl-pyrrolidin-1-yl]-methanone;
{4-[6-amino-5-(2-chloro-4-fluoro-benzyloxy)-pyridin-3-yl]-phenyl}-[(2S)-2-pyrrolidin-1-ylmethyl-pyrrolidin-1-yl]-methanone;
{4-[6-amino-5-(2-chloro-4-fluoro-benzyloxy)-pyridin-3-yl]-phenyl}-[(3S)-3-dimethylamino-pyrrolidin-1-yl]-methanone;
{4-[6-amino-5-(2-chloro-4-fluoro-benzyloxy)-pyridin-3-yl]-phenyl}-[(3S)-3-amino-pyrrolidin-1-yl]-methanone;
{4-[6-amino-5-(2-chloro-4-fluoro-benzyloxy)-pyridin-3-yl]-phenyl}-(4-methyl-piperazin-1-yl)-methanone;
1-(4-{4-[6-amino-5-(2-chloro-4-fluoro-benzyloxy)-pyridin-3-yl]-benzoyl}-piperazin-1-yl)-ethanone;
4-[6-amino-5-(2-chloro-4-fluoro-benzyloxy)-pyridin-3-yl]-N-(2-morpholin-4-yl-ethyl)-benzamide;
4-[6-amino-5-(2-chloro-4-fluoro-benzyloxy)-pyridin-3-yl]-N-(3-morpholin-4-yl-propyl)-benzamide;
4-[6-amino-5-(2-chloro-3,6-difluoro-benzyloxy)-pyridin-3-yl]-benzoic acid;
{4-[6-amino-5-(2-chloro-3,6-difluoro-benzyloxy)-pyridin-3-yl]-phenyl}-(4-methyl-piperazin-1-yl)-methanone;
{4-[6-amino-5-(2-chloro-3,6-difluoro-benzyloxy)-pyridin-3-yl]-phenyl}-(4-pyrrolidin-1-yl-piperidin-1-yl)-methanone;
{4-[6-amino-5-(2-chloro-3,6-difluoro-benzyloxy)-pyridin-3-yl]-phenyl}-(4-amino-piperidin-1-yl)-methanone;
{4-[6-amino-5-(2-chloro-3,6-difluoro-benzyloxy)-pyridin-3-yl]-phenyl}-(3,5-dimethyl-piperazin-1-yl)-methanone;
{4-[6-amino-5-(2-chloro-3,6-difluoro-benzyloxy)-pyridin-3-yl]-phenyl}-[(2S)-2-pyrrolidin-1-ylmethyl-pyrrolidin-1-yl]-methanone;
{4-[6-amino-5-(2-chloro-3,6-difluoro-benzyloxy)-pyridin-3-yl]-phenyl}-[(3S)-3-dimethylamino-pyrrolidin-1-yl]-methanone;
{4-[6-amino-5-(2-chloro-3,6-difluoro-benzyloxy)-pyridin-3-yl]-phenyl}-[(3R)-3-amino-pyrrolidin-1-yl]-methanone;
{4-[6-amino-5-(2-chloro-3,6-difluoro-benzyloxy)-pyridin-3-yl]-phenyl}-[(3S)-3-amino-pyrrolidin-1-yl]-methanone;
4-[6-amino-5-(2-chloro-3,6-difluoro-benzyloxy)-pyridin-3-yl]-N-(1-methyl-piperidin-4-yl)-benzamide;
4-[6-amino-5-(2-chloro-3,6-difluoro-benzyloxy)-pyridin-3-yl]-N-(2-pyrrolidin-1-yl-ethyl)-benzamide;
4-[6-amino-5-(2-chloro-3,6-difluoro-benzyloxy)-pyridin-3-yl]-N-(3-pyrrolidin-1-yl-propyl)-benzamide;
4-[6-amino-5-(2-chloro-3,6-difluoro-benzyloxy)-pyridin-3-yl]-N-(2-morpholin-4-yl-ethyl)-benzamide;
4-[6-amino-5-(2-chloro-3,6-difluoro-benzyloxy)-pyridin-3-yl]-N-(3-morpholin-4-yl-propyl)-benzamide;
3-[6-amino-5-(2-chloro-3,6-difluoro-benzyloxy)-pyridin-3-yl]-benzoic acid;
{3-[6-amino-5-(2-chloro-3,6-difluoro-benzyloxy)-pyridin-3-yl]-phenyl}-(4-methyl-piperazin-1-yl)-methanone;
{3-[6-amino-5-(2-chloro-3,6-difluoro-benzyloxy)-pyridin-3-yl]-phenyl}-(4-pyrrolidin-1-yl-piperidin-1-yl)-methanone;
{3-[6-amino-5-(2-chloro-3,6-difluoro-benzyloxy)-pyridin-3-yl]-phenyl}-(4-amino-piperidin-1-yl)-methanone;
{3-[6-amino-5-(2-chloro-3,6-difluoro-benzyloxy)-pyridin-3-yl]-phenyl}-(3,5-dimethyl-piperazin-1-yl)-methanone;
{3-[6-amino-5-(2-chloro-3,6-difluoro-benzyloxy)-pyridin-3-yl]-phenyl}-[(2S)-2-pyrrolidin-1-ylmethyl-pyrrolidin-1-yl]-methanone;
{3-[6-amino-5-(2-chloro-3,6-difluoro-benzyloxy)-pyridin-3-yl]-phenyl}-[(3S)-3-dimethylamino-pyrrolidin-1-yl]-methanone;
{3-[6-amino-5-(2-chloro-3,6-difluoro-benzyloxy)-pyridin-3-yl]-phenyl}-[(3R)-3-amino-pyrrolidin-1-yl]-methanone;
{3-[6-amino-5-(2-chloro-3,6-difluoro-benzyloxy)-pyridin-3-yl]-phenyl}-[(3S)-3-amino-pyrrolidin-1-yl]-methanone;
3-[6-amino-5-(2-chloro-3,6-difluoro-benzyloxy)-pyridin-3-yl]-N-(1-methyl-piperidin-4-yl)-benzamide;
3-[6-amino-5-(2-chloro-3,6-difluoro-benzyloxy)-pyridin-3-yl]-N-(2-pyrrolidin-1-yl-ethyl)-benzamide;
3-[6-amino-5-(2-chloro-3,6-difluoro-benzyloxy)-pyridin-3-yl]-N-(3-pyrrolidin-1-yl-propyl)-benzamide;
3-[6-amino-5-(2-chloro-3,6-difluoro-benzyloxy)-pyridin-3-yl]-N-(2-morpholin-4-yl-ethyl)-benzamide;
3-[6-amino-5-(2-chloro-3,6-difluoro-benzyloxy)-pyridin-3-yl]-N-(3-morpholin-4-yl-propyl)-benzamide;
N-[2-(4-acetyl-piperazin-1-yl)-ethyl]-3-[6-amino-5-(2-chloro-3,6-difluoro-benzyloxy)-pyridin-3-yl]-benzamide;
3-(2-chloro-3,6-difluoro-benzyloxy)-5-[4-(1,1-dioxo-1λ⁶-isothiazolidine2-yl)-phenyl]-pyridin-2-ylamine;
3-(2,6-dichloro-benzyloxy)-5-[4-(1,1-dioxo-1λ⁶-isothiazolidine2-yl)-phenyl]-pyridin-2-ylamine;
5-[4-(1,1-dioxo-1λ⁶-isothiazolidine2-yl)-phenyl]-3-(2-fluoro-6-trifluoromethyl-benzyloxy)-pyridin-2-ylamine;
2-diethylamino-ethanesulfonic acid {4-[6-amino-5-(2-chloro-3,6-difluoro-benzyloxy)-pyridin-3-yl]-phenyl}-amide;
2-cyclopropylamino-ethanesulfonic acid {4-[6-amino-5-(2-chloro-3,6-difluoro-benzyloxy)-pyridin-3-yl]-phenyl}-amide;
2-pyrrolidin-1-yl-ethanesulfonic acid {4-[6-amino-5-(2-chloro-3,6-difluoro-benzyloxy)-pyridin-3-yl]-phenyl}-amide
; 2-(4-hydroxy-piperidin-1-yl)-ethanesulfonic acid {4-[6-amino-5-(2-chloro-3,6-difluoro-benzyloxy)-pyridin-3-yl]-phenyl}-amide;
2-morpholin-4-yl-ethanesulfonic acid {4-[6-amino-5-(2-chloro-3,6-difluoro-benzyloxy)-pyridin-3-yl]-phenyl}-amide;
2-piperidin-1-yl-ethanesulfonic acid {4-[6-amino-5-(2-chloro-3,6-difluoro-benzyloxy)-pyridin-3-yl]-phenyl}-amide;
2-dimethylamino-ethanesulfonic acid {4-[6-amino-5-(2-chloro-3,6-difluoro-benzyloxy)-pyridin-3-yl]-phenyl}-amide;
2-(4-acetyl-piperazin-1-yl)-ethanesulfonic acid {4-[6-amino-5-(2-chloro-3,6-difluoro-benzyloxy)-pyridin-3-yl]-phenyl}-amide;
2-(cyclopropylmethyl-amino)-ethanesulfonic acid {4-[6-amino-5-(2-chloro-3,6-difluoro-benzyloxy)-pyridin-3-yl]-phenyl}-amide;
2-[(3R)-3-hydroxy-pyrrolidin-1-yl]-ethanesulfonic acid {4-[6-amino-5-(2-chloro-3,6-difluoro-benzyloxy)-pyridin-3-yl]-phenyl}-amide;
2-[(2S)-2-hydroxymethyl-pyrrolidin-1-yl]-ethanesulfonic acid {4-[6-amino-5-(2-chloro-3,6-difluoro-benzyloxy)-pyridin-3-yl]-phenyl}-amide;
2-[4-(2-hydroxy-acetyl)-piperazin-1-yl]-ethanesulfonic acid {4-[6-amino-5-(2-chloro-3,6-difluoro-benzyloxy)-pyridin-3-yl]-phenyl}-amide;
2-(4-acetyl-piperazin-1-yl)-ethanesulfonic acid {3-[6-amino-5-(2-chloro-3,6-difluoro-benzyloxy)-pyridin-3-yl]-phenyl}-amide;
2-pyrrolidin-1-yl-ethanesulfonic acid {3-[6-amino-5-(2-chloro-3,6-difluoro-benzyloxy)-pyridin-3-yl]-phenyl}-amide;
2-morpholin-4-yl-ethanesulfonic acid {3-[6-amino-5-(2-chloro-3,6-difluoro-benzyloxy)-pyridin-3-yl]-phenyl}-amide;
2-diethylamino-ethanesulfonic acid {3-[6-amino-5-(2-chloro-3,6-difluoro-benzyloxy)-pyridin-3-yl]-phenyl}-amide;
2-dimethylamino-ethanesulfonic acid {3-[6-amino-5-(2-chloro-3,6-difluoro-benzyloxy)-pyridin-3-yl]-phenyl}-amide;
2-piperidin-1-yl-ethanesulfonic acid {3-[6-amino-5-(2-chloro-3,6-difluoro-benzyloxy)-pyridin-3-yl]-phenyl}-amide;
2-[(3R)-3-hydroxymethyl-pyrrolidin-1-yl]-ethanesulfonic acid {3-[6-amino-5-(2-chloro-3,6-difluoro-benzyloxy)-pyridin-3-yl]-phenyl}-amide;
2-(4-hydroxy-piperidin-1-yl)-ethanesulfonic acid {3-[6-amino-5-(2-chloro-3,6-difluoro-benzyloxy)-pyridin-3-yl]-phenyl}-amide;
2-[4-(2-hydroxy-acetyl)-piperazin-1-yl]-ethanesulfonic acid {3-[6-amino-5-(2-chloro-3,6-difluoro-benzyloxy)-pyridin-3-yl]-phenyl}-amide;
2-[(3R)-3-hydroxy-pyrrolidin-1-yl]-ethanesulfonic acid {3-[6-amino-5-(2-chloro-3,6-difluoro-benzyloxy)-pyridin-3-yl]-phenyl}-amide;
2-(cyclopropylmethyl-amino)-ethanesulfonic acid {3-[6-amino-5-(2-chloro-3,6-difluoro-benzyloxy)-pyridin-3-yl]-phenyl}-amide;
2-cyclopropylamino-ethanesulfonic acid {3-[6-amino-5-(2-chloro-3,6-difluoro-benzyloxy)-pyridin-3-yl]-phenyl}-amide; 3-(2-chloro-3,6-difluoro-benzyloxy)-5-(2-dimethylaminomethyl-phenyl)-pyridin-2-ylamine (compound with trifluoroacetic acid);
3-(2-chloro-3,6-difluoro-benzyloxy)-5-(3-pyrrolidin-1-yl-phenyl)-pyridin-2-ylamine (compound with trifluoroacetic acid);
N-{4-[6-amino-5-(2-chloro-3,6-difluoro-benzyloxy)-pyridin-3-yl]-phenyl}-methanesulfonamide (compound with trifluoroacetic acid);
5-[6-amino-5-(2-chloro-3,6-difluoro-benzyloxy)-pyridin-3-yl]-thiophene-2-carboxylic acid;
{5-[6-amino-5-(2-chloro-3,6-difluoro-benzyloxy)-pyridin-3-yl]-thiophen-2-yl}-(4-methyl-piperazin-1-yl)-methanone;
{5-[6-amino-5-(2-chloro-3,6-difluoro-benzyloxy)-pyridin-3-yl]-thiophen-2-yl}-[(2R)-2-pyrrolidin-1-ylmethyl-pyrrolidin-1-yl]methanone;
5-[6-amino-5-(2-chloro-3,6-difluoro-benzyloxy)-pyridin-3-yl]-thiophene-2-carboxylic acid (1-methyl-piperidin-4-yl)-amide;
{5-[6-amino-5-(2-chloro-3,6-difluoro-benzyloxy)-pyridin-3-yl]-thiophen-2-yl}-(3,5-dimethyl-piperazin-1-yl)-methanone;
5-[6-amino-5-(2-chloro-3,6-difluoro-benzyloxy)-pyridin-3-yl]-thiophene-2-carboxylic acid (2-pyrrolidin-1-yl-ethyl)-amide;
{5-[6-amino-5-(2-chloro-3,6-difluoro-benzyloxy)-pyridin-3-yl]-thiophen-2-yl}-(4-pyrrolidin-1-yl-piperidin-1-yl)-methanone;
4-[6-amino-5-(3-fluoro-2-trifluoromethyl-benzyloxy)-pyridin-3-yl]-benzoic acid;
{4-[6-amino-5-(3-fluoro-2-trifluoromethyl-benzyloxy)-pyridin-3-yl]-phenyl}-(4-pyrrolidin-1-yl-piperidin-1-yl)-methanone;
4-[6-amino-5-(3-fluoro-2-trifluoromethyl-benzyloxy)-pyridin-3-yl]-N-(1-methyl-piperidin-4-yl)-benzamide;
{4-[6-amino-5-(3-fluoro-2-trifluoromethyl-benzyloxy)-pyridin-3-yl]-phenyl}-(3,5-dimethyl-piperazin-1-yl)-methanone;
{4-[6-amino-5-(3-fluoro-2-trifluoromethyl-benzyloxy)-pyridin-3-yl]-phenyl}-(3-dimethylamino-pyrrolidin-1-yl)-methanone;
{4-[6-amino-5-(3-fluoro-2-trifluoromethyl-benzyloxy)-pyridin-3-yl]-phenyl}-[(2S)-2-pyrrolidin-1-ylmethyl-pyrrolidin-1-yl]-methanone;
4-[6-amino-5-(3-fluoro-2-trifluoromethyl-benzyloxy)-pyridin-3-yl]-N-(2-morpholin-4-yl-ethyl)-benzamide;
{4-[6-amino-5-(3-fluoro-2-trifluoromethyl-benzyloxy)-pyridin-3-yl]-phenyl}-(4-methyl-piperazin-1-yl)-methanone;
N-[2-(4-acetyl-piperazin-1-yl)-ethyl]-4-[6-amino-5-(3-fluoro-2-trifluoromethyl-benzyloxy)-pyridin-3-yl]benzamide;
2-piperidin-1-yl-ethanesulfonic acid (4-{6-amino-5-[1-(2-chloro-3,6-difluoro-phenyl)-ethoxy]-pyridin-3-yl}-phenyl)-amide;
2-(4-hydroxy-piperidin-1-yl)-ethanesulfonic acid (4-{6-amino-5-[1-(2-chloro-3,6-difluoro-phenyl)-ethoxy]-pyridin-3-yl}-phenyl)-amide;
2-dimethylamino-ethanesulfonic acid (4-{6-amino-5-[1-(2-chloro-3,6-difluoro-phenyl)-ethoxy]-pyridin-3-yl}-phenyl)-amide;
2-cyclopropylamino-ethanesulfonic acid (4-{6-amino-5-[1-(2-chloro-3,6-difluoro-phenyl)-ethoxy]-pyridin-3-yl}-phenyl)-amide;
4-{6-amino-5-[1-(2,6-dichloro-3-fluoro-phenyl)-ethoxy]-pyridin-3-yl}-benzoic acid;
(4-{6-amino-5-[1-(2,6-dichloro-3-fluoro-phenyl)-ethoxy]-pyridin-3-yl}-phenyl)-[(2R)-2-pyrrolidin-1-ylmethyl-pyrrolidin-1-yl]-methanone;
4-{6-amino-5-[1-(2,6-dichloro-3-fluoro-phenyl)-ethoxy]-pyridin-3-yl}-N-(1-methyl-piperidin-4-yl)-benzamide;
(3-{6-amino-5-[1-(2,6-dichloro-3-fluoro-phenyl)-ethoxy]-pyridin-3-yl}-phenyl)-[(3R)-3-amino-pyrrolidin-1-yl]-methanone; (4-{6-amino-5-[1-(2,6-dichloro-3-fluoro-phenyl)-ethoxy]-pyridin-3-yl}-phenyl)-(4-pyrrolidin-1-yl-piperidin-1-yl)-methanone;
(4-{6-amino-5-[1-(2,6-dichloro-3-fluoro-phenyl)-ethoxy]-pyridin-3-yl}-phenyl)-(4-methyl-piperazin-1-yl)-methanone;
(4-{6-amino-5-[1-(2,6-dichloro-3-fluoro-phenyl)-ethoxy]-pyridin-3-yl}-phenyl)-(3,5-dimethyl-piperazin-1-yl)-methanone;
2-cyclopropylamino-ethanesulfonic acid (4-{6-amino-5-[1-(2,6-dichloro-3-fluoro-phenyl)-ethoxy]-pyridin-3-yl}-phenyl)-amide;
2-dimethylamino-ethanesulfonic acid (4-{6-amino-5-[1-(2,6-dichloro-3-fluoro-phenyl)-ethoxy]-pyridin-3-yl}-phenyl)-amide;
2-[(3R)-3-hydroxy-pyrrolidin-1-yl]-ethanesulfonic acid (4-{6-amino-5-[1-(2,6-dichloro-3-fluoro-phenyl)-ethoxy]-pyridin-3-yl}-phenyl)-amide;
4-[5-amino-6-(2,6-dichloro-benzyloxy)-pyrazin-2-yl]-phenol;
3-(2,6-dichloro-benzyloxy)-5-[4-(1,1-dioxo-1λ⁶-isothiazolidine2-yl)-phenyl]-pyrazin-2-ylamine;
3-(2,6-dichloro-benzyloxy)-5-[3-(2-morpholin-4-yl-ethoxy)-phenyl]-pyrazin-2-ylamine;
3-(2,6-dichloro-benzyloxy)-5-[4-(2-morpholin-4-yl-ethoxy)-phenyl]-pyrazin-2-ylamine;
5-(4-amino-phenyl)-3-(2,6-dichloro-benzyloxy)-pyrazin-2-ylamine;
4-[5-amino-6-(2,6-dichloro-benzyloxy)-pyrazin-2-yl]-benzoic acid;
{4-[5-amino-6-(2,6-dichloro-benzyloxy)-pyrazin-2-yl]-phenyl}-[(2R)-2-pyrrolidin-1-ylmethyl-pyrrolidin-1-yl]-methanone;
{4-[5-amino-6-(2,6-dichloro-benzyloxy)-pyrazin-2-yl]-phenyl}-(4-pyrrolidin-1-yl-piperidin-1-yl)-methanone;
2-morpholin-4-yl-ethanesulfonic acid {4-[5-amino-6-(2-chloro-3,6-difluoro-benzyloxy)-pyrazin-2-yl]-phenyl}-amide;
2-piperidin-1-yl-ethanesulfonic acid {4-[5-amino-6-(2-chloro-3,6-difluoro-benzyloxy)-pyrazin-2-yl]-phenyl}-amide;
2-(4-hydroxy-piperidin-1-yl)-ethanesulfonic acid {4-[5-amino-6-(2-chloro-3,6-difluoro-benzyloxy)-pyrazin-2-yl]-phenyl}-amide;
2-pyrrolidin-1-yl-ethanesulfonic acid {4-[5-amino-6-(2-chloro-3,6-difluoro-benzyloxy)-pyrazin-2-yl]-phenyl}-amide;
2-[(3R)-3-hydroxy-pyrrolidin-1-yl]-ethanesulfonic acid {4-[5-amino-6-(2-chloro-3,6-difluoro-benzyloxy)-pyrazin-2-yl]-phenyl}-amide;
2-[(2S)-2-hydroxymethyl-pyrrolidin-1-yl]-ethanesulfonic acid {4-[5-amino-6-(2-chloro-3,6-difluoro-benzyloxy)-pyrazin-2-yl]-phenyl}-amide;
2-(cyclopropylmethyl-amino)-ethanesulfonic acid {4-[5-amino-6-(2-chloro-3,6-difluoro-benzyloxy)-pyrazin-2-yl]-phenyl}-amide;
2-dimethylamino-ethanesulfonic acid {4-[5-amino-6-(2-chloro-3,6-difluoro-benzyloxy)-pyrazin-2-yl]-phenyl}-amide;
2-diethylamino-ethanesulfonic acid {4-[5-amino-6-(2-chloro-3,6-difluoro-benzyloxy)-pyrazin-2-yl]-phenyl}-amide;
2-(4-acetyl-piperazin-1-yl)-ethanesulfonic acid {4-[5-amino-6-(2-chloro-3,6-difluoro-benzyloxy)-pyrazin-2-yl]-phenyl}-amide;
2-[4-(2-hydroxy-acetyl)-piperazin-1-yl]-ethanesulfonic acid {4-[5-amino-6-(2-chloro-3,6-difluoro-benzyloxy)-pyrazin-2-yl]-phenyl}-amide;
2-cyclopropylamino-ethanesulfonic acid {4-[5-amino-6-(2-chloro-3,6-difluoro-benzyloxy)-pyrazin-2-yl]-phenyl}-amide;
2-[(3R)-3-hydroxymethyl-pyrrolidin-1-yl]-ethanesulfonic acid {3-[5-amino-6-(2-chloro-3,6-difluoro-benzyloxy)-pyrazin-2-yl]-phenyl}-amide;
2-(4-hydroxy-piperidin-1-yl)-ethanesulfonic acid {3-[5-amino-6-(2-chloro-3,6-difluoro-benzyloxy)-pyrazin-2-yl]-phenyl}-amide;
2-(4-acetyl-piperazin-1-yl)-ethanesulfonic acid {3-[5-amino-6-(2-chloro-3,6-difluoro-benzyloxy)-pyrazin-2-yl]-phenyl}-amide;
2-piperidin-1-yl-ethanesulfonic acid {3-[5-amino-6-(2-chloro-3,6-difluoro-benzyloxy)-pyrazin-2-yl]-phenyl}-amide;
2-diethylamino-ethanesulfonic acid {3-[5-amino-6-(2-chloro-3,6-difluoro-benzyloxy)-pyrazin-2-yl]-phenyl}-amide;
2-morpholin-4-yl-ethanesulfonic acid {3-[5-amino-6-(2-chloro-3,6-difluoro-benzyloxy)-pyrazin-2-yl]-phenyl}-amide;
2-pyrrolidin-1-yl-ethanesulfonic acid {3-[5-amino-6-(2-chloro-3,6-difluoro-benzyloxy)-pyrazin-2-yl]-phenyl}-amide;
2-dimethylamino-ethanesulfonic acid {3-[5-amino-6-(2-chloro-3,6-difluoro-benzyloxy)-pyrazin-2-yl]-phenyl}-amide;
2-[4-(2-hydroxy-acetyl)-piperazin-1-yl]-ethanesulfonic acid {3-[5-amino-6-(2-chloro-3,6-difluoro-benzyloxy)-pyrazin-2-yl]-phenyl}-amide;
2-(cyclopropylmethyl-amino)-ethanesulfonic acid {3-[5-amino-6-(2-chloro-3,6-difluoro-benzyloxy)-pyrazin-2-yl]-phenyl}-amide;
2-[(3R)-3-hydroxy-pyrrolidin-1-yl]-ethanesulfonic acid {3-[5-amino-6-(2-chloro-3,6-difluoro-benzyloxy)-pyrazin-2-yl]-phenyl}-amide;
2-cyclopropylamino-ethanesulfonic acid {3-[5-amino-6-(2-chloro-3,6-difluoro-benzyloxy)-pyrazin-2-yl]-phenyl}-amide;
4-[5-amino-6-(2-chloro-3,6-difluoro-benzyloxy)-pyrazin-2-yl]-benzoic acid;
{4-[5-amino-6-(2-chloro-3,6-difluoro-benzyloxy)-pyrazin-2-yl]-phenyl}-[(2R)-2-pyrrolidin-1-ylmethyl-pyrrolidin-1-yl]-methanone;
4-[5-amino-6-(2-chloro-3,6-difluoro-benzyloxy)-pyrazin-2-yl]-N-(2-pyrrolidin-1-yl-ethyl)-benzamide;
{4-[5-amino-6-(2-chloro-3,6-difluoro-benzyloxy)-pyrazin-2-yl]-phenyl}-[(3S)-3-amino-pyrrolidin-1-yl]-methanone;
N-[2-(4-acetyl-piperazin-1-yl)-ethyl]-4-[5-amino-6-(2-chloro-3,6-difluoro-benzyloxy)-pyrazin-2-yl]-benzamide;
4-[5-amino-6-(2-chloro-3,6-difluoro-benzyloxy)-pyrazin-2-yl]-N-(3-pyrrolidin-1-yl-propyl)-benzamide;
{4-[5-amino-6-(2-chloro-3,6-difluoro-benzyloxy)-pyrazin-2-yl]-phenyl}-[(3S)-3-dimethylamino-pyrrolidin-1-yl]-methanone;
{4-[5-amino-6-(2-chloro-3,6-difluoro-benzyloxy)-pyrazin-2-yl]-phenyl}-[(3R)-3-dimethylamino-pyrrolidin-1-yl]-methanone;
{4-[5-amino-6-(2-chloro-3,6-difluoro-benzyloxy)-pyrazin-2-yl]-phenyl}-(3,5-dimethyl-piperazin-1-yl)-methanone;
{4-[5-amino-6-(2-chloro-3,6-difluoro-benzyloxy)-pyrazin-2-yl]-phenyl}-(4-pyrrolidin-1-yl-piperidin-1-yl)-methanone;
4-[5-amino-6-(2-chloro-3,6-difluoro-benzyloxy)-pyrazin-2-yl]-N-(3-morpholin-4-yl-propyl)-benzamide;
4-[5-amino-6-(2-chloro-3,6-difluoro-benzyloxy)-pyrazin-2-yl]-N-(1-methyl-piperidin-4-yl)-benzamide;
4-[5-amino-6-(2-chloro-3,6-difluoro-benzyloxy)-pyrazin-2-yl]-N-(2-morpholin-4-yl-ethyl)-benzamide;
{4-[5-amino-6-(2-chloro-3,6-difluoro-benzyloxy)-pyrazin-2-yl]-phenyl}-(4-methyl-piperazin-1-yl)-methanone;
3-[5-amino-6-(2-chloro-3,6-difluoro-benzyloxy)-pyrazin-2-yl]-benzoic acid;
{3-[5-amino-6-(2-chloro-3,6-difluoro-benzyloxy)-pyrazin-2-yl]-phenyl}-(4-methyl-piperazin-1-yl)-methanone;
{3-[5-amino-6-(2-chloro-3,6-difluoro-benzyloxy)-pyrazin-2-yl]-phenyl}-[(3R)-3-amino-pyrrolidin-1-yl]-methanone;
{3-[5-amino-6-(2-chloro-3,6-difluoro-benzyloxy)-pyrazin-2-yl]-phenyl}-[(3S)-3-amino-pyrrolidin-1-yl]-methanone;
{3-[5-amino-6-(2-chloro-3,6-difluoro-benzyloxy)-pyrazin-2-yl]-phenyl}-(3,5-dimethyl-piperazin-1-yl)-methanone;
3-[5-amino-6-(2-chloro-3,6-difluoro-benzyloxy)-pyrazin-2-yl]-N-(3-morpholin-4-yl-propyl)-benzamide;
{3-[5-amino-6-(2-chloro-3,6-difluoro-benzyloxy)-pyrazin-2-yl]-phenyl}-(4-pyrrolidin-1-yl-piperidin-1-yl)-methanone;
{3-[5-amino-6-(2-chloro-3,6-difluoro-benzyloxy)-pyrazin-2-yl]-phenyl}-[(3S)-3-dimethylamino-pyrrolidin-1-yl]-methanone;
3-[5-amino-6-(2-chloro-3,6-difluoro-benzyloxy)-pyrazin-2-yl]-N-(2-pyrrolidin-1-yl-ethyl)-benzamide;
3-[5-amino-6-(2-chloro-3,6-difluoro-benzyloxy)-pyrazin-2-yl]-N-(1-methyl-piperidin-4-yl)-benzamide;
{3-[5-amino-6-(2-chloro-3,6-difluoro-benzyloxy)-pyrazin-2-yl]-phenyl}-[(2S)-pyrrolidin-1-ylmethyl-pyrrolidin-1-yl]-methanone;
3-[5-amino-6-(2-chloro-3,6-difluoro-benzyloxy)-pyrazin-2-yl]-N-(2-morpholin-4-yl-ethyl)-benzamide;
N-[2-(4-acetyl-piperazin-1-yl)-ethyl]-3-[5-amino-6-(2-chloro-3,6-difluoro-benzyloxy)-pyrazin-2-yl]-benzamide;
3-[5-amino-6-(2-chloro-3,6-difluoro-benzyloxy)-pyrazin-2-yl]-N-(3-pyrrolidin-1-yl-propyl)-benzamide;
3-(2-chloro-3,6-difluoro-benzyloxy)-5-(1H-indol-5-yl)-pyrazin-2-ylamine;
3-(2-chloro-3,6-difluoro-benzyloxy)-5-(3-pyrrolidin-1-ylmethyl-1H-indol-5-yl)-pyrazin-2-ylamine;
3-(2-chloro-3,6-difluoro-benzyloxy)-5-(3-diethylaminomethyl-1H-indol-5-yl)-pyrazin-2-ylamine;
1-(4-{5-[5-amino-6-(2-chloro-3,6-difluoro-benzyloxy)-pyrazin-2-yl]-1H-indol-3-ylmethyl}-piperazin-1-yl)-ethanone;
3-(2-chloro-3,6-difluoro-benzyloxy)-5-[3-(2,6-dimethyl-morpholin-4-ylmethyl)-1H-indol-5-yl]-pyrazin-2-ylamine;
N-(1-{5-[5-amino-6-(2-chloro-3,6-difluoro-benzyloxy)-pyrazin-2-yl]-1H-indol-3-ylmethyl}-(3S)-pyrrolidin-3-yl)-acetamide;
3-(2-chloro-3,6-difluoro-benzyloxy)-5-(3-piperidin-1-ylmethyl-1H-indol-5-yl)-pyrazin-2-ylamine;
3-(2-chloro-3,6-difluoro-benzyloxy)-5-(3-morpholin-4-ylmethyl-1H-indol-5-yl)-pyrazin-2-ylamine;
3-[1-(2-chloro-3,6-difluoro-phenyl)-2-methyl-propoxy]-5-[4-(2-morpholin-4-yl-ethoxy)-phenyl]-pyrazin-2-ylamine;
3-[1-(2-chloro-3,6-difluoro-phenyl)-ethoxy-5-[4-(2-morpholin-4-yl-ethoxy)-phenyl]-pyrazin-2-ylamine (compound with trifluoroacetic acid);
3-[1-(2,6-dichloro-3-fluoro-phenyl)-ethoxy]-5-[4-(2-morpholin-4-yl-ethoxy)-phenyl]-pyrazin-2-ylamine (compound with trifluoroacetic acid);
N-(4-{5-amino-6-[1-(2-chloro-3,6-difluoro-phenyl)-ethoxy]-pyrazin-2-yl}-phenyl)-methanesulfonamide;
2-pyrrolidin-1-yl-ethanesulfonic acid (4-{5-amino-6-[1-(2-chloro-3,6-difluoro-phenyl)-ethoxy]-pyrazin-2-yl}-phenyl)-amide;
2-(4-hydroxy-piperidin-1-yl)-ethanesulfonic acid (4-{5-amino-6-[1-(2-chloro-3,6-difluoro-phenyl)-ethoxy]-pyrazin-2-yl}-phenyl)-amide;
2-piperidin-1-yl-ethanesulfonic acid (4-{5-amino-6-[1-(2-chloro-3,6-difluoro-phenyl)-ethoxy]-pyrazin-2-yl}-phenyl)-amide;
2-(cyclopropylmethyl-amino)-ethanesulfonic acid (4-{5-amino-6-[1-(2-chloro-3,6-difluoro-phenyl)-ethoxy]-pyrazin-2-yl}-phenyl)-amide;
2-[(3R)-3-hydroxy-pyrrolidin-1-yl]-ethanesulfonic acid (4-{5-amino-6-[1-(2-chloro-3,6-difluoro-phenyl)-ethoxy]-pyrazin-2-yl}-phenyl)-amide;
2-[(2S)-2-hydroxymethyl-pyrrolidin-1-yl]-ethanesulfonic acid (4-{5-amino-6-[1-(2-chloro-3,6-difluoro-phenyl)-ethoxy]-pyrazin-2-yl}-phenyl)-amide;
2-dimethylamino-ethanesulfonic acid (4-{5-amino-6-[1-(2-chloro-3,6-difluoro-phenyl)-ethoxy]-pyrazin-2-yl}-phenyl)-amide;
2-morpholin-4-yl-ethanesulfonic acid (4-{5-amino-6-[1-(2-chloro-3,6-difluoro-phenyl)-ethoxy]-pyrazin-2-yl}-phenyl)-amide;
2-diethylamino-ethanesulfonic acid (4-{5-amino-6-[1-(2-chloro-3,6-difluoro-phenyl)-ethoxy]-pyrazin-2-yl}-phenyl)-amide;
2-cyclopropylamino-ethanesulfonic acid (4-{5-amino-6-[1-(2-chloro-3,6-difluoro-phenyl)-ethoxy]-pyrazin-2-yl}-phenyl)-amide;
3-{5-amino-6-[1-(2,6-dichloro-3-fluoro-phenyl)-ethoxy]-pyrazin-2-yl}-benzoic acid;
(3-{5-amino-6-[1-(2,6-dichloro-3-fluoro-phenyl)-ethoxy]-pyrazin-2-yl}-phenyl)-[(3S)-3-amino-pyrrolidin-1-yl]-methanone;
(3-{5-amino-6-[1-(2,6-dichloro-3-fluoro-phenyl)-ethoxy]-pyrazin-2-yl}-phenyl)-[(3R)-3-amino-pyrrolidin-1-yl]-methanone;
(3-{5-amino-6-[1-(2,6-dichloro-3-fluoro-phenyl)-ethoxy]-pyrazin-2-yl}-phenyl)-[(2R)-2-pyrrolidin-1-ylmethyl-pyrrolidin-1-yl]-methanone;
N-[2-(4-acetyl-piperazin-1-yl)-ethyl]-3-{5-amino-6-[1-(2,6-dichloro-3-fluoro-phenyl)-ethoxy]-pyrazin-2-yl}-benzamide;
(3-{5-amino-6-[1-(2,6-dichloro-3-fluoro-phenyl)-ethoxy]-pyrazin-2-yl}-phenyl)-[(2S)-2-pyrrolidin-1-ylmethyl-pyrrolidin-1-yl]-methanone;
3-{5-amino-6-[1-(2-chloro-3,6-difluoro-phenyl)-ethoxy]-pyrazin-2-yl}-benzoic acid;
3-{5-amino-6-[1-(2,6-dichloro-3-fluoro-phenyl)-ethoxy]-pyrazin-2-yl}-N-(1-methyl-piperidin-4-yl)-benzamide;
3-{5-amino-6-[1-(2,6-dichloro-3-fluoro-phenyl)-ethoxy]-pyrazin-2-yl}-N-(3-pyrrolidin-1-yl-propyl)-benzamide;
(3-{5-amino-6-[1-(2,6-dichloro-3-fluoro-phenyl)-ethoxy]-pyrazin-2-yl}-phenyl]-(4-pyrrolidin-1-yl-piperidin-1-yl)-methanone;
4-[5-amino-6-(3-fluoro-2-trifluoromethyl-benzyloxy)-pyrazin-2-yl]-benzoic acid;
4-[5-amino-6-(3-fluoro-2-trifluoromethyl-benzyloxy)-pyrazin-2-yl]-N-(2-morpholin-4-yl-ethyl)-benzamide;
4-[5-amino-6-(3-fluoro-2-trifluoromethyl-benzyloxy)-pyrazin-2-yl]-N-(1-methyl-piperidin-4-yl)-benzamide;
(4-{6-amino-5-[1-(2,6-dichloro-3-fluoro-phenyl)-ethoxy]-pyridin-3-yl}-phenyl)-(4-methyl-piperazin-1-yl)-methanone;
N-[2-(4-acetyl-piperazin-1-yl)-ethyl]-4-{6-amino-5-[1-(2,6-dichloro-3-fluoro-phenyl)-ethoxy]-pyridin-3-yl}-benzamide;
4-{6-amino-5-[1-(2,6-dichloro-3-fluoro-phenyl)-ethoxy]-pyridin-3-yl}-N-(3-pyrrolidin-1-yl-propyl)-benzamide;
4-{6-amino-5-[1-(2,6-dichloro-3-fluoro-phenyl)-ethoxy]-pyridin-3-yl}-N-(2-morpholin-4-yl-ethyl)-benzamide;
(4-{6-amino-5-[1-(2,6-dichloro-3-fluoro-phenyl)-ethoxy]-pyridin-3-yl}-phenyl)-((S)-3-amino-pyrrolidin-1-yl)-methanone;
(4-{6-amino-5-[1-(2,6-dichloro-3-fluoro-phenyl)-ethoxy]-pyridin-3-yl}-phenyl)-((R)-3-amino-pyrrolidin-1-yl)-methanone;
(4-{6-amino-5-[1-(2,6-dichloro-3-fluoro-phenyl)-ethoxy]-pyridin-3-yl}-phenyl)-(4-amino-piperidin-1-yl)-methanone;
(4-{6-amino-5-[1-(2,6-dichloro-3-fluoro-phenyl)-ethoxy]-pyridin-3-yl}-phenyl)-((S)-3-hydroxy-pyrrolidin-1-yl)-methanone;
(4-{6-amino-5-[1-(2,6-dichloro-3-fluoro-phenyl)-ethoxy]-pyridin-3-yl}-phenyl)-((R)-3-hydroxy-pyrrolidin-1-yl)-methanone;
(4-{6-amino-5-[1-(2,6-dichloro-3-fluoro-phenyl)-ethoxy]-pyridin-3-yl}-phenyl)-((R)-2-hydroxymethyl-pyrrolidin-1-yl)-methanone;
4-{6-amino-5-[1-(2,6-dichloro-3-fluoro-phenyl)-ethoxy]-pyridin-3-yl}-N-(2-diethylamino-ethyl)-benzamide;
4-{6-amino-5-[1-(2,6-dichloro-3-fluoro-phenyl)-ethoxy]-pyridin-3-yl}-N-(2-pyrrolidin-1-yl-ethyl)-benzamide;
3-{6-amino-5-[1-(2,6-dichloro-3-fluoro-phenyl)-ethoxy]-pyridin-3-yl}-benzoic acid;
(3-{6-amino-5-[1-(2,6-dichloro-3-fluoro-phenyl)-ethoxy]-pyridin-3-yl}-phenyl)-(4-methyl-piperazin-1-yl)-methanone;
3-{6-amino-5-[1-(2,6-dichloro-3-fluoro-phenyl)-ethoxy]-pyridin-3-yl}-N-(1-methyl-piperidin-4-yl)-benzamide;
(3-{6-amino-5-[1-(2,6-dichloro-3-fluoro-phenyl)-ethoxy]-pyridin-3-yl}-phenyl)-((S)-2-pyrrolidin-1-ylmethyl-pyrrolidin-1-yl)-methanone;
N-[2-(4-acetyl-piperazin-1-yl)-ethyl]-3-{6-amino-5-[1-(2,6-dichloro-3-fluoro-phenyl)-ethoxy]-pyridin-3-yl}-benzamide;
(3-{6-amino-5-[1-(2,6-dichloro-3-fluoro-phenyl)-ethoxy]-pyridin-3-yl}-phenyl)-((S)-3-amino-pyrrolidin-1-yl)-methanone;
3-{6-amino-5-[1-(2,6-dichloro-3-fluoro-phenyl)-ethoxy]-pyridin-3-yl}-N-(3-morpholin-4-yl-propyl)-benzamide;
(3-{6-amino-5-[1-(2,6-dichloro-3-fluoro-phenyl)-ethoxy]-pyridin-3-yl}-phenyl)-((R)-2-pyrrolidin-1-ylmethyl-pyrrolidin-1-yl)-methanone;
3-{6-amino-5-[1-(2,6-dichloro-3-fluoro-phenyl)-ethoxy]-pyridin-3-yl}-N-(2-pyrrolidin-1-yl-ethyl)-benzamide;
3-{6-amino-5-[1-(2,6-dichloro-3-fluoro-phenyl)-ethoxy]-pyridin-3-yl}-N-(3-pyrrolidin-1-yl-propyl)-benzamide;
3-{6-amino-5-[1-(2,6-dichloro-3-fluoro-phenyl)-ethoxy]-pyridin-3-yl}-N-(2-morpholin-4-yl-ethyl)-benzamide;
(3-{6-amino-5-[1-(2,6-dichloro-3-fluoro-phenyl)-ethoxy]-pyridin-3-yl}-phenyl)-(4-pyrrolidin-1-yl-piperidin-1-yl)-methanone;
2-diethylamino-ethanesulfonic acid (4-{6-amino-5-[1-(2,6-dichloro-3-fluoro-phenyl)-ethoxy]-pyridin-3-yl}-phenyl)-amide;
2-(4-hydroxy-piperidin-1-yl)-ethanesulfonic acid (4-{6-amino-5-[1-(2,6-dichloro-3-fluoro-phenyl)-ethoxy]-pyridin-3-yl}-phenyl)-amide;
2-piperidin-1-yl-ethanesulfonic acid (4-{6-amino-5-[1-(2,6-dichloro-3-fluoro-phenyl)-ethoxy]-pyridin-3-yl}-phenyl)-amide;
2-(cyclopropylmethyl)-amino-ethanesulfonic acid (4-{6-amino-5-[1-(2,6-dichloro-3-fluoro-phenyl)-ethoxy]-pyridin-3-yl}-phenyl)-amide;
2-((R)-3-hydroxy-pyrrolidin-1-yl)-ethanesulfonic acid (4-{6-amino-5-[1-(2-chloro-3,6-difluoro-phenyl)-ethoxy]-pyridin-3-yl}-phenyl)-amide;
2-cyclopropylamino-ethanesulfonic acid (4-{6-amino-5-[1-(2-chloro-3,6-difluoro-phenyl)-ethoxy]-pyridin-3-yl}-phenyl)-amide;
2-diethylamino-ethanesulfonic acid (4-{6-amino-5-[1-(2-chloro-3,6-difluoro-phenyl)-ethoxy]-pyridin-3-yl}-phenyl)-amide;
4-{6-amino-5-[1-(2-chloro-3,6-difluoro-phenyl)-ethoxy]-pyridin-3-yl}-benzoic acid;
4-{6-amino-5-[1-(2-chloro-3,6-difluoro-phenyl)-ethoxy]-pyridin-3-yl}-N-(2-morpholin-4-yl-ethyl)-benzamide;
4-{6-amino-5-[1-(2-chloro-3,6-difluoro-phenyl)-ethoxy]-pyridin-3-yl}-N-(1-methyl-piperidin-4-yl)-benzamide;
(4-{6-amino-5-[1-(2-chloro-3,6-difluoro-phenyl)-ethoxy]-pyridin-3-yl}-phenyl)-((R)-2-pyrrolidin-1-ylmethyl-pyrrolidin-1-yl)-methanone;
(4-{6-amino-5-[1-(2-chloro-3,6-difluoro-phenyl)-ethoxy]-pyridin-3-yl}-phenyl)-((R)-3-amino-pyrrolidin-1-yl)-methanone;
(4-{6-amino-5-[1-(2-chloro-3,6-difluoro-phenyl)-ethoxy]-pyridin-3-yl}-phenyl)-((3R,5S)-3,5-dimethyl-piperazin-1-yl)-methanone;
4-{6-amino-5-[1-(2-chloro-3,6-difluoro-phenyl)-ethoxy]-pyridin-3-yl}-N-(3-pyrrolidin-1-yl-propyl)-benzamide;
(4-{6-amino-5-[1-(2-chloro-3,6-difluoro-phenyl)-ethoxy]-pyridin-3-yl}-phenyl)-((S)-2-pyrrolidin-1-ylmethyl-pyrrolidin-1-yl)-methanone;
(4-{6-amino-5-[1-(2-chloro-3,6-difluoro-phenyl)-ethoxy]-pyridin-3-yl}-phenyl)-(4-methyl-piperazin-1-yl)-methanone;
(4-{6-amino-5-[1-(2-chloro-3,6-difluoro-phenyl)-ethoxy]-pyridin-3-yl}-phenyl)-(4-pyrrolidin-1-yl-piperidin-1-yl)-methanone;
4-{6-amino-5-[1-(2-chloro-3,6-difluoro-phenyl)-ethoxy]-pyridin-3-yl}-N-(2-pyrrolidin-1-yl-ethyl)-benzamide;
(4-{6-amino-5-[1-(2-chloro-3,6-difluoro-phenyl)-ethoxy]-pyridin-3-yl}-phenyl)-((S)-3-amino-pyrrolidin-1-yl)-methanone;
3-{6-amino-5-[1-(2-chloro-3,6-difluoro-phenyl)-ethoxy]-pyridin-3-yl}-benzoic acid;
(3-{6-amino-5-[1-(2-chloro-3,6-difluoro-phenyl)-ethoxy]-pyridin-3-yl}-phenyl)-((3R,5S)-3,5-dimethyl-piperazin-1-yl)-methanone;
(3-{6-amino-5-[1-(2-chloro-3,6-difluoro-phenyl)-ethoxy]-pyridin-3-yl}-phenyl)-((R)-3-amino-pyrrolidin-1-yl)-methanone;
3-{6-amino-5-[1-(2-chloro-3,6-difluoro-phenyl)-ethoxy]-pyridin-3-yl}-N-(1-methyl-piperidin-4-yl)-benzamide;
(3-{6-amino-5-[1-(2-chloro-3,6-difluoro-phenyl)-ethoxy]-pyridin-3-yl}-phenyl)-(4-methyl-piperazin-1-yl)-methanone;
3-{6-amino-5-[1-(2-chloro-3,6-difluoro-phenyl)-ethoxy]-pyridin-3-yl}-N-(3-pyrrolidin-1-yl-propyl)-benzamide;
3-{6-amino-5-[1-(2-chloro-3,6-difluoro-phenyl)-ethoxy]-pyridin-3-yl}-N-(2-pyrrolidin-1-yl-ethyl)-benzamide;
(3-{6-amino-5-[1-(2-chloro-3,6-difluoro-phenyl)-ethoxy]-pyridin-3-yl}-phenyl)-((S)-3-amino-pyrrolidin-1-yl)-methanone;
3-{6-amino-5-[1-(2-chloro-3,6-difluoro-phenyl)-ethoxy]-pyridin-3-yl}-N-(2-morpholin-4-yl-ethyl)-benzamide;
(3-{6-amino-5-[1-(2-chloro-3,6-difluoro-phenyl)-ethoxy]-pyridin-3-yl}-phenyl)-((R)-2-pyrrolidin-1-ylmethyl-pyrrolidin-1-yl)-methanone;
(3-{6-amino-5-[1-(2-chloro-3,6-difluoro-phenyl)-ethoxy]-pyridin-3-yl}-phenyl)-((S)-2-pyrrolidin-1-ylmethyl-pyrrolidin-1-yl)-methanone;
3-[1-(2-chloro-3,6-difluoro-phenyl)-ethoxy]-5-[4-(2-morpholin-4-yl-ethoxy)-phenyl]-pyridin-2-ylamine;
3-[1-(2-chloro-3,6-difluoro-phenyl)-ethoxy]-5-[3-(2-morpholin-4-yl-ethoxy)-phenyl]-pyridin-2-ylamine;
3-[1-(2,6-dichloro-3-fluoro-phenyl)-ethoxy]-5-[4-(2-pyrrolidin-1-yl-ethoxy)-phenyl]-pyridin-2-ylamine;
3-[1-(2,6-dichloro-3-fluoro-phenyl)-ethoxy]-5-{4-[2-(1-methylpyrrolidin-2-yl)-ethoxy]-phenyl}-pyridin-2-ylamine;
3-[1-(2,6-dichloro-3-fluoro-phenyl)-ethoxy]-5-[4-(2-morpholin-4-yl-ethoxy)-phenyl]-pyridin-2-ylamine;
3-[1-(2,6-dichloro-3-fluoro-phenyl)-ethoxy]-5-[3-(2-morpholin-4-yl-ethoxy)-phenyl]-pyridin-2-ylamine;
1-(4-{6-amino-5-[1-(2,6-dichloro-3-fluoro-phenyl)-ethoxy]-pyridin-3-yl}-phenoxy)-3-morpholin-4-yl-propan-2-ol;
3-[1-(2,6-dichloro-3-fluoro-phenyl)-ethoxy]-5-[4-(2-diethylamino-ethoxy)-phenyl]-pyridin-2-ylamine;
3-[1-(2,6-dichloro-3-fluoro-phenyl)-ethoxy]-5-[4-(1-methyl-piperazin-3-ylmethoxy)-phenyl]-pyridin-2-ylamine;
3-[1-(2,6-dichloro-3-fluoro-phenyl)-ethoxy]-5-[4-(2-diisopropylamino-ethoxy)-phenyl]-pyridin-2-ylamine;
3-[1-(2,6-dichloro-3-fluoro-phenyl)-ethoxy]-5-[4-(1-methyl-piperazin-4-yloxy)-phenyl]-pyridin-2-ylamine;
N-(4-{6-amino-5-[1-(2-chloro-3,6-difluoro-phenyl)-ethoxy]-pyridin-3-yl}-phenyl)-methanesulfonamide;
3-[1-(2-chloro-3,6-difluoro-phenyl)-ethoxy]-5-[4-(1,1-dioxo-1λ⁶-isothiazolidine2-yl)-phenyl]-pyridin-2-ylamine;
N-(4-{6-amino-5-[1-(2,6-dichloro-3-fluoro-phenyl)-ethoxy]-pyridin-3-yl}-phenyl)-methanesulfonamide;
3-[1-(2,6-dichloro-3-fluoro-phenyl)-ethoxy]-5-phenyl-pyridin-2-ylamine;
N-(4-{6-amino-5-[(R)-1-(2-chloro-3,6-difluoro-phenyl)-ethoxy]-pyridin-3-yl}-phenyl)-methanesulfonamide;
3-[1-(2,6-dichloro-3-fluoro-phenyl)-ethoxy]-5-thiophen-3-yl-pyridin-2-ylamine;
5-benzo[b]thiophen-2-yl-3-[1-(2,6-dichloro-3-fluoro-phenyl)-ethoxy]-pyridin-2-ylamine;
4-methyl-piperazine-1-carboxylic acid (4-{6-amino-5-[1-(2,6-dichloro-3-fluoro-phenyl)-ethoxy]-pyridin-3-yl}-phenyl)-amide;
1-(4-{6-amino-5-[1-(2,6-dichloro-3-fluoro-phenyl)-ethoxy]-pyridin-3-yl}-phenyl)-3-(2-pyrrolidin-1-yl-ethyl)-urea;
1-(4-{6-amino-5-[1-(2,6-dichloro-3-fluoro-phenyl)-ethoxy]-pyridin-3-yl}-phenyl)-3-(2-hydroxy-ethyl)-urea;
1-(4-{6-amino-5-[1-(2,6-dichloro-3-fluoro-phenyl)-ethoxy]-pyridin-3-yl}-phenyl)-3-(2-morpholin-4-yl-ethyl)-urea;
(R)-3-amino-pyrrolidine-1-carboxylic acid (4-{6-amino-5-[1-(2,6-dichloro-3-fluoro-phenyl)-ethoxy]-pyridin-3-yl}-phenyl)-amide;
(S)-3-amino-pyrrolidine-1-carboxylic acid (4-{6-amino-5-[1-(2,6-dichloro-3-fluoro-phenyl)-ethoxy]-pyridin-3-yl}-phenyl)-amide;
1-(4-{6-amino-5-[1-(2,6-dichloro-3-fluoro-phenyl)-ethoxy]-pyridin-3-yl}-phenyl)-3-(1-methyl-piperidin-4-yl)-urea;
1-(4-{6-amino-5-[1-(2-chloro-3,6-difluoro-phenyl)-ethoxy]-pyridin-3-yl}-phenyl)-3-(1-methyl-piperidin-4-yl)-urea;
(R)-3-amino-pyrrolidine-1-carboxylic acid (4-{6-amino-5-[1-(2-chloro-3,6-difluoro-phenyl)-ethoxy]-pyridin-3-yl}-phenyl)-amide;
(S)-3-amino-pyrrolidine-1-carboxylic acid (4-{6-amino-5-[1-(2-chloro-3,6-difluoro-phenyl)-ethoxy]-pyridin-3-yl}-phenyl)-amide;
1-(4-{6-amino-5-[1-(2-chloro-3,6-difluoro-phenyl)-ethoxy]-pyridin-3-yl}-phenyl)-3-(2-hydroxy-ethyl)-urea;
4-methyl-piperazine-1-carboxylic acid (4-{6-amino-5-[1-(2-chloro-3,6-difluoro-phenyl)-ethoxy]-pyridin-3-yl}-phenyl)-amide;
1-(4-{6-amino-5-[1-(2-chloro-3,6-difluoro-phenyl)-ethoxy]-pyridin-3-yl}-phenyl)-3-(2-pyrrolidin-1-yl-ethyl)-urea;
1-(4-{6-amino-5-[1-(2-chloro-3,6-difluoro-phenyl)-ethoxy]-pyridin-3-yl}-phenyl)-3-(2-morpholin-4-yl-ethyl)-urea;
(R)-2-pyrrolidin-1-ylmethyl-pyrrolidine-1-carboxylic acid (4-{6-amino-5-[1-(2-chloro-3,6-difluoro-phenyl)-ethoxy]-pyridin-3-yl}-phenyl)-amide;
3-{6-amino-5-[1-(2,6-dichloro-phenyl)-ethoxy]-pyridin-3-yl}-benzoic acid;
(3-{6-amino-5-[1-(2,6-dichloro-phenyl)-ethoxy]-pyridin-3-yl}-phenyl)-((3R,5S)-3,5-dimethyl-piperazin-1-yl)-methanone;
(3-{6-amino-5-[1-(2,6-dichloro-phenyl)-ethoxy]-pyridin-3-yl}-phenyl)-(4-pyrrolidin-1-yl-piperidin-1-yl)-methanone;
3-{6-amino-5-[1-(2,6-dichloro-phenyl)-ethoxy]-pyridin-3-yl}-N-(2-pyrrolidin-1-yl-ethyl)-benzamide;
3-{6-amino-5-[1-(2,6-dichloro-phenyl)-ethoxy]-pyridin-3-yl}-N-(2-morpholin-4-yl-ethyl)-benzamide;
(3-{6-amino-5-[1-(2,6-dichloro-phenyl)-ethoxy]-pyridin-3-yl}-phenyl)-((S)-2-pyrrolidin-1-ylmethyl-pyrrolidin-1-yl)-methanone;
3-{6-amino-5-[1-(2,6-dichloro-phenyl)-ethoxy]-pyridin-3-yl}-N-(3-pyrrolidin-1-yl-propyl)-benzamide;
N-[2-(4-acetyl-piperazin-1-yl)-ethyl]-3-{6-amino-5-[1-(2,6-dichloro-phenyl)-ethoxy]-pyridin-3-yl}-benzamide;
3-{6-amino-5-[1-(2,6-dichloro-phenyl)-ethoxy]-pyridin-3-yl}-N-(1-methyl-piperidin-4-yl)-benzamide;
(3-{6-amino-5-[1-(2,6-dichloro-phenyl)-ethoxy]-pyridin-3-yl}-phenyl)-(4-methyl-piperazin-1-yl)-methanone;
(3-{6-amino-5-[1-(2,6-dichloro-phenyl)-ethoxy]-pyridin-3-yl}-phenyl)-((R)-2-pyrrolidin-1-ylmethyl-pyrrolidin-1-yl)-methanone;
(3-{6-amino-5-[1-(2,6-dichloro-phenyl)-ethoxy]-pyridin-3-yl}-phenyl)-((S)-3-amino-pyrrolidin-1-yl)-methanone;
(3-{6-amino-5-[1-(2,6-dichloro-phenyl)-ethoxy]-pyridin-3-yl}-phenyl)-((R)-3-amino-pyrrolidin-1-yl)-methanone;
4-{6-amino-5-[1-(2,6-dichloro-phenyl)-ethoxy]-pyridin-3-yl}-benzoic acid;
4-{6-amino-5-[1-(2,6-dichloro-phenyl)-ethoxy]-pyridin-3-yl}-N-(2-pyrrolidin-1-yl-ethyl)-benzamide;
4-{6-amino-5-[1-(2,6-dichloro-phenyl)-ethoxy]-pyridin-3-yl}-N-(2-morpholin-4-yl-ethyl)-benzamide;
(4-{6-amino-5-[1-(2,6-dichloro-phenyl)-ethoxy]-pyridin-3-yl}-phenyl)-((S)-2-pyrrolidin-1-ylmethyl-pyrrolidin-1-yl)-methanone;
4-{6-amino-5-[1-(2,6-dichloro-phenyl)-ethoxy]-pyridin-3-yl}-N-(1-methyl-piperidin-4-yl)-benzamide;
(4-{6-amino-5-[1-(2,6-dichloro-phenyl)-ethoxy]-pyridin-3-yl}-phenyl)-((3R,5S)-3,5-dimethyl-piperazin-1-yl)-methanone;
N-[2-(4-acetyl-piperazin-1-yl)-ethyl]-4-{6-amino-5-[1-(2,6-dichloro-phenyl)-ethoxy]-pyridin-3-yl}-benzamide;
4-{6-amino-5-[1-(2,6-dichloro-phenyl)-ethoxy]-pyridin-3-yl}-N-(3-pyrrolidin-1-yl-propyl)-benzamide;
(4-{6-amino-5-[1-(2,6-dichloro-phenyl)-ethoxy]-pyridin-3-yl}-phenyl)-((S)-3-amino-pyrrolidin-1-yl)-methanone;
(4-{6-amino-5-[1-(2,6-dichloro-phenyl)-ethoxy]-pyridin-3-yl}-phenyl)-((R)-3-amino-pyrrolidin-1-yl)-methanone;
(4-{6-amino-5-[1-(2,6-dichloro-phenyl)-ethoxy]-pyridin-3-yl}-phenyl)-((R)-2-pyrrolidin-1-ylmethyl-pyrrolidin-1-yl)-methanone;
(4-{6-amino-5-[1-(2,6-dichloro-phenyl)-ethoxy]-pyridin-3-yl}-phenyl)-(4-pyrrolidin-1-yl-piperidin-1-yl)-methanone;
(4-{6-amino-5-[1-(2,6-dichloro-phenyl)-ethoxy]-pyridin-3-yl}-phenyl)-(4-methyl-piperazin-1-yl)-methanone;
(S)-2-pyrrolidin-1-ylmethyl-pyrrolidine-1-carboxylic acid (3-{6-amino-5-[1-(2,6-dichloro-3-fluoro-phenyl)-ethoxy]-pyridin-3-yl}-prop-2-ynyl)-amide;
4-methyl-piperazine-1-carboxylic acid (3-{6-amino-5-[1-(2,6-dichloro-3-fluoro-phenyl)-ethoxy]-pyridin-3-yl}-prop-2-ynyl)-amide;
4-pyrrolidin-1-yl-piperidine-1-carboxylic acid (3-{6-amino-5-[1-(2,6-dichloro-3-fluoro-phenyl)-ethoxy]-pyridin-3-yl}-prop-2-ynyl)-amide;
(3R,5S)-3,5-dimethyl-piperazine-1-carboxylic acid (3-{6-amino-5-[1-(2,6-dichloro-3-fluoro-phenyl)-ethoxy]-pyridin-3-yl}-prop-2-ynyl)-amide;
1-(3-{6-amino-5-[1-(2,6-dichloro-3-fluoro-phenyl)-ethoxy]-pyridin-3-yl}-prop-2-ynyl)-3-(1-methyl-piperidin-4-yl)-urea; 1-(3-{6-amino-5-[1-(2,6-dichloro-3-fluoro-phenyl)-ethoxy]-pyridin-3-yl}-prop-2-ynyl)-3-(3-pyrrolidin-1-yl-propyl)-urea;
1-(3-{6-amino-5-[1-(2,6-dichloro-3-fluoro-phenyl)-ethoxy]-pyridin-3-yl}-prop-2-ynyl)-3-(2-pyrrolidin-1-yl-ethyl)-urea;
1-(3-{6-amino-5-[1-(2,6-dichloro-3-fluoro-phenyl)-ethoxy]-pyridin-3-yl}-prop-2-ynyl)-3-(2-morpholin-4-yl-ethyl)-urea;
1-(3-{6-amino-5-[1-(2,6-dichloro-3-fluoro-phenyl)-ethoxy]-pyridin-3-yl}-prop-2-ynyl)-3-(3-morpholin-4-yl-propyl)-urea;
(R)-2-pyrrolidin-1-ylmethyl-pyrrolidine-1-carboxylic acid (3-{6-amino-5-[1-(2,6-dichloro-3-fluoro-phenyl)-ethoxy]-pyridin-3-yl}-prop-2-ynyl)-amide;
3-[1-(2,6-dichloro-3-fluoro-phenyl)-ethoxy]-5-(3-dimethylamino-prop-1-ynyl)-pyridin-2-ylamine;
(3-{6-amino-5-[1-(2,6-dichloro-3-fluoro-phenyl)-ethoxy]-pyridin-3-yl}-prop-2-ynyl)-urea;
N-(3-{6-amino-5-[1-(2,6-dichloro-3-fluoro-phenyl)-ethoxy]-pyridin-3-yl}-prop-2-ynyl)-2-piperidin-1-yl-acetamide;
N-(3-{6-amino-5-[1-(2,6-dichloro-3-fluoro-phenyl)-ethoxy]-pyridin-3-yl}-prop-2-ynyl)-2-morpholin-4-yl-acetamide;
N-(3-{6-amino-5-[1-(2,6-dichloro-3-fluoro-phenyl)-ethoxy]-pyridin-3-yl}-prop-2-ynyl)-2-pyrrolidin-1-yl-acetamide;
N-(3-{6-amino-5-[1-(2,6-dichloro-3-fluoro-phenyl)-ethoxy]-pyridin-3-yl}-prop-2-ynyl)-2-((R)-3-hydroxy-pyrrolidin-1-yl)-acetamide;
N-(3-{6-amino-5-[1-(2,6-dichloro-3-fluoro-phenyl)-ethoxy]-pyridin-3-yl}-prop-2-ynyl)-2-(4-hydroxy-piperidin-1-yl)-acetamide;
N-(3-{6-amino-5-[1-(2,6-dichloro-3-fluoro-phenyl)-ethoxy]-pyridin-3-yl}-prop-2-ynyl)-2-dimethylamino-acetamide;
N-(3-{6-amino-5-[1-(2,6-dichloro-3-fluoro-phenyl)-ethoxy]-pyridin-3-yl}-prop-2-ynyl)-2-diethylamino-acetamide;
2-(4-acetyl-piperazin-1-yl)-N-(3-{6-amino-5-[1-(2,6-dichloro-3-fluoro-phenyl)-ethoxy]-pyridin-3-yl}-prop-2-ynyl)-acetamide;
4-methyl-piperazine-1-carboxylic acid (3-{6-amino-5-[1-(2,6-dichloro-3-fluoro-phenyl)-ethoxy]-pyridin-3-yl}-1,1-dimethyl-prop-2-ynyl)-amide;
(3R,5S)-3,5-dimethyl-piperazine-1-carboxylic acid (3-{6-amino-5-[1-(2,6-dichloro-3-fluoro-phenyl)-ethoxy]-pyridin-3-yl}-1,1-dimethyl-prop-2-ynyl)-amide;
(R)-2-pyrrolidin-1-ylmethyl-pyrrolidine-1-carboxylic acid (3-{6-amino-5-[1-(2,6-dichloro-3-fluoro-phenyl)-ethoxy]-pyridin-3-yl}-1,1-dimethyl-prop-2-ynyl)-amide;
(S)-2-pyrrolidin-1-ylmethyl-pyrrolidine-1-carboxylic acid (3-{6-amino-5-[1-(2,6-dichloro-3-fluoro-phenyl)-ethoxy]-pyridin-3-yl}-1,1-dimethyl-prop-2-ynyl)-amide;
1-(3-{6-amino-5-[1-(2,6-dichloro-3-fluoro-phenyl)-ethoxy]-pyridin-3-yl}-1,1-dimethyl-prop-2-ynyl)-3-(2-morpholin-4-yl-ethyl)-urea;
1-(3-{6-amino-5-[1-(2,6-dichloro-3-fluoro-phenyl)-ethoxy]-pyridin-3-yl}-1,1-dimethyl-prop-2-ynyl)-3-(2-pyrrolidin-1-yl-ethyl)-urea;
4-pyrrolidin-1-yl-piperidine-1-carboxylic acid (3-{6-amino-5-[1-(2,6-dichloro-3-fluoro-phenyl)-ethoxy]-pyridin-3-yl}-1,1-dimethyl-prop-2-ynyl)-amide;
3-{6-amino-5-[1-(2,6-dichloro-3-fluoro-phenyl)-ethoxy]-pyridin-3-yl}-propynoic acid cyclohexylamide;
3-{6-amino-5-[1-(2,6-dichloro-3-fluoro-phenyl)-ethoxy]-pyridin-3-yl}-propynoic acid isopropylamide;
4-(3-amino-3-methyl-but-1-ynyl)-2-[1-(2,6-dichloro-3-fluorophenyl)-ethoxy]-phenylamine;
(4-{6-amino-5-[1-(3-fluoro-2-trifluoromethyl-phenyl)-ethoxy]-pyridin-3-yl}-phenyl)-(4-methyl-piperazin-1-yl)-methanone;
(4-{6-amino-5-[1-(3-fluoro-2-trifluoromethyl-phenyl)-ethoxy]-pyridin-3-yl}-phenyl)-(4-pyrrolidin-1-yl-piperidin-1-yl)-methanone;
(4-{6-amino-5-[1-(3-fluoro-2-trifluoromethyl-phenyl)-ethoxy]-pyridin-3-yl}-phenyl)-((3R,53)-3,5-dimethyl-piperazin-1-yl)-methanone;
(4-{6-amino-5-[1-(3-fluoro-2-trifluoromethyl-phenyl)-ethoxy]-pyridin-3-yl}-phenyl)-((S)-2-pyrrolidin-1-ylmethyl-pyrrolidin-1-yl)-methanone;
(4-{6-amino-5-[1-(3-fluoro-2-trifluoromethyl-phenyl)-ethoxy]-pyridin-3-yl}-phenyl)-((R)-2-pyrrolidin-1-ylmethyl-pyrrolidin-1-yl)-methanone;
4-{6-amino-5-[1-(3-fluoro-2-trifluoromethyl-phenyl)-ethoxy]-pyridin-3-yl}-N-(1-methyl-piperidin-4-yl)-benzamide;
4-{6-amino-5-[1-(3-fluoro-2-trifluoromethyl-phenyl)-ethoxy]-pyridin-3-yl}-N-(2-pyrrolidin-1-yl-ethyl)-benzamide;
4-{6-amino-5-[1-(3-fluoro-2-trifluoromethyl-phenyl)-ethoxy]-pyridin-3-yl}-N-(2-morpholin-4-yl-ethyl)-benzamide;
4-{6-amino-5-[1-(3-fluoro-2-trifluoromethyl-phenyl)-ethoxy]-pyridin-3-yl}-N-(3-pyrrolidin-1-yl-propyl)-benzamide;
4-{6-amino-5-[1-(3-fluoro-2-trifluoromethyl-phenyl)-ethoxy]-pyridin-3-yl}-N-(3-morpholin-4-yl-propyl)-benzamide;
6-amino-5-[1-(2,6-dichloro-3-fluoro-phenyl)-ethoxy]-nicotinonitrile;
6-amino-5-[1-(2,6-dichloro-3-cyano-phenyl)-ethoxy]-nicotinonitrile;
5-aminomethyl-3-[1-(2,6-dichloro-3-fluoro-phenyl)-ethoxy]-pyridin-2-ylamine;
(R)-2-pyrrolidin-1-ylmethyl-pyrrolidine-1-carboxylic acid {6-amino-5-[1-(2,6-dichloro-3-fluoro-phenyl)-ethoxy]-pyridin-3-ylmethyl}-amide;
N-{6-amino-5-[1-(2,6-dichloro-3-fluoro-phenyl)-ethoxy]-pyridin-3-ylmethyl}-methanesulfonamide;
N-{6-amino-5-[1-(2,6-dichloro-3-fluoro-phenyl)-ethoxy]-pyridin-3-ylmethyl}-acetamide;
N-{6-amino-5-[1-(2,6-dichloro-3-fluoro-phenyl)-ethoxy]-pyridin-3-ylmethyl}-4-methyl-benzenesulfonamide;
3-[1-(2,6-dichloro-3-fluoro-phenyl)-ethoxy]-5-vinyl-pyridin-2-ylamine;
(S)-1-{6-amino-5-[1-(2,6-dichloro-3-fluoro-phenyl)-ethoxy]-pyridin-3-yl}-ethane-1,2-diol;
(R)-1-{6-amino-5-[1-(2,6-dichloro-3-fluoro-phenyl)-ethoxy]-pyridin-3-yl}-ethane-1,2-diol;
3-[1-(2,6-dichloro-3-fluoro-phenyl)-ethoxy]-5-(1H-pyrazol-4-yl)-pyridin-2-ylamine;
3-[1-(2,6-dichloro-3-fluoro-phenyl)-ethoxy]-5-[1-(2-pyrrolidin-1-yl-ethyl)-1H-pyrazol-4-yl]-pyridin-2-ylamine;
3-[1-(2,6-dichloro-3-fluoro-phenyl)-ethoxy]-5-[1-(2-diisopropylamino-ethyl)-1H-pyrazol-4-yl]-pyridin-2-ylamine;
3-[1-(2,6-dichloro-3-fluoro-phenyl)-ethoxy]-5-[1-(2-morpholin-4-yl-ethyl)-1H-pyrazol-4-yl]-pyridin-2-ylamine;
5-bromo-3-(3-fluoro-2-methoxy-benzyloxy)-pyridin-2-ylamine; 5-bromo-3-[1-(3-fluoro-2-methoxy-phenyl)-ethoxy]-pyridin-2-ylamine;
{4-[6-amino-5-(3-fluoro-2-methoxy-benzyloxy)-pyridin-3-yl]-phenyl}-((3R,5S)-3,5-dimethyl-piperazin-1-yl)-methanone;
(4-{6-amino-5-[1-(3-fluoro-2-methoxy-phenyl)-ethoxy]-pyridin-3-yl}-phenyl)-((3R,5S)-3,5-dimethyl-piperazin-1-yl)-methanone;
5-bromo-3-(3-fluoro-2-isopropoxy-benzyloxy)-pyridin-2-ylamine;
{4-[6-amino-5-(3-fluoro-2-isopropoxy-benzyloxy)-pyridin-3-yl]-phenyl}-((3R,5S)-3,5-dimethyl-piperazin-1-yl)-methanone; 5-(4-amino-phenyl)-3-[1-(2,6-dichloro-3-fluoro-phenyl)-ethoxy]-pyridin-2-ylamine;
(4-{6-amino-5-[1-(2,6-dichloro-3-fluoro-phenyl)-ethoxy]-pyridin-3-yl}-phenoxy)-acetic acid methyl ester;
(4-{6-amino-5-[1-(2,6-dichloro-3-fluoro-phenyl)-ethoxy]-pyridin-3-yl}-phenoxy)-acetic acid;
2-(4-{6-amino-5-[1-(2,6-dichloro-3-fluoro-phenyl)-ethoxy]-pyridin-3-yl}-phenoxy)-1-((3R,5S)-3,5-dimethyl-piperazin-1-yl)-ethanone;
2-(4-{6-amino-5-[1-(2,6-dichloro-3-fluoro-phenyl)-ethoxy]-pyridin-3-yl}-phenoxy)-1-((R)-3-hydroxy-pyrrolidin-1-yl)-ethanone;
4-[2-(4-{6-amino-5-[1-(2,6-dichloro-3-fluoro-phenyl)-ethoxy]-pyridin-3-yl}-phenoxy)-acetyl]-piperazine-1-carboxylic acid tert-butyl ester;
2-(4-{6-amino-5-[1-(2,6-dichloro-3-fluoro-phenyl)-ethoxy]-pyridin-3-yl}-phenoxy)-1-((R)-2-pyrrolidin-1-ylmethyl-pyrrolidin-1-yl)-ethanone; 5-bromo-3-(3-fluoro-6,7,8,9-tetrahydro-5H-benzocyclohepten-5-yloxy)-pyridin-2-ylamine;
{4-[6-amino-5-(3-fluoro-6,7,8,9-tetrahydro-5H-benzocyclohepten-5-yloxy)-pyridin-3-yl]-phenyl}-((3R,5S)-3,5-dimethyl-piperazin-1-yl)-methanone;
3-(3-fluoro-6,7,8,9-tetrahydro-5H-benzocyclohepten-5-yloxy)-5-[4-(2-pyrrolidin-1-yl-ethoxy)-phenyl]-pyridin-2-ylamine;
N-{4-[6-amino-5-(3-fluoro-6,7,8,9-tetrahydro-5H-benzocyclohepten-5-yloxy)-pyridin-3-yl]-phenyl}-methanesulfonamide;
3-(3-fluoro-6,7,8,9-tetrahydro-5H-benzocyclohepten-5-yloxy)-5-(1H-pyrazol-4-yl)-pyridin-2-ylamine;
5-bromo-3-[1-(2-chloro-3-fluoro-phenyl)-ethoxy]-pyridin-2-ylamine;
3-[1-(2-chloro-3-fluoro-phenyl)-ethoxy]-5-[4-(2-pyrrolidin-1-yl-ethoxy)-phenyl]-pyridin-2-ylamine;
5'-benzyloxy-[2,3']bipyridinyl-6'-ylamine;
5-benzyloxy-[3,3']bipyridinyl-6-ylamine;
3-benzyloxy-5-pyrimidin-5-yl-pyridin-2-ylamine; 5-benzyloxy-[3,3']bipyridinyl-6,6'-diamine;
5'-(2-chloro-benzyloxy)-[2,3']bipyridinyl-6'-ylamine; 5-(2-chloro-benzyloxy)-[3,3']bipyridinyl-6-ylamine;
3-(2-chloro-benzyloxy)-5-pyrimidin-5-yl-pyridin-2-ylamine;
5-(2-chloro-benzyloxy)-[3,3']bipyridinyl-6,6'-diamine;
5'-(4-chloro-benzyloxy)-[2,3']bipyridinyl-6'-ylamine;
5-(4-chloro-benzyloxy)-[3,3']bipyridinyl-6-ylamine;
3-(4-chloro-benzyloxy)-5-pyrimidin-5-yl-pyridin-2-ylamine;
5-(4-chloro-benzyloxy)-[3,3']bipyridinyl-6,6'-diamine; 5'-(2-chloro-3,6-difluoro-benzyloxy)-[2,3']bipyridinyl-6'-ylamine;
5-(2-chloro-3,6-difluoro-benzyloxy)-[3,3']bipyridinyl-6-ylamine;
5-(2-chloro-3,6-difluoro-benzyloxy)-[3,4']bipyridinyl-6-ylamine;
3-(2-chloro-3,6-difluoro-benzyloxy)-5-pyrimidin-5-yl-pyridin-2-ylamine;
5-(2-chloro-3,6-difluoro-benzyloxy)-[3,3']bipyridinyl-6,6'-diamine;
5'-(2,6-dichloro-benzyloxy)-[2,3']bipyridinyl-6'-ylamine;
5-(2,6-dichloro-benzyloxy)-[3,3']bipyridinyl-6-ylamine;
5-(2,6-dichloro-benzyloxy)-[3,4']bipyridinyl-6-ylamine;
3-(2,6-dichloro-benzyloxy)-5-pyrimidin-5-yl-pyridin-2-ylamine;
5-(2,6-dichloro-benzyloxy)-[3,3']bipyridinyl-6,6'-diamine;
5-[1-(2,6-dichloro-3-fluoro-phenyl)-ethoxy]-[3,3']bipyridinyl-6,6'-diamine;
{6'-amino-5'-[1-(2,6-dichloro-3-fluoro-phenyl)-ethoxy]-[2,3']bipyridinyl-4-yl}-(4-methyl-piperazin-1-yl)-methanone;
{6'-amino-5'-[1-(2,6-dichloro-3-fluoro-phenyl)-ethoxy]-[2,3']bipyridinyl-6-yl}-(4-methyl-piperazin-1-yl)-methanone;
{6'-amino-5'-[1-(2,6-dichloro-3-fluoro-phenyl)-ethoxy]-[3,3']bipyridinyl-5-yl}-(4-methyl-piperazin-1-yl)-methanone;
{6'-amino-5'-[1-(2,6-dichloro-3-fluoro-phenyl)-ethoxy]-[3,3']bipyridinyl-6-yl}-(4-methyl-piperazin-1-yl)-methanone;
{6'-amino-5-[1-(2,6-dichloro-3-fluoro-phenyl)-ethoxy]-[3,4']bipyridinyl-2'-yl}-(4-methyl-piperazin-1-yl)-methanone;
5-[1-(2-chloro-3,6-difluoro-phenyl)-ethoxy]-[3,3']bipyridinyl-6,6'-diamine;
{6'-amino-5'-[1-(2-chloro-3,6-difluoro-phenyl)-ethoxy]-[2,3']bipyridinyl-5-yl}-(4-methyl-piperazin-1-yl)-methanone;
{6'-amino-5'-[1-(2-chloro-3,6-difluoro-phenyl)-ethoxy]-[2,3']bipyridinyl-4-yl}-(4-methyl-piperazin-1-yl)-methanone;
{6'-amino-5'-[1-(2-chloro-3,6-difluoro-phenyl)-ethoxy]-[2,3']bipyridinyl-6-yl}-(4-methyl-piperazin-1-yl)-methanone;
{6'-amino-5'-[1-(2-chloro-3,6-difluoro-phenyl)-ethoxy]-[3,3']bipyridinyl-5-yl}-(4-methyl-piperazin-1-yl)-methanone;
{6'-amino-5'-[1-(2-chloro-3,6-difluoro-phenyl)-ethoxy]-[3,3']bipyridinyl-6-yl}-(4-methyl-piperazin-1-yl)-methanone;
{6-amino-5-[1-(2-chloro-3,6-difluoro-phenyl)-ethoxy]-[3,4']bipyridinyl-2'-yl}-(4-methyl-piperazin-1-yl)-methanone;
5'-[1-(2,6-dichloro-3-fluoro-phenyl)-ethoxy]-[2,3']bipyridinyl-6'-ylamine;
5'-[1-(2-chloro-3,6-difluoro-phenyl)-ethoxy]-[2,3']bipyridinyl-6'-ylamine;
5-[1-(2-chloro-3,6-difluoro-phenyl)-ethoxy]-[3,3']bipyridinyl-6-ylamine;
3-[1-(2-chloro-3,6-difluoro-phenyl)-ethoxy]-5-pyrimidin-5-yl-pyridin-2-ylamine;
{6'-amino-5'-[1-(2,6-dichloro-3-fluoro-phenyl)-ethoxy]-[2,3']bipyridinyl-5-yl}-(4-methyl-piperazin-1-yl)-methanone;
5-[1-(2-chloro-3,6-difluoro-phenyl)-ethoxy]-[3,4']bipyridinyl-6-ylamine;
5-benzyloxy-3-[1-(2-chloro-3,6-difluoro-phenyl)-ethoxy]-pyridin-2-ylamine;
3-[1-(2-chloro-3,6-difluoro-phenyl)-ethoxy]-5-(2-ethyl-butoxy)-pyridin-2-ylamine;
3-[1-(2-chloro-3,6-difluoro-phenyl)-ethoxy]-5-(3-methylbutoxy)-pyridin-2-ylamine;
3-[1-(2-chloro-3,6-difluoro-phenyl)-ethoxy]-5-butoxy -pyridin-2-ylamine;
3-[1-(2-chloro-3,6-difluoro-phenyl)-ethoxy]-5-propoxy-pyridin-2-ylamine;
3-[1-(2-chloro-3,6-difluoro-phenyl)-ethoxy]-5-cyclohexylmethoxy-pyridin-2-ylamine;
6-amino-5-[1-(2-chloro-3,6-difluoro-phenyl)-ethoxy]-pyridin-3-ol;
3-[1-(2-chloro-3,6-difluoro-phenyl)-ethoxy]-5-(2-cyclohexylethoxy)-pyridin-2-ylamine;
3-[1-(2-chloro-3,6-difluoro-phenyl)-ethoxy]-5-isobutoxypyridin-2-ylamine;
3-[1-(2-chloro-3,6-difluoro-phenyl)-ethoxy]-5-phenethyloxypyridin-2-ylamine;
3-[1-(2-chloro-3,6-difluoro-phenyl)-ethoxy]-5-(pyridin-2-ylmethoxy)-pyridin-2-ylamine;
3-[1-(2-chloro-3,6-difluoro-phenyl)-ethoxy]-5-(pyridin-4-ylmethoxy)-pyridin-2-ylamine;
(4-{6-amino-5-[1-(2,6-dichloro-3-fluoro-phenyl)-ethoxy]-pyridin-3-yl}-phenyl)-((3R,5S)-3,5-dimethyl-piperazin-1-yl)-methanone;
(4-{6-amino-5-[1-(2,6-dichloro-3-fluoro-phenyl)-ethoxy]-pyridin-3-yl}-phenyl)-((3R,5S)-3,5-dimethyl-piperazin-1-yl)-methanone;
5-{6-amino-5-[1-(2,6-dichloro-3-fluoro-phenyl)-ethoxy]-pyridin-3-yl}-2-fluoro-benzonitrile;
4-(4-{6-amino-5-[1-(2,6-dichloro-3-fluoro-phenyl)-ethoxy]-pyridin-3-yl}-phenyl)-piperidin-4-ol;
(4-{6-amino-5-[1-(2,6-dichloro-3-fluoro-phenyl)-ethoxy]-pyridin-3-yl}-phenyl)-piperidin-1-yl-methanone;
(4-{6-amino-5-[1-(2,6-dichloro-3-fluoro-phenyl)-ethoxy]-pyridin-3-yl}-phenyl)-pyrrolidin-1-yl-methanone;
4-{6-amino-5-[1-(2,6-dichloro-3-fluoro-phenyl)-ethoxy]-pyridin-3-yl}-3-methyl-benzoic acid methyl ester;
3-[1-(2,6-dichloro-3-fluoro-phenyl)-ethoxy]-5-[4-(dimethylpiperazine-1-ylmethyl)-phenyl]-pyridin-2-ylamine;
(4-{6-amino-5-[1-(2,6-dichloro-3-fluoro-phenyl)-ethoxy]-pyridin-3-yl}-3,5-dimethoxy-phenyl)-(dimethyl-piperazin-1-yl)-methanone;
(4-{6-amino-5-[1-(2,6-dichloro-3-fluoro-phenyl)-ethoxy]-pyridin-3-yl}-2-fluoro-phenyl)-(dimethyl-piperazin-1-yl)-methanone;
(4-{6-amino-5-[1-(2,6-dichloro-3-fluoro-phenyl)-ethoxy]-pyridin-3-yl}-3-fluoro-phenyl)-(dimethyl-piperazin-1-yl)-methanone;
(4-{6-amino-5-[1-(2,6-dichloro-3-fluoro-phenyl)-ethoxy]-pyridin-3-yl}-3-methyl-phenyl)-(dimethyl-piperazin-1-yl)-methanone;
(4-{6-amino-5-[1-(2,6-dichloro-3-fluoro-phenyl)-ethoxy]-pyridin-3-yl}-phenyl)-(4-methyl-[1,4]diazepan-1-yl)-methanone;
(4-{6-amino-5-[1-(2,6-dichloro-3-fluoro-phenyl)-ethoxy]-pyridin-3-yl}-phenyl)-[1,4]diazepan-1-yl-methanone;
(4-{6-amino-5-[1-(2,6-dichloro-3-fluoro-phenyl)-ethoxy]-pyridin-3-yl}-phenyl)-piperazin-1-yl-methanone;
3-[1-(2,6-dichloro-3-fluoro-phenyl)-ethoxy]-5-vinyl-pyridin-2-ylamine;
(4-{6-amino-5-[1-(2,6-dichloro-3-fluoro-phenyl)-ethoxy]-pyridin-3-yl}-phenyl)-((3R,4S)-3,4-dihydroxy-pyrrolidin-1-yl)-methanone;
5-[(1-benzyl-pyrrolidin-3-ylamino)-methyl]-3-[1-(2,6-dichloro-3-fluoro-phenyl)-ethoxy]-pyridin-2-ylamine;
4-{6-amino-5-[1-(2,6-dichloro-3-fluoro-phenyl)-ethoxy]-pyridin-3-yl}-N-azetidin-3-yl-benzamide;
4-{6-amino-5-[1-(2,6-dichloro-3-fluoro-phenyl)-ethoxy]-pyridin-3-yl}-N,N-dimethyl-benzenesulfonamide;
3-[1-(2,6-dichloro-3-fluoro-phenyl)-ethoxy]-5-(6-methoxy-1H-benzimidazol-2-yl)-pyridin-2-ylamine;
3-[1-(2,6-dichloro-3-fluoro-phenyl)-ethoxy]-5-(6-methoxy-1-methyl-1H-benzimidazol-2-yl)-pyridin-2-ylamine;
3-[1-(2,6-dichloro-3-fluoro-phenyl)-ethoxy]-5-[4-(4-methyl-[1,4]diazepane-1-sulfonyl)-phenyl]-pyridin-2-ylamine;
6-{6-amino-5-[1-(2,6-dichloro-3-fluoro-phenyl)-ethoxy]-pyridin-3-yl}-1-methyl-1H-indazole-3-carboxylic acid amide;
3-[1-(2,6-dichloro-3-fluoro-phenyl)-ethoxy]-5-(1-methyl-1H-pyrazol-4-yl)-pyridin-2-ylamine;
5-(3-chloro-phenyl)-3-[1-(2,6-dichloro-3-fluoro-phenyl)-ethoxy]-pyridin-2-ylamine;
3-[1-(2,6-dichloro-3-fluoro-phenyl)-ethoxy]-5-(4-fluoro-3-methyl-phenyl)-pyridin-2-ylamine;
3-[1-(2,6-dichloro-3-fluoro-phenyl)-ethoxy]-5-(3-trifluoromethyl-phenyl)-pyridin-2-ylamine;
3-[1-(2,6-dichloro-3-fluoro-phenyl)-ethoxy]-5-(3-fluorophenyl)-pyridin-2-ylamine;
3-[1-(2,6-dichloro-3-fluoro-phenyl)-ethoxy]-5-(3-trifluoromethoxy-phenyl)-pyridin-2-ylamine;
5-benzo[1,3]dioxol-5-yl-3-[1-(2,6-dichloro-3-fluoro-phenyl)-ethoxy]-pyridin-2-ylamine;
3-{6-amino-5-[1-(2,6-dichloro-3-fluoro-phenyl)-ethoxy]-pyridin-3-yl}-phenol;
(3-{6-amino-5-[1-(2,6-dichloro-3-fluoro-phenyl)-ethoxy]-pyridin-3-yl}-phenyl)-methanol;
3-{6-amino-5-[1-(2,6-dichloro-3-fluoro-phenyl)-ethoxy]-pyridin-3-yl}-benzonitrile;
3-[1-(2,6-dichloro-3-fluoro-phenyl)-ethoxy]-5-(3-methoxyphenyl)-pyridin-2-ylamine;
3-[1-(2,6-dichloro-3-fluoro-phenyl)-ethoxy]-5-(3,5-dichlorophenyl)-pyridin-2-ylamine;
3-[1-(2,6-dichloro-3-fluoro-phenyl)-ethoxy]-5-(2,5-dimethylphenyl)-pyridin-2-ylamine;
5-(5-chloro-2-methoxy-phenyl)-3-[1-(2,6-dichloro-3-fluorophenyl)-ethoxy]-pyridin-2-ylamine;
5-(3-chloro-4-fluoro-phenyl)-3-[1-(2,6-dichloro-3-fluorophenyl)-ethoxy]-pyridin-2-ylamine;
3-[1-(2,6-dichloro-3-fluoro-phenyl)-ethoxy]-5-(5-fluoro-2-methoxy-phenyl)-pyridin-2-ylamine;
3-[1-(2,6-dichloro-3-fluoro-phenyl)-ethoxy]-5-(3-isopropylphenyl)-pyridin-2-ylamine;
3-[1-(2,6-dichloro-3-fluoro-phenyl)-ethoxy]-5-(3,4-dichlorophenyl)-pyridin-2-ylamine;
4-{6-amino-5-[1-(2,6-dichloro-3-fluoro-phenyl)-ethoxy]-pyridin-3-yl}-benzonitrile;
3-[1-(2,6-dichloro-3-fluoro-phenyl)-ethoxy]-5-(3,4-difluorophenyl)-pyridin-2-ylamine;
(4-{6-amino-5-[1-(2,6-dichloro-3-fluoro-phenyl)-ethoxy]-pyridin-3-yl}-phenyl)-((2R,6S)-2,6-dimethyl-morpholin-4-yl)-methanone;
3-[1-(2,6-dichloro-3-fluoro-phenyl)-ethoxy]-5-(2-ethoxyphenyl)-pyridin-2-ylamine;
3-[1-(2,6-dichloro-3-fluoro-phenyl)-ethoxy]-5-(2,5-dimethoxyphenyl)-pyridin-2-ylamine;
3-[1-(2,6-dichloro-3-fluoro-phenyl)-ethoxy]-5-(2,4-dimethoxyphenyl)-pyridin-2-ylamine;
3-[1-(2,6-dichloro-3-fluoro-phenyl)-ethoxy]-5-(2,6-dimethoxyphenyl)-pyridin-2-ylamine;
3-[1-(2,6-dichloro-3-fluoro-phenyl)-ethoxy]-5-(2-trifluoromethyl-phenyl)-pyridin-2-ylamine;
5-(2-chloro-phenyl)-3-[1-(2,6-dichloro-3-fluoro-phenyl)-ethoxy]-pyridin-2-ylamine;
3-[1-(2,6-dichloro-3-fluoro-phenyl)-ethoxy]-5-(2-trifluoromethoxy-phenyl)-pyridin-2-ylamine;
1-(2-{6-amino-5-[1-(2,6-dichloro-3-fluoro-phenyl)-ethoxy]-pyridin-3-yl}-phenyl)-ethanone;
3-[1-(2,6-dichloro-3-fluoro-phenyl)-ethoxy]-5-(2-fluorophenyl)-pyridin-2-ylamine; (2-{6-amino-5-[1-(2,6-dichloro-3-fluoro-phenyl)-ethoxy]-pyridin-3-yl}-phenyl)-methanol;
3-[1-(2,6-dichloro-3-fluoro-phenyl)-ethoxy]-5-o-tolyl-pyridin-2-ylamine;
3-[1-(2,6-dichloro-3-fluoro-phenyl)-ethoxy]-5-(2-methoxyphenyl)-pyridin-2-ylamine;
3-[1-(2,6-dichloro-3-fluoro-phenyl)-ethoxy]-5-(2,6-dimethylphenyl)-pyridin-2-ylamine;
(4-{6-amino-5-[1-(2,6-dichloro-3-fluoro-phenyl)-ethoxy]-pyridin-3-yl}-phenyl)-morpholin-4-yl-methanone;
(4-{6-amino-5-[1-(2,6-dichloro-3-fluoro-phenyl)-ethoxy]-pyridin-3-yl}-2-chloro-phenyl)-((3R,5S)-dimethyl-piperazin-1-yl)-methanone;
(4-{6-amino-5-[1-(2,6-dichloro-3-fluoro-phenyl)-ethoxy]-pyridin-3-yl}-2-methyl-phenyl)-((3R,5S)-dimethyl-piperazin-1-yl)-methanone;
3-[1-(2,6-dichloro-3-fluoro-phenyl)-ethoxy]-5-[4-((2R,6S)-2,6-dimethyl-morpholin-4-ylmethyl)-phenyl]-pyridin-2-ylamine;
3-[1-(2,6-dichloro-3-fluoro-phenyl)-ethoxy]-5-(4-morpholin-4-ylmethyl-phenyl)-pyridin-2-ylamine;
3-[1-(2,6-dichloro-3-fluoro-phenyl)-ethoxy]-5-(3,5-dimethylphenyl)-pyridin-2-ylamine;
3-[1-(2,6-dichloro-3-fluoro-phenyl)-ethoxy]-5-m-tolyl-pyridin-2-ylamine;
3-[1-(2,6-dichloro-3-fluoro-phenyl)-ethoxy]-5-(3,4-dimethoxyphenyl)-pyridin-2-ylamine;
5-biphenyl-3-yl-3-[1-(2,6-dichloro-3-fluoro-phenyl)-ethoxy]-pyridin-2-ylamine;
5-(3,5-bis-trifluoromethyl-phenyl)-3-[1-(2,6-dichloro-3-fluorophenyl)-ethoxy]-pyridin-2-ylamine;
3-[1-(2,6-dichloro-3-fluoro-phenyl)-ethoxy]-5-(3,4-dichlorophenyl)-pyridin-2-ylamine;
1-(3-{6-amino-5-[1-(2,6-dichloro-3-fluoro-phenyl)-ethoxy]-pyridin-3-yl}-phenyl)-ethanone;
3-[1-(2,6-dichloro-3-fluoro-phenyl)-ethoxy]-5-(3,5-difluorophenyl)-pyridin-2-ylamine;
3-[1-(2,6-dichloro-3-fluoro-phenyl)-ethoxy]-5-(2,5-dichlorophenyl)-pyridin-2-ylamine;
(4-{6-amino-5-[1-(2,6-dichloro-4-trifluoromethyl-phenyl)-ethoxy]-pyridin-3-yl}-phenyl)-((3R,5S)-3,5-dimethyl-piperazin-1-yl)-methanone;
3-[1-(2,6-dichloro-3-fluoro-phenyl)-ethoxy]-5-(3-ethoxyphenyl)-pyridin-2-ylamine;
(4-{6-amino-5-[1-(2-trifluoromethyl-phenyl)-ethoxy]-pyridin-3-yl}-phenyl)-(3,5-dimethyl-piperazin-1-yl)-methanone;
(4-{6-amino-5-[1-(3-trifluoromethyl-phenyl)-ethoxy]-pyridin-3-yl}-phenyl)-(3,5-dimethyl-piperazin-1-yl)-methanone;
7-[4-(3,5-dimethyl-piperazine-1-carbonyl)-phenyl]-2-phenyl-4H-pyrido[3,2-b][1,4]oxazin-3-one;
{4-[6-amino-5-(3-fluoro-2-trifluoromethyl-benzyloxy)-pyridin-3-yl]-phenyl}-(3,5-dimethyl-piperazin-1-yl)-methanone;
{4-[6-amino-5-(2,6-difluoro-benzyloxy)-pyridin-3-yl]-phenyl}-(3,5-dimethyl-piperazin-1-yl)-methanone;
[4-(6-amino-5-benzyloxy-pyridin-3-yl)-phenyl]-(3,5-dimethylpiperazin-1-yl)-methanone;
(4-{6-amino-5-[1-(2-chloro-3,6-difluoro-phenyl)-ethoxy]-pyridin-3-yl}-phenyl)-(4-ethyl-piperazin-1-yl)-methanone;
[4-(6-amino-5-benzyloxy-pyridin-3-yl)-phenyl]-(4-ethylpiperazin-1-yl)-methanone;
{4-[6-amino-5-(2-methyl-benzyloxy)-pyridin-3-yl]-phenyl}-(3,5-dimethyl-piperazin-1-yl)-methanone;
3-{2-amino-5-[4-(4-pyrrolidin-1-yl-piperidine-1-carbonyl)-phenyl]-pyridin-3-yloxymethyl}-benzoic acid methyl ester;
3-{2-amino-5-[4-(3,5-dimethyl-piperazine-1-carbonyl)-phenyl]-pyridin-3-yloxymethyl}-benzoic acid methyl ester;
{4-[6-amino-5-(2-methyl-benzyloxy)-pyridin-3-yl]-phenyl}-(4-pyrrolidin-1-yl-piperidin-1-yl)-methanone;
[4-(6-amino-5-cyclohexylmethoxy-pyridin-3-yl)-phenyl]-(4-pyrrolidin-1-yl-piperidin-1-yl)-methanone;
4-(1-{2-amino-5-[4-(4-pyrrolidin-1-yl-piperidine-1-carbonyl)-phenyl]-pyridin-3-yloxy}-ethyl)-[2-(3-hydroxy-phenyl)-ethyl]-benzamide;
4-(1-{2-amino-5-[4-(4-pyrrolidin-1-yl-piperidine-1-carbonyl)-phenyl]-pyridin-3-yloxy}-ethyl)-[2-(2,6-dichloro-phenyl)-ethyl]-benzamide;
4-(1-{2-amino-5-[4-(4-pyrrolidin-1-yl-piperidine-1-carbonyl)-phenyl]-pyridin-3-yloxy}-ethyl)-(1-benzyl-piperidin-4-yl)-benzamide;
4-(1-{2-amino-5-[4-(4-pyrrolidin-1-yl-piperidine-1-carbonyl)-phenyl]-pyridin-3-yloxy}-ethyl)-[3-(2-oxo-pyrrolidin-1-yl)-propyl]-benzamide;
(4-{6-amino-5-[1-(2,6-dichloro-3-fluoro-phenyl)-ethoxy]-pyridin-3-yl}-phenyl)-(4-ethyl-piperazin-1-yl)-methanone;
{4-[6-amino-5-(2,6-dichloro-benzyloxy)-pyridin-3-yl]-phenyl}-(3,5-dimethyl-piperazin-1-yl)-methanone;
(6-amino-3-aza-bicyclo[3.1.0]hex-3-yl)-(4-{6-amino-5-[1-(2,6-dichloro-3-fluoro-phenyl)-ethoxy]-pyridin-3-yl}-phenyl)-methanone;
5-[1-(2,6-dichloro-3-fluoro-phenyl)-ethoxy]-6'-(2-morpholin-4-yl-ethoxy)-[3,3']bipyridinyl-6-ylamine;
6'-amino-5'-[1-(2,6-dichloro-3-fluoro-phenyl)-ethoxy]-1-(2-pyrrolidin-1-yl-ethyl)-1H-[3,3']bipyridinyl-6-one;
5-[1-(2,6-dichloro-3-fluoro-phenyl)-ethoxy]-6'-(2-pyrrolidin-1-yl-ethoxy)-[3,3']bipyridinyl-6-ylamine;
6'-amino-5'-[1-(2,6-dichloro-3-fluoro-phenyl)-ethoxy]-1-[2-(1-methyl-pyrrolidin-2-yl)-ethyl]-1H-[3,3']bipyridinyl-6-one;
(4-{6-amino-5-[1-(2,4,6-trimethyl-phenyl)-ethoxy]-pyridin-3-yl}-phenyl)-(4-pyrrolidin-1-yl-piperidin-1-yl)-methanone;
(4-{6-amino-5-[1-(2-chloro-6-fluoro-phenyl)-ethoxy]-pyridin-3-yl}-phenyl)-(4-pyrrolidin-1-yl-piperidin-1-yl)-methanone;
3-[1-(2,6-dichloro-3-fluoro-phenyl)-ethoxy]-5-(4-fluorophenyl)-pyridin-2-ylamine;
6'-amino-5'-[1-(2,6-dichloro-3-fluoro-phenyl)-ethoxy]-1H-[3,3']bipyridinyl-6-one;
5'-bromo-5-[1-(2,6-dichloro-3-fluoro-phenyl)-ethoxy]-[3,3']bipyridinyl-6-ylamine;
3-[1-(2,6-dichloro-3-fluoro-phenyl)-ethoxy]-5-(4-dimethylaminophenyl)-pyridin-2-ylamine;
5-[1-(2,6-dichloro-3-fluoro-phenyl)-ethoxy]-2'-methoxy-[3,3']bipyridinyl-6-ylamine;
3-[1-(2,6-dichloro-3-fluoro-phenyl)-ethoxy]-5-(1H-indol-5-yl)-pyridin-2-ylamine;
(4-{6-amino-5-[1-(2,6-dichloro-phenyl)-propoxy]-pyridin-3-yl}-phenyl)-(3,5-dimethyl-piperazin-1-yl)-methanone;
[4-(6-amino-5-benzyloxy-pyridin-3-yl)-phenyl]-(4-pyrrolidin-1-yl-piperidin-1-yl)-methanone;
3-(2,6-dichloro-3-fluoro-benzyloxy)-5-thiazol-2-yl-pyridin-2-ylamine;
(4-{6-amino-5-[1-(2-fluoro-6-trifluoromethyl-phenyl)-ethoxy]-pyridin-3-yl}-phenyl)-(4-pyrrolidin-1-yl-piperidin-1-yl)-methanone;
3-(2,6-dichloro-3-fluoro-benzyloxy)-5-(1-methyl-1H-imidazol-2-yl)-pyridin-2-ylamine;
{4-[6-amino-5-(2,4,6-trimethyl-benzyloxy)-pyridin-3-yl]-phenyl}-(4-pyrrolidin-1-yl-piperidin-1-yl)-methanone;
{4-[6-amino-5-(2,3,5,6-tetramethyl-benzyloxy)-pyridin-3-yl]-phenyl}-(4-pyrrolidin-1-yl-piperidin-1-yl)-methanone;
{4-[6-amino-5-(2,4,6-trifluoro-benzyloxy)-pyridin-3-yl]-phenyl}-(4-pyrrolidin-1-yl-piperidin-1-yl)-methanone;
(4-{6-amino-5-[1-(2-fluoro-6-trifluoromethyl-phenyl)-ethoxy]-pyridin-3-yl}-phenyl)-(4-pyrrolidin-1-yl-piperidin-1-yl)-methanone;
6-amino-5-[1-(2,6-dichloro-3-fluoro-phenyl)-ethoxy]-N-methylnicotinamidine;
6-amino-5-[1-(2,6-dichloro-3-fluoro-phenyl)-ethoxy]-N-(2-morpholin-4-yl-ethyl)-nicotinamidine; (4-{6-amino-5-[1-(2,4,5-trifluoro-phenyl)-propoxy]-pyridin-3-yl}-phenyl)-(4-pyrrolidin-1-yl-piperidin-1-yl)-methanone;
(4-{6-amino-5-[1-(6-chloro-2-fluoro-3-methyl-phenyl)-ethoxy]-pyridin-3-yl}-phenyl)-(4-pyrrolidin-1-yl-piperidin-1-yl)-methanone;
3-(1-{2-amino-5-[4-(4-pyrrolidin-1-yl-piperidine-1-carbonyl)-phenyl]-pyridin-3-yloxy}-ethyl)-benzoic acid;
3-{5-amino-6-[1-(2,6-dichloro-3-fluoro-phenyl)-ethoxy]-pyrazin-2-yl}-N-(3-pyrrolidin-1-yl-propyl)-benzamide;
3-{5-amino-6-[1-(2-chloro-3,6-difluoro-phenyl)-ethoxy]-pyrazin-2-yl}-N-(3-pyrrolidin-1-yl-propyl)-benzamide;
3-{5-amino-6-[1-(2-chloro-3,6-difluoro-phenyl)-ethoxy]-pyrazin-2-yl}-N-(1-methyl-piperidin-4-yl)-benzamide;
3-{5-amino-6-[1-(2-chloro-3,6-difluoro-phenyl)-ethoxy]-pyrazin-2-yl}-N-(2-pyrrolidin-1-yl-ethyl)-benzamide;
3-{5-amino-6-[1-(2-chloro-3,6-difluoro-phenyl)-ethoxy]-pyrazin-2-yl}-N-(2-morpholin-4-yl-ethyl)-benzamide;
N-[2-(4-acetyl-piperazin-1-yl)-ethyl]-3-{5-amino-6-[1-(2-chloro-3,6-difluoro-phenyl)-ethoxy]-pyrazin-2-yl}-benzamide; (3-{5-amino-6-[1-(2-chloro-3,6-difluoro-phenyl)-ethoxy]-pyrazin-2-yl}-phenyl)-(4-methyl-piperazin-1-yl)-methanone; (3-{5-amino-6-[1-(2-chloro-3,6-difluoro-phenyl)-ethoxy]-pyridin-2-yl}-phenyl)-(4-pyrrolidin-1-yl-piperidin-1-yl)-methanone;
(3-{5-amino-6-[1-(2-chloro-3,6-difluoro-phenyl)-ethoxy]-pyrazin-2-yl}-phenyl)-((3R,5S)-3,5-dimethyl-piperazin-1-yl)-methanone;
(3-{5-amino-6-[1-(2-chloro-3,6-difluoro-phenyl)-ethoxy]-pyrazin-2-yl}-phenyl)-((S)-2-pyrrolidin-1-ylmethyl-pyrrolidin-1-yl)-methanone;
(3-{5-amino-6-[1-(2-chloro-3,6-difluoro-phenyl)-ethoxy]-pyrazin-2-yl}-phenyl)-((R)-3-amino-pyrrolidin-1-yl)-methanone;
(3-{5-amino-6-[1-(2-chloro-3,6-difluoro-phenyl)-ethoxy]-pyrazin-2-yl}-phenyl)-((S)-3-amino-pyrrolidin-1-yl)-methanone;
4-{5-amino-6-[1-(2-chloro-3,6-difluoro-phenyl)-ethoxy]-pyrazin-2-yl}-benzoic acid;
4-{5-amino-6-[1-(2-chloro-3,6-difluoro-phenyl)-ethoxy]-pyrazin-2-yl}-N-(3-pyrrolidin-1-yl-propyl)-benzamide;
(4-{5-amino-6-[1-(2-chloro-3,6-difluoro-phenyl)-ethoxy]-pyrazin-2-yl}-phenyl)-(4-methyl-piperazin-1-yl)-methanone;
(4-{5-amino-6-[1-(2-chloro-3,6-difluoro-phenyl)-ethoxy]-pyrazin-2-yl}-phenyl)-(4-pyrrolidin-1-yl-piperidin-1-yl)-methanone;
(4-{5-amino-6-[1-(2-chloro-3,6-difluoro-phenyl)-ethoxy]-pyrazin-2-yl}-phenyl)-((3R,5S)-3,5-dimethyl-piperazin-1-yl)-methanone;
(4-{5-amino-6-[1-(2-chloro-3,6-difluoro-phenyl)-ethoxy]-pyrazin-2-yl}-phenyl)-((S)-2-pyrrolidin-1-ylmethyl-pyrrolidin-1-yl)-methanone;
(4-{5-amino-6-[1-(2-chloro-3,6-difluoro-phenyl)-ethoxy]-pyrazin-2-yl}-phenyl)-((R)-2-pyrrolidin-1-ylmethyl-pyrrolidin-1-yl)-methanone;
(4-{5-amino-6-[1-(2-chloro-3,6-difluoro-phenyl)-ethoxy]-pyrazin-2-yl}-phenyl)-((R)-3-amino-pyrrolidin-1-yl)-methanone; 4-{5-amino-6-[1-(2-chloro-3,6-difluoro-phenyl)-ethoxy]-pyrazin-2-yl}-N-(1-methyl-piperidin-4-yl)-benzamide;
4-{5-amino-6-[1-(2-chloro-3,6-difluoro-phenyl)-ethoxy]-pyrazin-2-yl}-N-(2-pyrrolidin-1-yl-ethyl)-benzamide;
4-{5-amino-6-[1-(2-chloro-3,6-difluoro-phenyl)-ethoxy]-pyrazin-2-yl}-N-(2-morpholin-4-yl-ethyl)-benzamide;
N-[2-(4-acetyl-piperazin-1-yl)-ethyl]-4-{5-amino-6-[1-(2-chloro-3,6-difluoro-phenyl)-ethoxy]-pyrazin-2-yl}-benzamide;
2-[4-(2-hydroxy-acetyl)-piperazin-1-yl]-ethanesulfonic acid (4-{5-amino-6-[1-(2-chloro-3,6-difluoro-phenyl)-ethoxy]-pyrazin-2-yl}-phenyl)-amide;
3-[5-amino-6-(3-fluoro-2-trifluoromethyl-benzyloxy)-pyrazin-2-yl]-benzoic acid;
{3-[5-amino-6-(3-fluoro-2-trifluoromethyl-benzyloxy)-pyrazin-2-yl]-phenyl}-(4-pyrrolidin-1-yl-piperidin-1-yl)-methanone;
3-[5-amino-6-(3-fluoro-2-trifluoromethyl-benzyloxy)-pyrazin-2-yl]-N-{2-[ethyl-(2-methoxy-ethyl)-amino]-ethyl}-benzamide;
{3-[5-amino-6-(3-fluoro-2-trifluoromethyl-benzyloxy)-pyrazin-2-yl]-phenyl}-(4-methyl-piperazin-1-yl)-methanone;
3-[5-amino-6-(3-fluoro-2-trifluoromethyl-benzyloxy)-pyrazin-2-yl]-N-(3-pyrrolidin-1-yl-propyl)-benzamide;
N-[2-(4-acetyl-piperazin-1-yl)-ethyl]-3-[5-amino-6-(3-fluoro-2-trifluoromethyl-benzyloxy)-pyrazin-2-yl]-benzamide;
{4-[5-amino-6-(3-fluoro-2-trifluoromethyl-benzyloxy)-pyrazin-2-yl]-phenyl}-(4-pyrrolidin-1-yl-piperidin-1-yl)-methanone;
{4-[5-amino-6-(3-fluoro-2-trifluoromethyl-benzyloxy)-pyrazin-2-yl]-phenyl}-(4-methyl-piperazin-1-yl)-methanone;
{4-[5-amino-6-(3-fluoro-2-trifluoromethyl-benzyloxy)-pyrazin-2-yl]-phenyl}-((S)-2-pyrrolidin-1-ylmethyl-pyrrolidin-1-yl)-methanone;
(3-{5-amino-6-[1-(2,6-dichloro-3-fluoro-phenyl)-ethoxy]-pyrazin-2-yl}-phenyl)-(4-methyl-piperazin-1-yl)-methanone;
(3-{5-amino-6-[1-(2,6-dichloro-3-fluoro-phenyl)-ethoxy]-pyrazin-2-yl}-phenyl)-((3R,5S)-3,5-dimethyl-piperazin-1-yl)-methanone;
3-{5-amino-6-[1-(2,6-dichloro-3-fluoro-phenyl)-ethoxy]-pyrazin-2-yl}-N-(1-methyl-piperidin-4-yl)-benzamide;
3-{5-amino-6-[1-(2,6-dichloro-3-fluoro-phenyl)-ethoxy]-pyrazin-2-yl}-N-(2-pyrrolidin-1-yl-ethyl)-benzamide;
3-{5-amino-6-[1-(2,6-dichloro-3-fluoro-phenyl)-ethoxy]-pyrazin-2-yl}-N-(2-morpholin-4-yl-ethyl)-benzamide;
3-{5-amino-6-[1-(2,6-dichloro-3-fluoro-phenyl)-ethoxy]-pyrazin-2-yl}-N-(3-morpholin-4-yl-propyl)-benzamide;
(3-{5-amino-6-[1-(2,6-dichloro-3-fluoro-phenyl)-ethoxy]-pyrazin-2-yl}-phenyl)-(4-cyclopropylamino-piperidin-1-yl)-methanone;
3-{5-amino-6-[1-(2,6-dichloro-3-fluoro-phenyl)-ethoxy]-pyrazin-2-y1}-N-((S)-2-hydroxy-3-morpholin-4-yl-propyl)-benzamide; 3-{5-amino-6-[1-(2,6-dichloro-3-fluoro-phenyl)-ethoxy]-pyrazin-
2-y1}-N-((R)-2-hydroxy-3-pyrrolidin-1-yl-propyl)-benzamide; (3-{6-amino-5-[1-(2,6-dichloro-3-fluoro-phenyl)-ethoxy]-pyridin-3-yl}-phenyl)-(4-pyrrolidin-1-yl-piperidin-1-yl)-methanone;
2-diethylamino-ethanesulfonic acid (4-{5-amino-6-[1-(2,6-dichloro-3-fluoro-phenyl)-ethoxy]-pyrazin-2-yl}-phenyl)-amide;
2-(4-hydroxy-piperidin-1-yl)-ethanesulfonic acid (4-{5-amino-6-[1-(2,6-dichloro-3-fluoro-phenyl)-ethoxy]-pyrazin-2-yl}-phenyl)-amide;
2-dimethylamino-ethanesulfonic acid (4-{5-amino-6-[1-(2,6-dichloro-3-fluoro-phenyl)-ethoxy]-pyrazin-2-yl}-phenyl)-amide;
2-((R)-3-hydroxy-pyrrolidin-1-yl)-ethanesulfonic acid (4-{5-amino-6-[1-(2,6-dichloro-3-fluoro-phenyl)-ethoxy]-pyrazin-2-yl}-phenyl)-amide;
2-pyrrolidin-1-ylethanesulfonic acid (4-{5-amino-6-[1-(2,6-dichloro-3-fluoro-phenyl)-ethoxy]-pyrazin-2-yl}-phenyl)-amide;
4-{5-amino-6-[1-(2,6-dichloro-3-fluoro-phenyl)-ethoxy]-pyrazin-2-yl}-benzoic acid;
4-{5-amino-6-[1-(2,6-dichloro-3-fluoro-phenyl)-ethoxy]-pyrazin-2-yl}-N-((R)-2-hydroxy-3-pyrrolidin-1-yl-propyl)-benzamide;
(4-{5-amino-6-[1-(2,6-dichloro-3-fluoro-phenyl)-ethoxy]-pyrazin-2-yl}-phenyl)-(4-cyclopropylamino-piperidin-1-yl)-methanone;
4-{5-amino-6-[1-(2,6-dichloro-3-fluoro-phenyl)-ethoxy]-pyrazin-2-y1}-N-((S)-2-hydroxy-3-pyrrolidin-1-yl-propyl)-benzamide;
4-{5-amino-6-[1-(2,6-dichloro-3-fluoro-phenyl)-ethoxy]-pyrazin-2-y1}-N-((R)-2-hydroxy-3-morpholin-4-yl-propyl)-benzamide;
4-{5-amino-6-[1-(2,6-dichloro-3-fluoro-phenyl)-ethoxy]-pyrazin-2-yl}-N-(1-methyl-piperidin-4-yl)-benzamide;
(4-{5-amino-6-[1-(2,6-dichloro-3-fluoro-phenyl)-ethoxy]-pyrazin-2-yl}-phenyl)-((S)-2-pyrrolidin-1-ylmethyl-pyrrolidin-1-yl)-methanone;
(4-{5-amino-6-[1-(2,6-dichloro-3-fluoro-phenyl)-ethoxy]-pyrazin-2-yl}-phenyl)-(4-pyrrolidin-1-yl-piperidin-1-yl)-methanone;
4-{5-amino-6-[1-(2,6-dichloro-3-fluoro-phenyl)-ethoxy]-pyrazin-2-yl}-N-(2-morpholin-4-yl-ethyl)-benzamide;
(4-{5-amino-6-[1-(2,6-dichloro-3-fluoro-phenyl)-ethoxy]-pyrazin-2-yl}-phenyl)-(4-methyl-piperazin-1-yl)-methanone;
(4-{5-amino-6-[1-(2,6-dichloro-3-fluoro-phenyl)-ethoxy]-pyrazin-2-yl}-phenyl)-((3R,5S)-3,5-dimethyl-piperazin-1-yl)-methanone;
4-{5-amino-6-[1-(2,6-dichloro-phenyl)-ethoxy]-pyrazin-2-yl}-benzoic acid;
(4-{5-amino-6-[1-(2,6-dichloro-phenyl)-ethoxy]-pyrazin-2-yl}-phenyl)-(4-pyrrolidin-1-yl-piperidin-1-yl)-methanone; 4-{5-amino-6-[1-(2,6-dichloro-phenyl)-ethoxy]-pyrazin-2-yl}-N-(2-morpholin-4-yl-ethyl)-benzamide;
(4-{5-amino-6-[1-(2,6-dichloro-phenyl)-ethoxy]-pyrazin-2-yl}-phenyl)-((3R,5S)-3,5-dimethyl-piperazin-1-yl)-methanone;
4-{5-amino-6-[1-(2,6-dichloro-phenyl)-ethoxy]-pyrazin-2-yl}-N-(1-methyl-piperidin-4-yl)-benzamide;
(4-{5-amino-6-[1-(2,6-dichloro-phenyl)-ethoxy]-pyrazin-2-yl}-phenyl)-((R)-2-pyrrolidin-1-ylmethyl-pyrrolidin-1-yl)-methanone;
(4-{5-amino-6-[1-(2,6-dichloro-phenyl)-ethoxy]-pyrazin-2-yl}-phenyl)-((S)-2-pyrrolidin-1-ylmethyl-pyrrolidin-1-yl)-methanone;
(4-{5-amino-6-[1-(2,6-dichloro-phenyl)-ethoxy]-pyrazin-2-yl}-phenyl)-(4-methyl-piperazin-1-yl)-methanone;
(4-{5-amino-6-[1-(2,6-dichloro-phenyl)-ethoxy]-pyrazin-2-yl}-phenyl)-((R)-3-aminopyrrolidin-1-yl)-methanone;
(4-{5-amino-6-[1-(2,6-dichloro-phenyl)-ethoxy]-pyrazin-2-yl}-phenyl)-((S)-3-aminopyrrolidin-1-yl)-methanone hydrochloride;
4-{5-amino-6-[1-(2,6-dichloro-phenyl)-ethoxy]-pyrazin-2-yl}-N-(2-pyrrolidin-1-yl-ethyl)-benzamide;
4-{5-amino-6-[1-(2,6-dichloro-phenyl)-ethoxy]-pyrazin-2-yl}-N-(3-pyrrolidin-1-yl-propyl)-benzamide;
3-{5-amino-6-[1-(2,6-dichloro-phenyl)-ethoxy]-pyrazin-2-yl}-benzoic acid;
3-{5-amino-6-[1-(2,6-dichloro-phenyl)-ethoxy]-pyrazin-2-yl}-N-(1-methyl-piperidin-4-yl)-benzamide;
3-{5-amino-6-[1-(2,6-dichloro-phenyl)-ethoxy]-pyrazin-2-yl}-N-(2-pyrrolidin-1-yl-ethyl)-benzamide;
(3-{5-amino-6-[1-(2,6-dichloro-phenyl)-ethoxy]-pyrazin-2-yl}-phenyl)-((3R,5S)-3,5-dimethyl-piperazin-1-yl)-methanone;
3-{5-amino-6-[1-(2,6-dichloro-phenyl)-ethoxy]-pyrazin-2-yl}-N-(2-morpholin-4-yl-ethyl)-benzamide;
(3-{5-amino-6-[1-(2,6-dichloro-phenyl)-ethoxy]-pyrazin-2-yl}-phenyl)-((S)-2-pyrrolidin-1-ylmethyl-pyrrolidin-1-yl)-methanone;
(3-{5-amino-6-[1-(2,6-dichloro-phenyl)-ethoxy]-pyrazin-2-yl}-phenyl)-(4-pyrrolidin-1-yl-piperidin-1-yl)-methanone;
N-[2-(4-acetyl-piperazin-1-yl)-ethyl]-4-{5-amino-6-[1-(2,6-dichloro-phenyl)-ethoxy]-pyrazin-2-yl}-benzamide;
N-[2-(4-acetyl-piperazin-1-yl)-ethyl]-3-{5-amino-6-[1-(2,6-dichloro-phenyl)-ethoxy]-pyrazin-2-yl}-benzamide;
(3-{5-amino-6-[1-(2,6-dichloro-phenyl)-ethoxy]-pyrazin-2-yl}-phenyl)-((R)-2-pyrrolidin-1-ylmethyl-pyrrolidin-1-yl)-methanone;
3-{5-amino-6-[1-(2,6-dichloro-phenyl)-ethoxy]-pyrazin-2-yl}-N-(3-pyrrolidin-1-yl-propyl)-benzamide;
(3-{5-amino-6-[1-(2,6-dichloro-phenyl)-ethoxy]-pyrazin-2-yl}-phenyl)-((S)-3-amino-pyrrolidin-1-yl)-methanone;
(3-{5-amino-6-[1-(2,6-dichloro-phenyl)-ethoxy]-pyrazin-2-yl}-phenyl)-((R)-3-amino-pyrrolidin-1-yl)-methanone hydrochloride;
(3-{5-amino-6-[1-(2,6-dichloro-phenyl)-ethoxy]-pyrazin-2-yl}-phenyl)-(4-methyl-piperazin-1-yl)-methanone; 1-(4-{5-amino-6-[1-(2-chloro-3,6-difluoro-phenyl)-ethoxy]-pyrazin-2-yl}-phenyl)-3-(2-morpholin-4-yl-ethyl)-urea;
(R)-2-pyrrolidin-1-ylmethyl-pyrrolidine-1-carboxylic acid (4-{5-amino-6-[1-(2-chloro-3,6-difluoro-phenyl)-ethoxy]-pyrazin-2-yl}-phenyl)-amide;
1-(4-{5-amino-6-[1-(2-chloro-3,6-difluoro-phenyl)-ethoxy]-pyrazin-2-yl}-phenyl)-3-(2-pyrrolidin-1-yl-ethyl)-urea;
4-methyl-piperazine-1-carboxylic acid (4-{5-amino-6-[1-(2-chloro-3,6-difluoro-phenyl)-ethoxy]-pyrazin-2-yl}-phenyl)-amide;
1-(4-{5-amino-6-[1-(2-chloro-3,6-difluoro-phenyl)-ethoxy]-pyrazin-2-yl}-phenyl)-3-(2-hydroxy-ethyl)-urea;
(S)-3-amino-pyrrolidine-1-carboxylic acid (4-{5-amino-6-[1-(2-chloro-3,6-difluoro-phenyl)-ethoxy]-pyrazin-2-yl}-phenyl)-amide;
1-(4-{5-amino-6-[1-(2-chloro-3,6-difluoro-phenyl)-ethoxy]-pyrazin-2-yl}-phenyl)-3-(1-methyl-piperidin-4-yl)-urea;
4-methyl-piperazine-1-carboxylic acid (4-{5-amino-6-[1-(2,6-dichloro-3-fluoro-phenyl)-ethoxy]-pyrazin-2-yl}-phenyl)-amide;
1-(4-{5-amino-6-[1-(2,6-dichloro-3-fluoro-phenyl)-ethoxy]-pyrazin-2-yl}-phenyl)-3-(2-hydroxy-ethyl)-urea;
(S)-3-amino-pyrrolidine-1-carboxylic acid (4-{5-amino-6-[1-(2,6-dichloro-3-fluoro-phenyl)-ethoxy]-pyrazin-2-yl}-phenyl)-amide;
1-(4-{5-amino-6-[1-(2,6-dichloro-3-fluoro-phenyl)-ethoxy]-pyrazin-2-yl}-phenyl)-3-(1-methyl-piperidin-4-yl)-urea;
5-[5-amino-6-(2-chloro-3,6-difluoro-benzyloxy)-pyrazin-2-yl]-thiophene-2-carboxylic acid;
{5-[5-amino-6-(2-chloro-3,6-difluoro-benzyloxy)-pyrazin-2-yl]-thiophen-2-yl}-(4-methyl-piperazin-1-yl)-methanone;
{5-[5-amino-6-(2-chloro-3,6-difluoro-benzyloxy)-pyrazin-2-yl]-thiophen-2-yl}-(4-pyrrolidin-1-yl-piperidin-1-yl)-methanone;
{5-[5-amino-6-(2-chloro-3,6-difluoro-benzyloxy)-pyrazin-2-yl]-thiophen-2-yl}-((3R,5S)-3,5-dimethyl-piperazin-1-yl)-methanone;
{5-[5-amino-6-(2-chloro-3,6-difluoro-benzyloxy)-pyrazin-2-yl]-thiophen-2-yl}-((R)-2-pyrrolidin-1-ylmethyl-pyrrolidin-1-yl)-methanone;
5-[5-amino-6-(2-chloro-3,6-difluoro-benzyloxy)-pyrazin-2-yl]-thiophene-2-carboxylic acid (2-morpholin-4-yl-ethyl)-amide;
3-[1-(2,6-dichloro-3-fluoro-phenyl)-ethoxy]-5-{5-[(4-methylpiperazin-1-yl)carbonyl]pyridin-2-yllpyrazine-2-amine-trifluoroacetate;
3-[1-(2,6-dichloro-3-fluoro-phenyl)-ethoxy]-5-pyridin-4-yl-pyrazin-2-ylamine;
3-[1-(2,6-dichloro-3-fluoro-phenyl)-ethoxy]-5-(1H-pyrrol-2-yl)-pyrazin-2-ylamine;
(6-{5-amino-6-[1-(2,6-dichloro-3-fluoro-phenyl)-ethoxy]-pyrazin-2-yl}-pyridin-3-yl)-(4-methyl-piperazin-1-yl)-methanone;
(2-{5-amino-6-[1-(2,6-dichloro-3-fluoro-phenyl)-ethoxy]-pyrazin-2-yl}-pyridin-4-yl)-(4-methyl-piperazin-1-yl)-methanone;
(6-{5-amino-6-[1-(2,6-dichloro-3-fluoro-phenyl)-ethoxy]-pyrazin-2-yl}-pyridin-2-yl)-(4-methyl-piperazin-1-yl)-methanone;
(5-{5-amino-6-[1-(2,6-dichloro-3-fluoro-phenyl)-ethoxy]-pyrazin-2-yl}-pyridin-3-yl)-(4-methyl-piperazin-1-yl)-methanone;
(4-{5-amino-6-[1-(2,6-dichloro-3-fluoro-phenyl)-ethoxy]-pyrazin-2-yl}-pyridin-2-yl)-(4-methyl-piperazin-1-yl)-methanone;
6-{5-amino-6-[1-(2,6-dichloro-3-fluoro-phenyl)-ethoxy]-pyrazin-2-yl}-N-(2-morpholin-4-yl-ethyl)-nicotinamide;
5-{5-amino-6-[1-(2,6-dichloro-3-fluoro-phenyl)-ethoxy]-pyrazin-2-yl}-N-(2-morpholin-4-yl-ethyl)-nicotinamide;
6-{5-amino-6-[1-(2,6-dichloro-3-fluoro-phenyl)-ethoxy]-pyrazin-2-yl}-N-(3-morpholin-4-yl-propyl)-nicotinamide;
5-{5-amino-6-[1-(2,6-dichloro-3-fluoro-phenyl)-ethoxy]-pyrazin-2-yl}-N-(3-morpholin-4-yl-propyl)-nicotinamide;
(6-{5-amino-6-[1-(2,6-dichloro-3-fluoro-phenyl)-ethoxy]-pyrazin-2-yl}-pyridin-3-yl)-(4-isopropyl-piperazin-1-yl)-methanone

A JNK inhibitor as an active ingredient of the prophylaxis or therapeutic agent for ALS in the present invention includes all of naturally-occurring substances derived from microorganism etc., a semi-synthetic substance derived therefrom, and fully synthetic compounds. Examples thereof include, but are not limited to, SP600125 (see Table 4), JNK-IN-8, JNK Inhibitor IX, AS-601245/JNK Inhibitor V and the like. Preferably, it may be SP600125. In another embodiment, JNK inhibitor may be a JNK selective inhibitor. Examples of the JNK selective inhibitor include SP600125.

A Syk inhibitor as an active ingredient of the prophylactic or therapeutic agent for ALS in the present invention includes all of naturally-occurring substances derived from microorganism etc., a semi-synthetic substances induced therefrom, and fully synthetic compounds. Examples thereof include, but are not limited to, Syk Inhibitor (see Table 4), fostamatinib disodium/R935788/R788, PRT-062607, GS-9973, TAK-659 and the like. Preferably, it may be Syk Inhibitor. In another embodiment, Syk inhibitor may be a Syk selective inhibitor. Examples of the Syk selective inhibitor include Syk Inhibitor.

A JAK inhibitor as an active ingredient of the prophylactic or therapeutic agent for ALS in the present invention includes all of naturally-occurring substances derived from microorganism etc., a semi-synthetic substances derived therefrom, and a fully synthetic compounds. Examples thereof include, but are not limited to, JAK Inhibitor I (see Table 1), AT9283 (see Table 8), fedratinib/SAR302503/TG101348, pacritinib/SB1518, LY2784544, momelotinib/CYT387, AZD1480, XL-019, BMS-911543, NS-018, INCB039110, INCB47986, AT9283, lestaurtinib/CEP-701 and the like. Preferably, it may be JAK Inhibitor I or AT9283. In another embodiment, JAK inhibitor may be a JAK selective inhibitor. Examples of the JAK selective inhibitor include JAK Inhibitor I.

A prostaglandin analogue as an active ingredient of the prophylaxis or therapeutic agent for ALS in the present invention includes all of naturally-occurring substances derived from mammal etc., a semi-synthetic substances derived therefrom, and fully synthetic compounds. Examples thereof include, but are not limited to, Bimatoprost or an analog thereof and the like.

Here, the "analog of Bimatoprost" is, for example, a compound represented by the following formula (III): 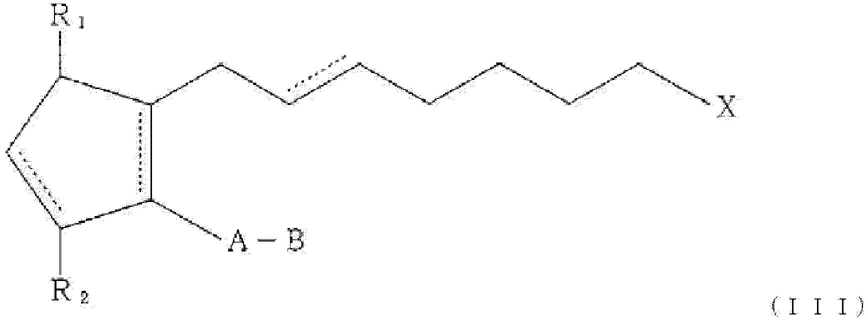 or the following formula (III'): 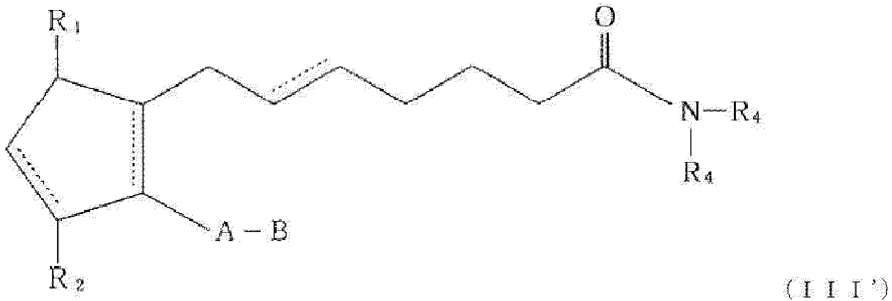 wherein
a bond with a dotted line is a single bond, or a double bond possibly with a cis or trans configuration;
A is alkylene or an alkenylene group having 2 - 6 carbon atoms, and is optionally substituted by one or more hydroxy, oxo, alkyloxy or alkylcarboxy groups;
B is a cycloalkyl group having 3 - 7 carbon atoms, or an aryl group selected from the group consisting of hydrocarbylaryl and a heteroaryl group (hetero atom is selected from the group consisting of nitrogen, oxygen and sulfur atoms);
X is a group selected from the group consisting of halo, hydryl, nitro, amino, azido, oxime, cyano, thiol, alkoxy and thioether groups;
one of R₁ and R₂ is =O, -OH or -O(CO)R₆, and the other is -OH or -O(CO)Rₑ, or R₁ is =O and R₂ is H;
R₆ is a saturated or unsaturated acyclic hydrocarbon group having 1 - about 20 carbon atoms, or -(CH₂)ₘR₇;
m is 0 - 10, R₇ is a cycloalkyl group having 3 - 7 carbon atoms, or hydrocarbylaryl or a heteroaryl group (hetero atom is selected from the group consisting of nitrogen, oxygen and sulfur atoms); and
two R₄ are the same or different and each is a hydrogen atom or a C₁ - C₃ alkyl group.

Each term described as higher concept in the explanation of each group in the above-mentioned formulas (III) and (III') (e.g., "alkylene or alkenylene group having 2 - 6 carbon atoms", "alkyloxy or alkylcarboxy group" for A, "cycloalkyl group having 3 - 7 carbon atoms", "hydrocarbylaryl group", "heteroaryl group" for B and R₇, "alkoxy group", "thioether group" for X, "saturated or unsaturated acyclic hydrocarbon group having 1 to about 20 carbon atoms" for R₆, "C₁ - C₃ alkyl group" for R₄ etc.) is as defined in JP-A-2004-346080.

Preferable examples of the analog of Bimatoprost include a compound represented by the following formula (IV): 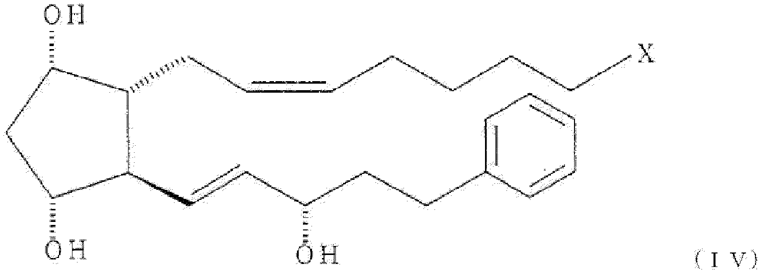 or the following formula (IV'): 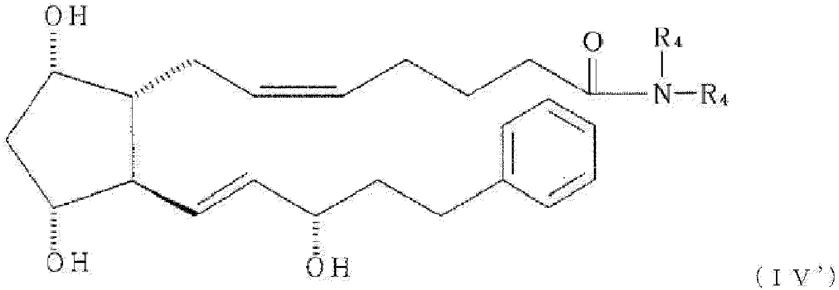 wherein R₄ is as defined above.

Specific examples of the analog of Bimatoprost include the following compounds.
Cyclopentane heptenamide-5-cis-2-(3-αhydroxy-5-phenyl-1-trans-pentenyl)-3,5-dihydroxy, [1α,2β,3α,5α]
Cyclopentane N,N-dimethylheptenamide-5-cis-2-(3-αhydroxy-5-phenyl-1-trans-pentenyl)-3,5-dihydroxy, [1α,2β,3α,5α]
Cyclopentane heptenylmethoxide-5-cis-2-(3-αhydroxy-5-phenyl-1-trans-pentenyl)-3,5-dihydroxy, [1α,2β,3α,5α]
Cyclopentane heptenylfluoride-5-cis-2-(3-αhydroxy-5-phenyl-1-trans-pentenyl)-3,5-dihydroxy, [1α,2β,3α,5α]
Cyclopentane heptenylnitrate-5-cis-2-(3-αhydroxy-5-phenyl-1-trans-pentenyl)-3,5-dihydroxy, [1α,2β,3α,5α]
Cyclopentane heptenyliodide-5-cis-2-(3-αhydroxy-5-phenyl-1-trans-pentenyl)-3,5-dihydroxy, [1α,2β,3α,5α]
Cyclopentane hepteneamine-5-cis-2-(3-αhydroxy-5-phenyl-1-transpentenyl)-3,5-dihydroxy, [1α,2β,3α,5α]
Cyclopentane heptenecyanide-5-cis-2-(3-αhydroxy-5-phenyl-1-trans-pentenyl)-3,5-dihydroxy, [1α,2β,3α,5α]
Cyclopentane hepteneazide-5-cis-2-(3-αhydroxy-5-phenyl-1-trans-pentenyl)-3,5-dihydroxy, [1α,2β,3α,5α]
Cyclopentane heptene-5-cis-2-(3-αhydroxy-5-phenyl-1-transpentenyl)-3,5-dihydroxy, [1α,2β,3α,5α]
Cyclopentane N-isopropylheptenamide-5-cis-2-(3-αhydroxy-5-phenyl-1-trans-pentenyl)-3,5-dihydroxy, [1α,2β,3α,5α]
Cyclopentane N-methylheptenamide-5-cis-2-(3-αhydroxy-5-phenyl-1-trans-pentenyl)-3,5-dihydroxy, [1α,2β,3α,5α]
Cyclopentaneheptenamide-5-cis-2-(3-αhydroxy-4-m-chlorophenoxy-1-trans-butenyl)-3,5-dihydroxy, [1α,2β,3α,5α]

An estrogen receptor antagonist as an active ingredient of the prophylactic or therapeutic agent for ALS in the present invention includes all of naturally-occurring substances derived from microorganism etc., semi-synthetic substances derived therefrom, and fully synthetic compounds. Examples thereof include, but are not limited to, Raloxifene, an analog thereof and the like.

Examples of the "analog of Raloxifene" include a compound represented by the following formula (V): 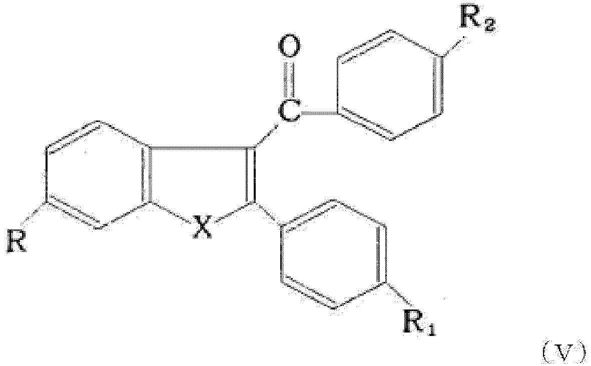 wherein
X is -S- or -S(O)-;
R is a hydrogen atom, a hydroxy group or a C₁-C₅ alkoxy group;
R₁ is a hydrogen atom, a hydroxy group, a C₁-C₅ alkoxy group, a C₁-C₅ acyloxy group, a C₁-C₅ alkoxycarbonyloxy group, a benzoyloxy group, adamantoyloxy group, a chlorine atom, a bromine atom, or -O(CH₂)₂N(R₃)R₄;
R₂ is a hydrogen atom, a hydroxy group, a C₁-C₅ alkoxy group, or -O(CH₂)₂N(R₃)R₄; and
R₃ and R₄ are each independently a C₁-C₄ alkyl group, or R₃ and
R₄ are bonded to the adjacent nitrogen atom to form a hetero ring selected from pyrrolidino, piperidino, hexamethyleneimino and morpholino groups.

Each term described as higher concept in the explanation of each group in the above-mentioned formula (V) (e.g., "C₁-C₅ alkoxy group" for R, R₁ and R₂, "C₁-C₅ acyloxy group", "C₁-C₅ alkoxycarbonyloxy group" for R₁, "C₁-C₄ alkyl group" for R₃ and R₄ etc.) is as defined in JP-A-52-53851.

Specific examples of the analog of Raloxifene include the compounds described in Table 1 (excerpt from the tables in JP-A-52-53851 with partial modification).

**[Table 1-1]**

| 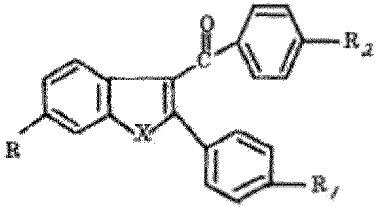 | | | |
|---|---|---|---|
| compound | | | |
| R | R₁ | R₂ | X |
| -OH | H | H | S |
| H | H | -OH | S |
| -OCH*₃* | H | -OCH*₃* | S |
| -OH | H | --OR | S |
| -OH | -OH | H | S |
| -OH | -OH | -OCH*₃* | S |
| H | 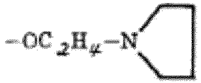 | -OCH*₃* | S |

**[Table 1-2]**

| compound | | | |
|---|---|---|---|
| R | R*₁* | R*₂* | X |
| H | -OC*₂*H*₄*N(C*₂*H*₅*)*₂* | -OCH*₃* | S |
| H | 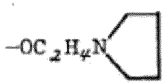 | 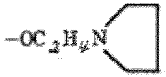 | S |
| H | -OCH*₃* | 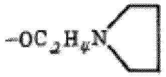 | S |
| H | H | 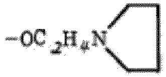 | S |
| H | 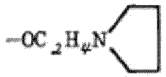 | -OCH*₃* | S |
| H | 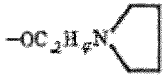 | -OCH*₃* | S |
| -OCH*₃* | -OCH*₃* | 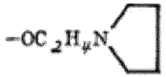 | S |
| H | -OH | 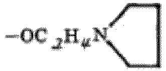 | S |
| H | -O-CO-CH*₃* | 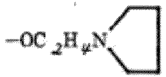 | S |

**[Table 1-3]**

| compound | | | |
|---|---|---|---|
| R | R*₁* | R*₂* | X |
| -OCH*₃* | H | 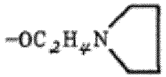 | S |
| H | -OCH*₃* | 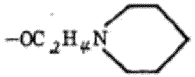 | S |
| H | -OCH*₃* | 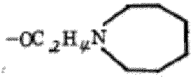 | S |
| H | -OCH*₃* | -OC*₂*H*₄*N-(CH(CH*₃*)*₂*)*₂* | S |
| H | Cl | 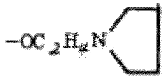 | S |
| H | -O-CO-CH*₂*CH*₃* | 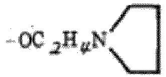 | S |
| H | -O-CO-O-CH*₂*CH*₃* | 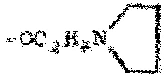 | S |
| H | -O-CO-(CH*₂*)*₃*CH*₃* | 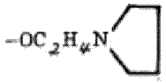 | S |
| H | 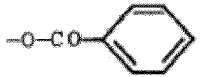 | 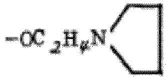 | S |
| H | -O-CO-adamantyl | 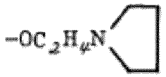 | S |

As the active ingredient of the prophylactic or therapeutic agent for ALS of the present invention, which does not belong to any of the above-mentioned kinase inhibitor, prostaglandin analogue, and estrogen receptor antagonist, Tivozanib, SB 216763, Cdk2 Inhibitor II, BUDESONIDE, RIBOFLAVIN, alpha-TOCHOPHEROL, AMODIAQUINE, SU9516, Sunitinib, GSK-3 Inhibitor XIII, Bisindolylmaleimide I, HYDROQUINONE, FLUNISOLIDE, MGCD-265, Indirubin-3'-monoxime, HYDRASTINE (1R,9S), PIPERINE, BUTAMBEN, Axitinib, APOMORPHINE, FENBUFEN, Bosutinib (SKI-606), Wee1 Inhibitor and Cdk2 Inhibitor IV, NU6140 (see Tables 4-1 - 4-5), 3-hydroxybutyric acid (see Table 7), Imatinib, Nilotinib, Rebastinib, Bafetinib (see Table 8), and analogs of Tivozanib, analogs of Sunitinib, analogs of Axitinib and analogs of Bosutinib (SKI-606) can be mentioned.

Here, the "analog of Tivozanib" is, for example, a compound represented by the following formula (VI): 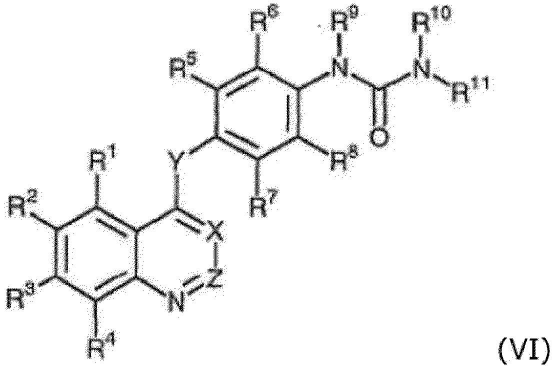 wherein
X and Z are each CH or N,
Y is 0 or S,
R¹, R² and R³ are each independently a hydrogen atom, a C₁₋₆ alkyl group, a C₁₋₆ alkoxy group, a C₂₋₆ alkenyl group, a C₂₋₆ alkynyl group, a nitro group, or an amino group, wherein the C₁₋₆ alkyl group, C₁₋₆ alkoxy group, C₂₋₆ alkenyl group, and C₂₋₆ alkynyl group are optionally substituted by a halogen atom, a hydroxyl group, a C₁₋₄ alkyl group, a C₁₋₄ alkoxycarbonyl group, an amino group (1 or 2 hydrogen atoms of the amino group may be each substituted by a C₁₋₄ alkyl group (the C₁₋₄ alkyl group is optionally substituted by a hydroxyl group or a C₁₋₄ alkoxy group)), a group R¹²R¹³N-C(=0)-O-(R¹² and R¹³ are each independently a hydrogen atom or a C₁₋₄ alkyl group (the C₁₋₄ alkyl group is optionally substituted by a hydroxyl group or a C₁₋₄ alkoxy group)), or group R¹⁴-(S)ₘ-(R¹⁴ is a saturated or unsaturated 3-7-membered carbon cyclic group or heterocyclic group optionally substituted by a C₁₋₄ alkyl group, m is 0 or 1), R⁴ is a hydrogen atom,
R⁵, R⁶, R⁷ and R⁸ are each independently a hydrogen atom, a halogen atom, a C₁₋₄ alkyl group, a C₁₋₄ alkoxy group, a C₁₋₄ alkylthio group, a trifluoromethyl group, a nitro group, or an amino group,
R⁹ and R¹⁰ are each independently a hydrogen atom, a C₁₋₆ alkyl group or a C₁₋₄ alkylcarbonyl group, wherein the alkyl moiety of the C₁₋₆ alkyl group or C₁₋₄ alkylcarbonyl group is optionally substituted by a halogen atom, a C₁₋₄ alkoxy group, an amino group (the amino group is optionally substituted by a C₁₋₄ alkyl group optionally substituted by a C₁₋₄ alkoxy group), or a saturated or unsaturated 3-7-membered carbon cyclic group or heterocyclic group,
R¹¹ is an azolyl group, one or more hydrogen atoms on the azolyl group are optionally substituted by a halogen atom, a C₁₋₄ alkyl group, a C₁₋₄ alkoxy group, a C₁₋₄ alkylthio group, a trifluoromethyl group, a nitro group, an amino group (1 or 2 hydrogen atoms on the amino group are each optionally substituted by a C₁₋₄ alkyl group), a C₁₋₄ alkoxycarbonyl C₁₋₄ alkyl, C₁₋₄ alkylcarbonyl, or C₃₋₅ cyclic alkyl group.

Each term described as higher concept in the explanation of each group in the above-mentioned formula (VI) (e.g., "C₁₋₆ alkyl group", "C₁₋₆ alkoxy group", "C₂₋₆ alkenyl group", "C₂₋₆ alkynyl group", "C₁₋₄ alkyl group", "C₁₋₄ alkoxycarbonyl group", "C₁₋₄ alkoxy group", "saturated or unsaturated 3-7-membered carbon cyclic group or heterocyclic group" etc. for R¹, R² and R³) is as defined in WO 02/088110.

In the above-mentioned formula (VI), R¹¹ is preferably a group represented by the following formula (i)-(iv): 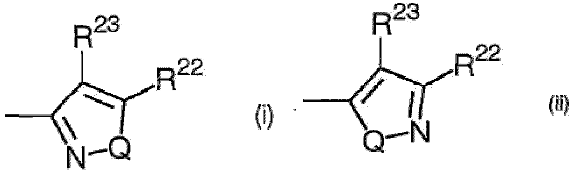 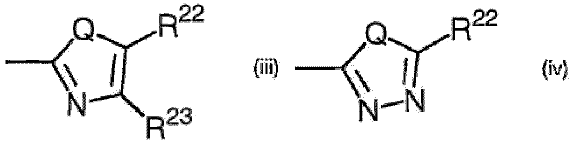 wherein
Q is O, S or NH,
R²² and R²³ are each independently a hydrogen atom, a halogen atom, a C₁₋₄ alkyl group, a C₁₋₄ alkoxy group, a C₁₋₄ alkylthio group, a trifluoromethyl group, a nitro group, an amino group (1 or 2 hydrogen atoms on the amino group are each optionally substituted by a C₁₋₄ alkyl group), C₁₋₄ alkoxycarbonyl C₁₋₄ alkyl, C₁₋₄ alkylcarbonyl, or C₃₋₅ cyclic alkyl group.

Specific examples of the analog of Tivozanib include the compounds shown by the following formula and Tables 2-1 - 2-3 (excerpt from Table 1 in WO 02/088110 with partial modification).

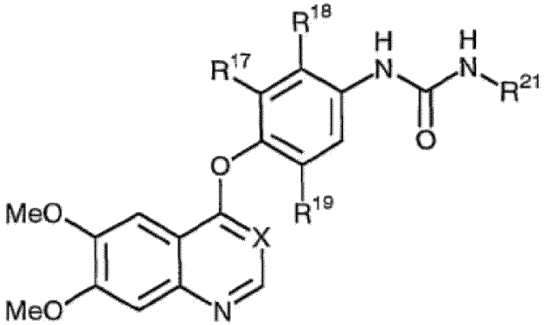

**[Table 2-1]**

| compound | X | R¹⁷ | R¹⁸ | R¹⁹ | R²¹ | Q | R²² | R²³ |
|---|---|---|---|---|---|---|---|---|
| 1 | CH | Cl | H | H | (i) | O | H | H |
| 2 | CH | Cl | H | H | (ii) | O | CH₃ | H |
| 3 | CH | F | H | H | (i) | O | H | H |
| 4 | CH | H | Cl | H | (i) | O | CH₃ | H |
| 5 | CH | H | Cl | H | (ii) | O | CH₃ | H |
| 6 | CH | H | F | H | (ii) | O | CH₃ | H |
| 7 | CH | F | H | H | (ii) | O | CH₃ | H |
| 8 | CH | F | H | H | (i) | O | CH₃ | H |
| 9 | CH | H | F | H | (i) | O | CH₃ | H |
| 10 | N | H | H | H | (i) | O | CH₃ | H |
| 11 | N | H | H | H | (ii) | O | CH₃ | H |
| 12 | N | H | Cl | H | (i) | O | CH₃ | H |
| 13 | N | H | Cl | H | (ii) | O | CH₃ | H |
| 14 | N | Cl | H | H | (i) | O | CH₃ | H |
| 15 | N | Cl | H | H | (ii) | O | CH₃ | H |
| 16 | N | H | H | H | (ii) | S | CH₃ | H |
| 17 | N | H | Cl | H | (ii) | S | CH₃ | H |
| 18 | N | Cl | H | H | (ii) | S | CH₃ | H |
| 19 | OH | Cl | H | H | (ii) | NH | H | H |
| 20 | CH | H | F | H | (ii) | NH | H | H |
| 21 | CH | H | F | H | (iii) | S | CH₃ | H |
| 22 | CH | H | Cl | H | (iii) | S | H | CH₃ |
| 23 | CH | H | F | H | (iii) | S | CH₃ | CH₃ |
| 24 | CH | H | Cl | H | (iii) | S | CH₃ | CH₃ |
| 25 | CH | Cl | H | H | (iii) | S | CH₃ | CH₃ |
| 26 | CH | H | CF₃ | H | (iii) | S | CH₃ | CH₃ |

**[Table 2-2]**

| | | | | | | | | |
|---|---|---|---|---|---|---|---|---|
| 27 | CH | H | F | H | (iii) | S | H | H |
| 28 | CH | H | F | H | (iii) | S | H | CH₃ |
| 29 | CH | H | F | H | (iii) | S | H | A |
| 30 | CH | H | F | H | (iii) | S | H | tBu |
| 31 | CH | H | Cl | H | (iii) | S | H | A |
| 32 | CH | H | Cl | H | (iii) | S | Br | H |
| 33 | CH | H | Cl | H | (iii) | S | H | tBu |
| 34 | CH | H | Cl | H | (iii) | S | Cl | H |
| 35 | CH | H | F | H | (iii) | S | Br | H |
| 36 | CH | H | F | H | (iii) | S | Ac | CH₃ |
| 37 | CH | H | F | H | (iii) | S | Cl | H |
| 38 | N | H | Cl | H | (iii) | S | H | H |
| 39 | N | H | Cl | H | (iii) | S | CH₃ | H |
| 40 | N | H | Cl | H | (iii) | S | CH₃ | CH₃ |
| 41 | N | H | Cl | H | (iii) | S | H | CH₃ |
| 42 | N | H | H | H | (iii) | S | H | H |
| 43 | N | H | H | H | (iii) | S | CH₃ | H |
| 44 | N | H | H | H | (iii) | S | H | CH₃ |
| 45 | N | H | H | H | (iii) | S | CH₃ | CH₃ |
| 46 | N | Cl | H | H | (iii) | S | H | H |
| 47 | N | Cl | H | H | (iii) | S | CH₃ | H |
| 48 | N | Cl | H | H | (iii) | S | H | CH₃ |
| 49 | N | Cl | H | H | (iii) | S | CH₃ | CH₃ |
| 50 | CH | H | H | H | (iv) | S | CF₃ | H |
| 51 | CH | H | F | H | (iv) | S | CF₃ | H |
| 52 | CH | F | H | H | (iv) | S | CF₃ | H |
| 53 | CH | CH₃ | CH₃ | H | (iv) | S | CF₃ | H |

**[Table 2-3]**

| | | | | | | | | |
|---|---|---|---|---|---|---|---|---|
| 54 | CH | H | H | H | (iv) | S | CH₃ | H |
| 55 | CH | H | CH₃ | H | (iv) | S | CH₃ | H |
| 66 | CH | CH₃ | H | H | (iv) | S | CH₃ | H |
| 57 | CH | CH₃ | CH₃ | H | (iv) | S | CH₃ | H |
| 58 | CH | H | F | H | (iv) | S | CH₃ | H |
| 59 | CH | H | H | H | (iv) | S | Et | H |
| 60 | CH | H | CH₃ | H | (iv) | S | Et | H |
| 61 | CH | CH₃ | H | H | (iv) | S | Et | H |
| 62 | CH | CH₃ | CH₃ | H | (iv) | S | Et | H |
| 63 | CH | H | F | H | (iv) | S | Et | H |
| 64 | CH | F | H | H | (iv) | S | Et | H |
| 65 | CH | H | Cl | H | (iv) | S | Et | H |
| 66 | CH | Cl | H | H | (iv) | S | Et | H |
| 67 | CH | H | Cl | H | (iv) | S | cPr | H |
| 68 | CH | Cl | H | H | (iv) | S | cPr | H |
| 69 | CH | H | CH₃ | CH₃ | (iv) | S | cPr | H |
| 70 | CH | H | F | H | (iv) | S | EtS | H |
| 71 | CH | CH₃ | CH₃ | H | (iv) | S | EtS | H |
| 72 | CH | H | Cl | H | (iv) | S | CF₃ | H |
| 73 | CH | Cl | H | H | (iv) | S | CF₃ | H |
| 74 | CH | H | F | H | (iv) | S | tBu | H |
| 75 | CH | CH₃ | CH₃ | H | (iv) | S | tBu | H |

In Tables 2-1 - 2-3, A is ethoxycarbonylmethyl, tBu is t-butyl, Ac is acetyl, Et is ethyl, cPr is cyclopropyl, and EtS is ethylthio, respectively.

Examples of the analog of Pazopanib include a 3-pyrrole-substituted 2-indolinone compound represented by the following formula (VII): 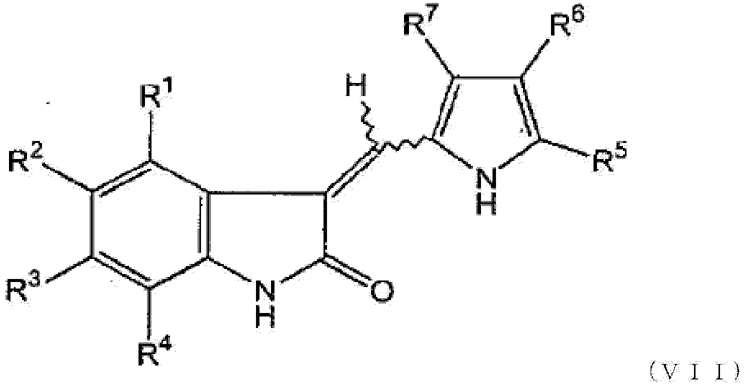 wherein
R₁ is selected from the group consisting of hydrogen, halo, alkyl, cycloalkyl, aryl, heteroaryl, heteroalicyclic, hydroxy, alkoxy, -(CO)R¹⁵, -NR¹³R¹⁴, - (CH₂)ᵣR¹⁶ and -C(O)NR⁸R⁹,
R² is selected from the group consisting of hydrogen, halo, alkyl, trihalomethyl, hydroxy, alkoxy, cyano, -NR¹³R¹⁴, -
NR¹³C(O)R¹⁴, -C(O)R¹⁵, aryl, heteroaryl, -S(O)₂NR¹³R¹⁴ and -SO₂R²⁰ (wherein R²⁰ is alkyl, aryl, aralkyl, heteroaryl or heteroaralkyl),
R₃ is hydrogen, halogen, alkyl, trihalomethyl, hydroxy, alkoxy, - (CO)R¹⁵, -NR¹³R¹⁴, aryl, heteroaryl, -NR¹³S(O)₂R¹⁴, -S(O)₂NR¹³R¹⁴ -NR¹³C(O)R¹⁴, -NR¹³C(O)OR¹⁴ and -SO₂R²⁰ (wherein R²⁰ is selected from the group consisting of alkyl, aryl, aralkyl, heteroaryl and heteroaralkyl),
R⁴ is selected from the group consisting of hydrogen, halogen, alkyl, hydroxy, alkoxy and -NR¹³R¹⁴,
R⁵ is selected from the group consisting of hydrogen, alkyl and -C(O)R¹⁰,
R⁶ is selected from the group consisting of hydrogen, alkyl and -C(O)R¹⁰,
R⁷ is selected from the group consisting of hydrogen, alkyl, aryl, heteroaryl, -C(O)R¹⁷ and -C(O)R¹⁰, or R⁶ and R⁷ may be joined to form a group selected from the group consisting of - (CH₂)₄-, - (CH₂)₅- and -(CH₂)₆-;
at least one of R⁵, R⁶ and R⁷ should be -C(O)R¹⁰;
R⁸ and R⁹ are each independently selected from the group consisting of hydrogen, alkyl and aryl,
R¹⁰ is selected from the group consisting of hydroxy, alkoxy, aryloxy, -N(R¹¹) (CH₂)ₙR¹² and -NR¹³R¹⁴
R¹¹ is selected from the group consisting of hydrogen and alkyl, R¹² is selected from the group consisting of -NR¹³R¹⁴, hydroxy, - C(O)R¹⁵, aryl, heteroaryl, -N⁺(O-)R¹³R¹⁴, N(OH)R³, and -NHC(O)R^{a} (wherein R^{a} is unsubstituted alkyl, haloalkyl, or aralkyl),
R¹³ and R¹⁴ are each independently selected from the group consisting of hydrogen, alkyl, cyanoalkyl, cycloalkyl, aryl and heteroaryl, or R¹³ and R¹⁴ may be joined to form a heterocyclic group;
R¹⁵ is selected from the group consisting of hydrogen, hydroxy, alkoxy and aryloxy,
R¹⁶ is selected from the group consisting of hydroxy, -C(O)R¹⁵, -NR¹³R¹⁴ and -C(O)NR¹³R¹⁴,
R¹⁷ is selected from the group consisting of alkyl, cycloalkyl, aryl and heteroaryl,
R²⁰ is alkyl, aryl, aralkyl or heteroaryl; and
n and r are each independently 1, 2, 3, or 4.

In the above-mentioned formula (VII), preferably,
R¹ is selected from the group consisting of hydrogen, halo, alkyl, cycloalkyl, aryl, heteroaryl, heteroalicyclic, hydroxy, alkoxy, -C(O)R¹⁵, -NR¹³R¹⁴-(CH₂)ᵣR¹⁶ and -C(O)NR⁸R⁹,
R² is selected from the group consisting of hydrogen, halo, alkyl, trihalomethyl, hydroxy, alkoxy, -NR¹³R¹⁴, -NR¹³C(O)R¹⁴, - C(O)R¹⁵, aryl, heteroaryl, and -S(O)₂NR¹³R¹⁴,
R³ is selected from the group consisting of hydrogen, halogen, alkyl, trihalomethyl, hydroxy, alkoxy, -(CO)R¹⁵, -NR¹³R¹⁴, aryl, heteroaryl, -NR¹³S (O) ₂R¹⁴, -S (O) ₂NR¹³R¹⁴, -NR¹³C (O) R¹⁴, and - NR¹³C (O) OR¹⁴,
R⁴ is selected from the group consisting of hydrogen, halogen, alkyl, hydroxy, alkoxy and -NR¹³R¹⁴,
R⁵ is selected from the group consisting of hydrogen, alkyl and -C (O) R¹⁰,
R⁶ is selected from the group consisting of hydrogen, alkyl and -C (O) R¹⁰,
R⁷ is selected from the group consisting of hydrogen, alkyl, aryl, heteroaryl, -C(O)R¹⁷ and -C(O)R¹⁰,
R⁶ and R⁷ may be joined to form a group selected from the group consisting of -(CH₂)₄-, -(CH₂)₅- and -(CH₂)₆-;
at least one of R⁵, R⁶ and R⁷ should be -C(O)R¹⁰;
R⁸ and R⁹ are each selected from the group consisting of independently hydrogen, alkyl and aryl,
R¹⁰ is selected from the group consisting of hydroxy, alkoxy, aryloxy, -N (R¹¹) (CH₂) ₙR¹² and -NR¹³R¹⁴,
R¹¹ is selected from the group consisting of hydrogen and alkyl,
R¹² is selected from the group consisting of -NR¹³R¹⁴, hydroxy, - C(O)R¹⁵, aryl and heteroaryl,
R¹³ and R¹⁴ are each independently selected from the group consisting of hydrogen, alkyl, cycloalkyl, aryl and heteroaryl,
R¹³ and R¹⁴ may be joined to form a group selected from the group consisting of - (CH₂) ₄-, -(CH₂)₅-, - (CH₂) ₂O (CH₂)₂-, and (CH₂) ₂N (CH₃) (CH₂) ₂-;
R¹⁵ is selected from the group consisting of hydrogen, hydroxy, alkoxy and aryloxy,
R¹⁶ is selected from the group consisting of hydroxy, -C(O)R¹⁵, -NR¹³R¹⁴ and -C (O) NR¹³R¹⁴,
R¹⁷ is selected from the group consisting of alkyl, cycloalkyl, aryl and heteroaryl, and
n and r are each independently 1, 2, 3, or 4.

Each term described as higher concept in the explanation of each group in the above-mentioned formula (VII) (e.g., "halo", "alkyl", "cycloalkyl", "aryl", "heteroaryl", "heteroalicycle", "alkoxy" etc. for R¹) is as defined in WO 01/060814 (National Publication of International Patent Application No. 2003-523340).

Specific examples of the analog of Sunitinib include the compounds shown by Tables 3-1 - 3-25 (excerpt from Tables 1 - 25 in National Publication of International Patent Application No. 2003-523340 with partial modification).

**[Table 3-1]**

| **compound** | Structure | Name |
|---|---|---|
| 1 | 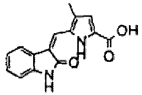 | 4-Methyl-5-(2-oxo-1,2-dihydroindol-3-ylidenemethyl)-1H-pyrmole-2-carboxylic acid |
| 2 | 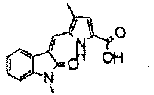 | 4-Methyl-5-(1-methyl-2-oxo-1,2-dihydroindol-3-ylidenemethyl)-1H-pyrrole.2 carboxylic acid |
| 3 | 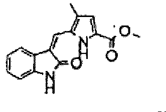 | 4-Methyl-5-(2-oxo-1,2-dihydroindol-3-ylidenemethyl)-1H-pyrrole-2-carboxylic acid methyl ester |
| 4 | 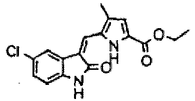 | 5-(5-Chloro-2-oxo-1,2-dihydroindol-3-ylidenernethyl)-4-methyl-1H-pyrrole-2 carboxylic acid ethyl ester |
| 5 | 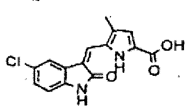 | 5-(S-Chtero-2-oxo-1,2-dihydroindoi-3-yltdenemethyl)-4-methyl-1H-pyrrale-2 carboxylic acid |
| 6 | 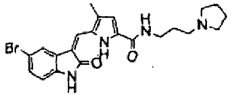 | 5-(5-Bromo-2-oxo-1,2-dihydroindob3-ylidenemethyl)-4-methyl-1H-pyrrole-2 carboxylic *acid* (3-pyrrolidin-1-ylpropyl)amide |
| 7 | 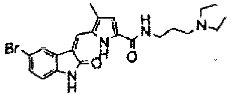 | 6-(5-Bromo-2-oxo-1,2-dihydroindol-3-ylidenemelhyl)-4-methyl-1H-pyrrole-2 carboxylic acid (3-diethylaminopropyljamide |
| 8 | 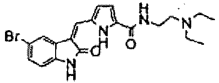 | 5-(5-Bromo-2-oxo-1,2-dihydroindol-3-ylidenemethyl)-1H-pyrrole-2-carboxylic acid (2-diethylaminoethyl)amide |
| 9 | 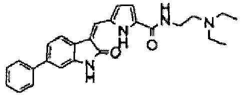 | 5-(2-Oxo-6-phenyl-1,2-dihydroindo-3-yidenemethyl)-1H-pyrrole-2-carboxylic acid (2-diethylaminoethyl)amide |

**[Table 3-2]**

| | | |
|---|---|---|
| 10 | 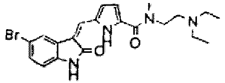 | 5-(5-Bromo-2-oxo-1,2-dihydroindol-3-ylidenemethyl)-1H-pyrrole-2-carboxylic acid (2-diethylaminoethyl)methylamide |
| 11 | 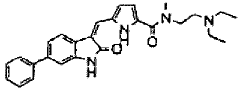 | 5-(2-Oxo-6-phenyl-1,2-dihydroindol-3-ylidenemethyl)-1H-pyrrole-2-carboxylic acid (2-diethylaminoethyl)methylamide |
| 12 | 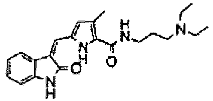 | 3-Methyl-5-(2-oxo-1,2-dihydrolodol-3-ytidenemethyl)-1H-pyrrole-2-carboxylic acid (3-diethylaminopropyl)amide |
| 13 | 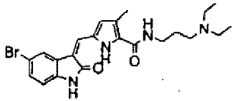 | 5-(5-Bromo-2-oxo-1,2dihydroindol-3-ylidenemethyl)-3-methyl-1H-pyrrole-2 carboxylic acid (3-diethylaminopropyl)amide |
| 14 | 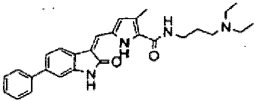 | 3-Methyl-5-(2-oxo-6-phenyl-1,2-dihydroindol-3-ylidenemethyl)-1H-pyrrole-2 carboxylic acid (3-diethylaminapropyl)amide |
| 15 | 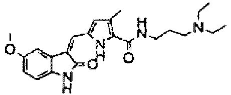 | 5-(5-Methoxy-2-oxo-1,2-dihydroindol-3-ylidenemethyl)-3-methyl-1H-pyrrole 2-carboxylic acid (3-diethylaminopropyl)amide |
| 16 | 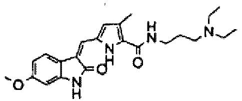 | 5-(6-Methoxy-2-oxo-1,2-dihydroindol4ylidenemethyl)-3-methyl-1H-pyrrole 2-carboxylic acid (3-diethylaminopropyl)amide |
| 17 | 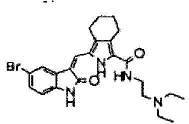 | 3-(5-Bromo-2-oxo-1,2-dihydroindol-3-ylidenemathyl)-4,5,6,7-tetrahydro-2H-isoindole-1-carboxylic acid (2-diethylaminoethyl)amide |
| 18 | 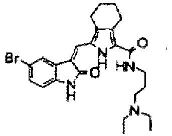 | 3-(5-Bromo-2-oxo-1,2-dihydroindol-3-ylidenemethyl)-4,5,6,7-tetrahydro-2H-isoindole-1-carboxylic acid (3-diethylaminopropyl)amide |

**[Table 3-3]**

| | | |
|---|---|---|
| 19 | 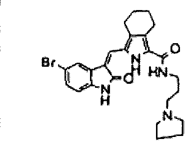 | 3-(5-Bromo-2-oxo-1,2-dihydraindol-3-ylidenemethyl)-4.5,6,7-tetrahydro-2H isoindole-1-carboxylic acid (3-pyrrolidin-1-ylpropyl)amide |
| 20 | 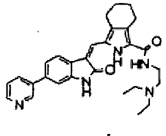 | 3-(2-Oxo-6-pyridin-3-yl-1,2-dihydroindol-3-ylidenemethyl)-4,5,6,7-tetrahydro-2H-isoindole-1-carboxyric acid (2-diethylaminoethyl)amide |
| 21 | 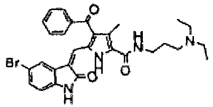 | 4-Benzoyl-5-(5-bromo-2-oxo-1,2-dihydroindol-3-ylidenemethl)-3-methyl-1H-pyrrole-2-carboxylic acid (3-diethylaminopropyl) amide |
| 22 | 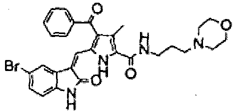 | 4-Benzoyl-5-(5-bromo-2-oxo-1,2-dihydroindol-3-ylidenemethyl)-3-methyl-1H-pyrrole-2-carboxylic acid (3-morpholin-4-yipropyl)amide |
| 23 | 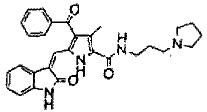 | 4-Benzoyl-3-methyl-5-(2-oxo-1,2-dihydroindol-3-ylidenemethyl)-1H-pyrrole-2-carboxylic acid (3-pyrrolidin-1-ylpropyl)amide |
| 24 | 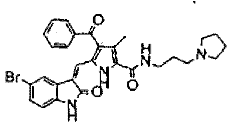 | 4-Benzoyl-5-(5-bromo-2-oxo-1,2-dihydraindol-3-ylidenemethyl)-3-methyl-1H-pyrrole-2-carboxylic acid (3-pyrrolidin-1-ylpropyl)amide |
| 25 | 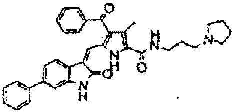 | 4-Benzoyl-3-mathyl-5-(2-oxo-6-phenyl-1,2-dihydroindol-3-ylidenemethyl)-1H-pyrrole-2-carboxylic acid (3-pyrrolidin-1-ylpropyl)amide |
| 26 | 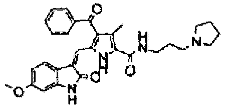 | 4-Benzoyl-5-(6-methoxy-2-oxo-1,2-dihydroindal-3-ylidenemethyl)-3-methyl-1H-pyrrole-2-carboxylic acid (3-pyrrolidin-1-ylpropyl)amide |

**[Table 3-4]**

| | | |
|---|---|---|
| 27 | 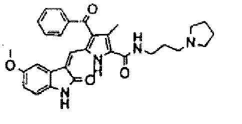 | 4-Benzoyl-5-(5-methoxy-2-oxo-1,2-dihydroindol-3-ylidenemethyl)-3-methyl-1H-pyrrole-2-carboxylic acid (3-pyrrolidin-1-ylpropyl)amide |
| 28 | 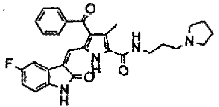 | 4-Benzoyl-5-(5-fluoro-2-oxo-1,2-dihydroindol-3-ylidenemethyl)-3-methyl-1H pyrrole-2-carboxylic acid (3-pyrrolidin-1-ylpropyl)amide |
| 29 | 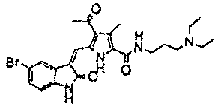 | 4-Acetyl-5-(5-bromo-2-oxo-1,2-dihydroindol-3-ylidenemethyl)-3-methyl-H-pyrrole-2-carboxylic acid (3-diethylaminopropyl)amide |
| 30 | 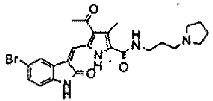 | 4-Acetyl-5-(5-bromo-2-oxo-1,2-dihydroindol-3-ylidenemethyl)-3-methyl-1H-pyrrole-2-carboxylic acid (3-pyrrolidin-1-ylopropyl)amide |
| 31 | 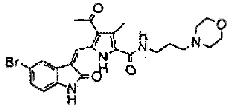 | 4-Acetyl-5-(5-bromo-2-oxo-1,2-dihydroindol-3-ylidenemethyl)-3-methyl-1H-pyrrole-2-carboxylic acid (3-morpholin-4-ylpropyl)amide |
| 32 | 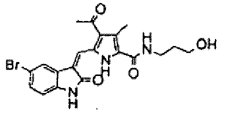 | 4-Acetyl-5-(5-bromo-2-oxo-1,2-dihydroindol-3-ylidenemethyl)-3-methyl-1H-pyrrole-2-carboxylic acid (3-hydroxy-propyl)amide |
| 33 | 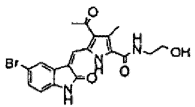 | 4-Acetyl-5-(5-bromo-2-oxo-1,2-dihydroindol-3-ylidenemethyl)-3-methyl-1H-pyrrole-2-carboxylic acid (2-hydroxy-ethyl)amide |
| 34 | 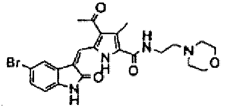 | 4-Acetyl-5-(5-bromo-2-oxo-1,2-dihydroindol-3-ylidenemethyl)-3-melhyl-1H-pyrrole-2-carboxylic acid (2-morpholin-4-yl-ethyl)amide |
| 35 | 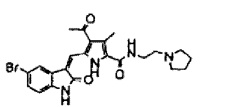 | 4-Acetyl-5-(5-bromo-2-oxo-1,2-dihydroindol-3-ylidenemethyl)-3-methyl-1H-pyrrole-2-carboxylic acid (2-pyrrolidin-1-yl-ethyl)amide |

**[Table 3-5]**

| | | |
|---|---|---|
| 36 | 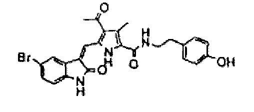 | 4-Acetyl-5-(5-bromo-2-oxo-1,2-dihydroindoi-3-ylidenemethyl)-3-melhyl-1H-pyrrole-2-carboxylic acid [2-(4-hydroxy-phenyl)-ethyl]amide |
| 37 | 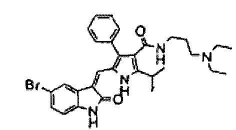 | 5-(5-Bromo-2-oxo-1,2-dihydroindol-3-ylidenemethyl)-2-jsopropyl-4-phenyl-1H-pyrrole-3-carboxylic acid (3-diethylaminopropyl)amide |
| 38 | 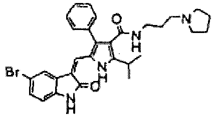 | 5-(5-Bromo-2-oxo-1,2-dihydroindol-3-ylidenemethyl)-2-isopropyl-4-phenyl-1H-pyrrole-3-carboxylic acid (3-pyrrolidin-1-ylpropyl)amide |
| 39 | 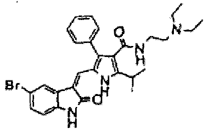 | 5-(5-Bromo-2-oxo-1,2-dihydroindol-3-ylidenemethyl)-2-isopropyl-4-phenyl-1H-pyrrole-3-carboxylic acid (2-diethylaminoethyl)amide |
| 40 | 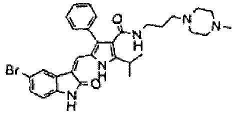 | 5-(5-Bromo-2-oxo-1,2-dihydroindo1-3-ylidenemethyl)-2-isopropyl-4-phenyl-1H-pyrrole-3-carboxylic acid [3-(4-methyl-piperazin-1-yl)-propyl]amide |
| 41 | 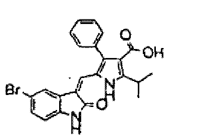 | 5-(5-Bromo-2-oxo-1,2dihydroindol-3-ylidenemethyl)-2-isopropyl-4-phenyl-1H-pyrrole-3-carboxylic acid |
| 42 | 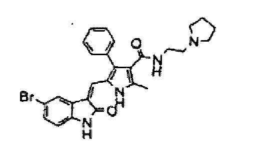 | 5-(5-Bromo-2-oxo-1,2-dihydroindol-3-ylidenemethyl)-2-methyl-4-phenyl-1H-pyrrole-3-carboxylic acid (2-pyrrolidin-1-yl-ethyl)amide |
| 43 | 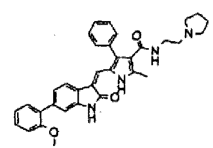 | 5-[6-(2-Methoxy-phenyl)-2-oxo-1,2-dihydroindol-3-ylidenemethyl]-2-methy(-4-phenyl-1H-pyrrale-3-carboxylic acid (2-pyrrolidin-1-yl-ethyl)amide |

**[Table 3-6]**

| | | |
|---|---|---|
| 44 | 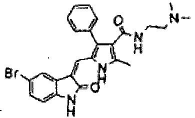 | 5-(5-Bromo-2-oxo-1,2-dihydroindol-3-ylidenemethyl)-2-methyl-4-phenyl-1H pyrrole-3-carboxylic acid (2-dimethylamino-ethyl)amide |
| 45 | 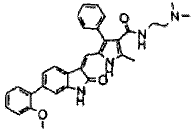 | 5-[6-(2-Methoxy-phenyl)-2-oxo-1,2-dihydroindol-3-ylidenemethyl]-2-methyl-4-phenyl-1H-pyrrole-3-carboxylic acid (2-dimelhylamino-ethyl)amide |
| 46 | 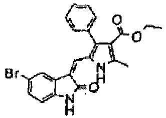 | 5-(5-8romo-2-oxo-1,2-dihydraindol-3-ylidenemethyl)-2-methyl-4-phenyl-1H pyrrole-3-carboxylicacid ethyl ester |
| 47 | 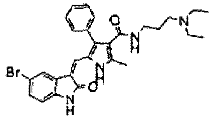 | 5-(5-Bromo-2-oxo-1,2-dihydroindol-3-ylidenemethyl)-2-methyl-4-phenyl-1H pyrrole-3-carboxylic acid (3-diethylaminopropyl)amide |
| 48 | 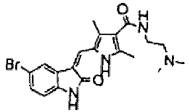 | 5-(5-Bromo-2-oxo-1,2-dihydroindol-3-ylidenemetho)-2,4-dimethl-1H-pyrrole-3-carboxylic acid (2-dimethylamirio-ethyl)amide |
| 49 | 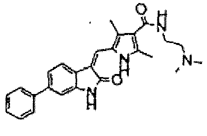 | 2,4-Dimethyl-5-(2-oxo-6-phenyl-1,2-dihydroindel-3-ylidenemethyl)-1H-pyrrale-3-carboxylicacid (2-dimethylamino-ethyl)amide |
| 50 | 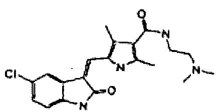 | 5-(5-Chloro-2-oxo-1,2-dihydroindol-3-ylidenemethyl)-2,4-dimethyl- 1H-pyrrole-3-carboxylic acid (2-dimethylamino-ethyl)amide |
| 51 | 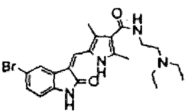 | 5-(5-Bromo-2-oxo-1,2-dihydroindo(-3-ylidenemethyl)-2,4-dimethyl-1H-pyrrole-3-carboxylic acid (2-diethylaminoethyl)amide |

**[Table 3-7]**

| | | |
|---|---|---|
| 52 | 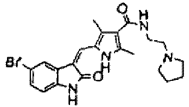 | 5-(5-Bromo-2-oxo- 1,2-dihydroindol-3-ylidenemethyl)-2,4-dimethyl-1H-pyrrole-3-carboxylic acid (2-pyrrolidin-1-yl-ethyl)amide |
| 53 | 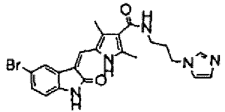 | 5-(5-Bromo-2-oxo-1,2-dihydroindol-3-ylidenemethyl)-2,4-dimethyl-1H-pyrrole-3-carboxylic acid (3-imidazol-1-ylpropyl)amide |
| 54 | 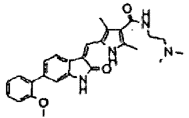 | 5-(6-(2-Methoxy-phenyl)-2-oxo-1,2-dihydroindol-3-ylidenemethlyl)-2,4-dimethyl-1H-pyrrole-3-carboxylic acid (2-dimethylamino-ethyl)amide |
| 55 | 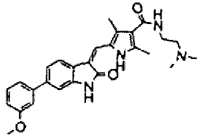 | 5-[6-(3-Methoxy-phenyl)-2-oxo-1,2-dihydroindol-3-ylidenemethyl]-2,4-dimethyl-1H-pyrrole-3-carboxylic acid (2-dimethylamino-ethyl)amide |
| 56 | 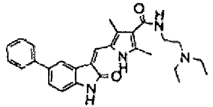 | 2,4-Dimethyl-5-(2-oxo-5-phenyl-1,2-dihydroindol-3-ylidenemethyl)-1H-pyrrole-3-carboxylic acid (2-diethylaminoethyl)amide |
| 57 | 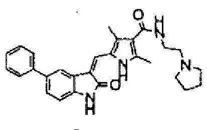 | 2,4-Dimethyl-5-(2-oxo-5-phenyl-1,2-dihydraindol-3-ylidenemethyl)-1H-pyrrole-3-carboxylic acid (2-pyrrotidin-1-yl-ethyl)amide |
| 56 | 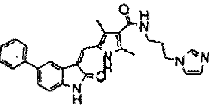 | *2,4-Dimethyl-5-(2-oxo-5-phenyl-1,2-dihydrgindol-3-ylidenemethyl)-1H-*pyrrole-3-carboxylic acid (3-imidazol-1-ylpropyl)amide |
| 59 | 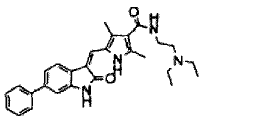 | 2,4-Dimethyl-5-(2-oxo-6-phenyl-1.2-dihydroindol-3-ylidenemethyl)-1H-pyrrote-3-carboxyic acid (2-diethylaminoethyl)amide |
| 60 | 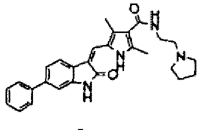 | 2,4-Dimethyl-5-(2-oxo-6-phenyl-1,2-dihydroindol-3-ylidenemethyl)-1H-pyrrole-3-carhoxylic acid (2-pyrrolidin-1-yl-ethyl)amide |

**[Table 3-8]**

| | | |
|---|---|---|
| 61 | 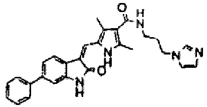 | 2,4-Dimethyl-5-(2-oxo-6-phenyl-1,2-dihydraindol-3-ylidenemethyl)-1H-pyrrole-3-carboxylic acid (3-imidazol-1-ylpropyl)amide |
| 62 | 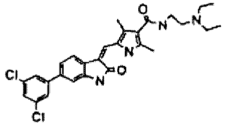 | 5-[6-(3,5-Dichloro-phenyl)-2-oxo-1,2-dihydroindol-3-ylidenemethyl]-2,4-dimethyl-1H-pyrrole-3-carboxylic acid (2-diethylaminoethyl)amide |
| 63 | 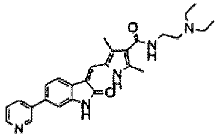 | 2,4-Dimethyl-5-(2-oxo-6-pyridin-3-yl-1,2-dihydroindol-3-ylidenemethyl)-1H-pyrrole-3-carboxylic acid (2-diethylaminoethyl)amide |
| 64 | 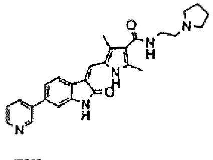 | 2,4-Dimethyl-5-(2-oxo-6-pyridin-3-yl-1,2-dihydroiodol-3-ylidenemethyl)-1H-pyrrole-3-carboxylic acid (2-pyrrolidin-1-yl-ethyl)amide |
| 65 | 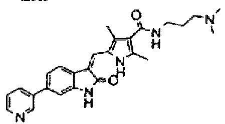 | 2,4-Dimethyl-5-(2-oxo-6-pyridin-3-yl-1,2-dihydroindol-3-ytidenemethyl)-1H-pyrrole-3-carboxylic acid (3-dimelhylamino-propyl)amide |
| 66 | 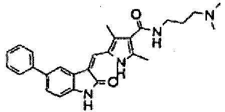 | 2,4-Dimethyl-5-(2-oxo-5-phenyl-1,2-dihydroindol-3-ylidenemethyl)-1H-pyrrole-3-carboxylic acid (3-dimethylamino-propyt)amide |
| 67 | 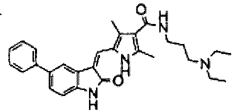 | 2,4-Dimethyl-5-(2-oxo-5-phenyl-1,2-dihydroindol-3-ylidenemethyl)-1H-pyrrole-3-carboxylic acid (3-diethylaminopropyl)amide |
| 68 | 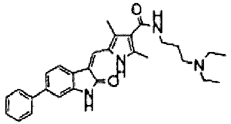 | 2,4-Dimethyl-5-(2-oxo-6-phenyl-1,2-dihydroindol-3-ylidenemethyl)-1H-pyrrole-3-carboxylic acid (3-diethylaminopropyl)amide |

**[Table 3-9]**

| | | |
|---|---|---|
| 69 | 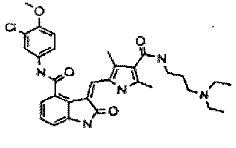 | 3-[4-(3-Diethylamino-propylcarbamoyl)-3,5-dimethyl-1H-pyrrol-2-ylmethylene]-2-oxo-2,3-dihydro-1H-indole-4-carboxylic acid (3-chloro-4-methoxy-phenyl)amide |
| 70 | 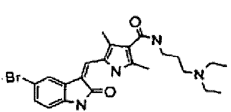 | 5-(5-Bromo-2-oxo-1,2-dihydroindol-3-ylidenemethyl)-2,4-dimethyl-1H-pyrrole-3-carboxylic acid (3-diethylaminopropyl)amide |
| 71 | 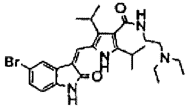 | 5-(5-Bromo-2-oxo-1,2-dihydroindol-3-ylidenemethyl)-2,4-diisopropyl-1H-pyrrole-3-carboxylic acid (2-diethylaminoethyl)amide |
| 72 | 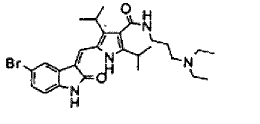 | 5-(5-Bromo-2-oxo-1,2-dihydroindol-3-ylidenemethyl)-2,4-diisopropyl-1H-pyrrole-3-carboxylic acid (3-diethylaminopropyl)amide |
| 73 | 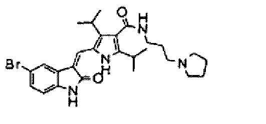 | 5-(6-Bromo-2-oxo-1,2-dihydroindol-3-ylidenemethyl)-2,4-diisopropyl-1H-pyrrole-3-carboxylic acid (3-pyrrofidin-1-ylpropyl)amide |
| 74 | 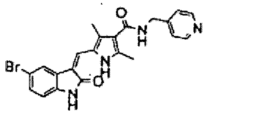 | 5-(5-Bromo-2-oxo-1,2-dihydroindob3-ylidenemethyl)-2,4-dimethyl-1H-pyrrole-3-carboxylic acid (pyridin4-ylmethyl)amide |
| 75 | 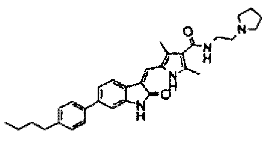 | 5-[6-(4-Butyl-phenyl)-2-oxo-1,2-dihydroindol-3-ylidenemethyl]-2,4-dimethyl-1*H*-pyrrole-3-carboxylic acid (2-pyrrolidin-1-yl-ethyl)amide |
| 76 | 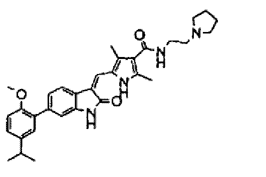 | 5-(6-(5-lsopropyl-2-methoxy-phenyl)-2-oxo-1,2-dihydroindol-3-ylidenemethyl]-2,4-dimethyl-1H-pyrrole-3-carboxylic acid (2-pyrrolidin-1-yl-ethyl)amide |

**[Table 3-10]**

| | | |
|---|---|---|
| 77 | 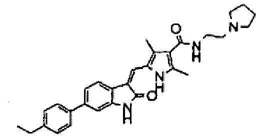 | 5-[6-(4-Elhyl-phenyl)-2-oxo-1,2-dihydroindol-3-ylidenemethyl]-2,4-dimethyl-1H-pyrrole-3-carboxylic acid (2-pyrroljdin-1-yl-ethyl]amide |
| 78 | 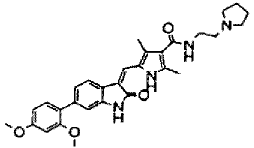 | 5-[6-(2,4-Dimethoxy-phenyl)-2-oxo-1,2-dihydroindol-3-ylidenemethyl)-2,4-dimethyl-1H-pyrrole-3-carboxylic acid (2-pyrrolidin-1-yl-ethyl)amide |
| 79 | 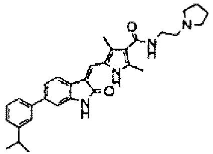 | 5-[6-(3-lsopropyl-phenyl)-2-oxo-1,2-dihydroindol-3-ylidenemethlyl]-2,4-dimelhyl-1H-pyrrole-3-carboxylic acid (2-pyrrolidin-1-yl-ethyl)amide |
| 80 | 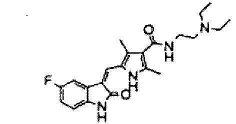 | 5-(5-Fluoro-2-oxo-1,2-dihydroindol-3-ylidenemethyt)-2,4-dimethyl-1H-pyrrole-3-carboxylic acid (2-diethylaminoethyl)amide |
| 81 | 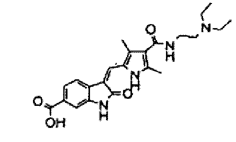 | 3-[4-(2-diethylaminoethylcarbamoyl)-3,5-dimethyl-1H-pyrrol-2-ylmelhylene) 2-oxo-2,3-dihydro-1H-indole-6-carboxylic acid |
| 82 | 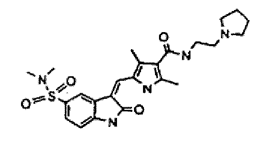 | 5-(5-Dimethylsulfamoyl-2-oxo-1,2-dihydroindol-3-ylidenemethyl)-2,4-dimethyl-1H-pyrrole-3-carboxylic acid (2-pyrrolidin-1-yl-ethyl)amide |
| 83 | 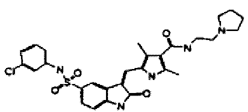 | 5-[5-(3-Chloro-phenylsulfamoyl)-2-oxo-1,2-dihydroindo-3-ylidenemethyl]-2,4-dimethyl-1H-pyrrole-3-carboxylic acid (2-pyrrolidin-1-yl-ethyl)amide |

**[Table 3-11]**

| | | |
|---|---|---|
| 84 | 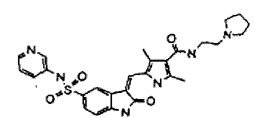 | 2,4-Dimethyl-5-[2-oxo-5-(pyridin-3-ylsulfamaoyl)-1,2-dihydroindol-3-ylidenemethyl]-1H-pyrrole-3-carboxylic acid (2-pyrrolidin-1-yl-ethyl)amide |
| 85 | 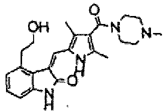 | 3-[3,5-Dimethyl-4-(4-methyl-piperazine-1-carbonyl)-1H-pyrrol-2-ylmethylene]-4-(2-hydroxy-ethyl)-1,3-dihydroindol-2-one |
| 86 | 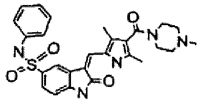 | 3-[3,5-Dimethyl-4-(4-methyl-piperazine-1-carbonyl)-1*H*-pyrrol-2-ylmethylene]-2-oxo-2,3-dihydro-1*H*-indole-5-sulfonic acid phenylamide |
| 87 | 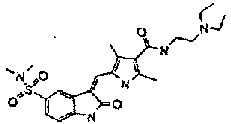 | 5-(5-Dimethylsulfamoyl-2-oxo-1,2-dihydroindol-3-ylidenemethyl)-2,4-dimethyl-1H-pyrrole-3-carboxylic acid (2-diethylaminoethyl)amide |
| 88 | 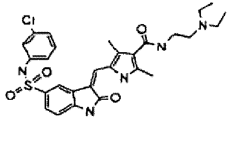 | 5-[5-(3-Chloro-phenyisulfamoyl)-2-oxo-1,2-dihydroindol-3-ylidenemethyl]-2,4-dimethyl-1H-pyrrole-3-carboxylic acid (2-diethylaminoethyl)amide |
| 89 | 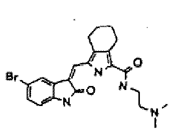 | 3-[5-8romo-2-oxo-1,2-dihydro-indal-3-ylidenemethyl)-4,5,6,7-tetrahydro-*2H*-isoindola-1-carboxylic acid (2-dimethylaming-ethyl)-amide |
| 90 | 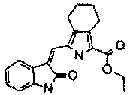 | 3-(2-Oxo-1,2-dihydro-indol-3-ylidenemethyl)-4,5,6,7-tetrahydro-*2H-*isoindole-1-carboxylic acid ethyl ester |
| 91 | 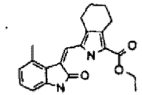 | 3-(4-Methyl-2-oxo-1,2-dihydro-indol-3-ylidenemethyl)-4,5,6,7-tetrahydro-*2H*-isoindole-1-carboxylic acid ethyl ester |

**[Table 3-12]**

| | | |
|---|---|---|
| 92 | 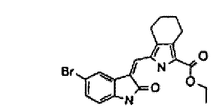 | 3-(5-Bromo-2-oxo-1,2-dihydro-indol-3-ylidenemethyl)-4,5,6, 7-tetrahydro-*2H-*isoindole-1-carboxylic acid ethyl ester |
| 93 | 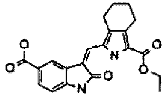 | 3-(3-Ethoxycarbonyl-4,5,6,7-tetrahydro-*2H*-isoindol-1-ylmethylene)-2-oxo-2,3-dihydro-1*H*-indole-5-carboxylic acid |
| 94 | 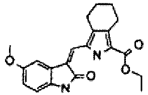 | 3-(5-Methoxy-2-oxo-1,2-dihydro-indol-3-ylidenernethyl)-4,5,6,7-tetrahydro-*2H*-isoindole-1-carboxylic acid ethyl ester |
| 95 | 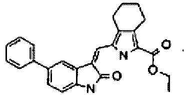 | 3-(2-Oxo-5-phenyl-1,2-dihydro-indol-3-ylidenemethyl)-4,5,6,7-tetrahydro-*2H*-isoindole-1-carboxylic acid ethyl ester |
| 96 | 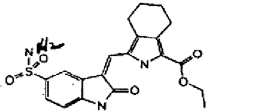 | 3-(2-Oxo-5-sulfamoyl-1,2-dihydro-indol-3-ylidenemethyl)-4,5,6,7-tetrahydro *2H*-isoindole-1-carboxylic acid ethyl ester |
| 97 | 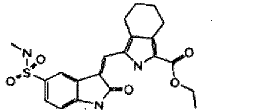 | 3-(5-Methylsulfamoyt-2-oxo-1,2-dihydro-indol-3-ylidenemethyl)-4,5,6,7-tetrahydro-2H-isoindole-1-carboxylic acid ethyl ester |
| 98 | 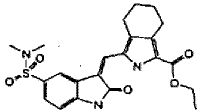 | 3-(5-Dimethylsulfamoyl-2-oxo-1,2-dihydro-indol-3-ylidenemethyl)-4,5,6,7-tetrahydro-2*H*-isoindole-1-carboxylic acid ethyl ester |
| 99 | 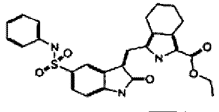 | 3-(2-Oxo-5-phenylsulfamoyl-1,2-dihydro-indol-3-ylidenemethyf)-4,5,6,7-tetrahydro-*2H*-isoindole-1-carboxylic acid ethyl ester |
| 100 | 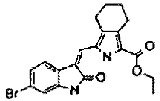 | 3-(6-Bromo-2-oxo-1,2-dihydro-indol-3-ylidenemelhyl)-4, 5,6,7-letrahydro-*2H*-isoindole-1-carboxylic acid ethyl ester |

**[Table 3-13]**

| | | |
|---|---|---|
| 101 | 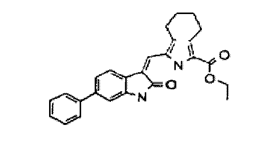 | 3-(2-Oxo-6-phenyl-1,2-dihydro-indol-3-ylidenemethyl)-4,5,6,7-tetrahydro-*2H*-isoindole-1-carboxylic acid ethyl ester |
| 102 | 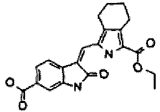 | 3-[3-Ethoxycarbonyl-4,5,6,7-tetrahydro-*2H*-isoindol-1-ylmelhylene)-2-oxo-2,3-dihydro-1H-indole-6-cataxylic acid |
| 103 | 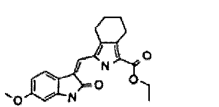 | 3-(6-Methoxy-2-oxo-1,2-dihydro-indol-3-ylidenemelhyl)-4,5,6,7-tetrahydro-*2H*-isoindole-1-carboxylic acid ethyl ester |
| 104 | 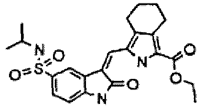 | 3-(5-Isopropylsulfamoyl-2-oxo-1,2-dihydro-indol-3-ylidenemethyl)-4,5,6,7-tetrahydro-*2H*-isoindole-1-carboxylic acid ethyl ester |
| 105 | 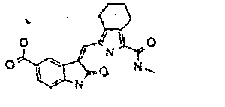 | 3-(3-Methylcarbamoyl-4,5,6,7-tetrahydro-2*H*-isoindol-1-ylmethylene)-2-oxo-2,3-dihydro-1*H*-indole-5-carboxylic acid |
| 106 | 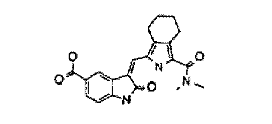 | 3-(3-Dimethylcarbamoyl-4,5,6,7-tetrahydro-2*H*-isoindol-1-ylmethylene)-2-oxo-2,3-dihydro-1*H*-indole-5-carboxylic acid |
| 107 | 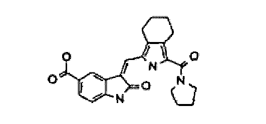 | 2-Oxo-3-13-(pyrrolidine-1-carbonyl)-4,5,6,7-tetrahydro-2*H*-isoindol-1-ylmethylene]-2,3-dihydro-1*H*-indole-5-carboxylic acid |
| 108 | 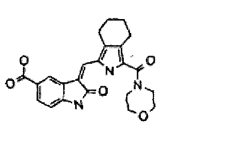 | 3-[3-(Morpholine-4-carbonyl)-4,5,6,7-tetrahydro-2H-isoindol-1-ylmethylene]-2-oxo-2,3-dihydro-1*H*-indole-5-carboxylic acid |
| 109 | 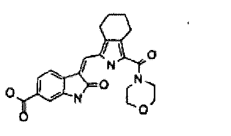 | 3-[3-(Morpholine-4-carbonyl)-4,5,6,7-tetrahydro-*2H*-isoindol-1-ylmethylene]-2-oxo-2,3-dihydro-1*H*-indole-6-carboxylic acid |

**[Table 3-14]**

| | | |
|---|---|---|
| 110 | 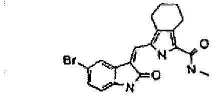 | 3-(5-Bromo-2-oxo-1,2-dihydro-indol-3-ylidenemethyl)-4,5,6,7-tetrahydro-2*H*-isoindole-1-carboxylic acid methylamide |
| 111 | 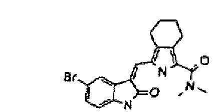 | 3-(S-Bromo-2-oxo-1,2-dihydro-indol-3-ylidenemethyl)-4,5,6,7-tetrahydro-*2H*-isoindole-1-carboxylic *acid dimethylamide* |
| 112 | 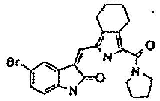 | 5-Bromo-3-[3-(pyrrolidine-1-carbonyl)-4,5,6,7-tetrahydro-2*H*-isoindol-1-ylmethylene]-1,3-dihydro-indol-2-one |
| 113 | 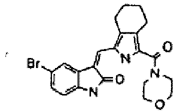 | 5-Bromo-3-[3-(morpholine-4-carbonyl)-4,5,6,7-tetrahydro-2*H*-isaindol-1-ylmethylene]-1,3-dihydro-indol-2-one |
| 114 | 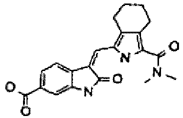 | *3-*(*3-Dimethylcarbamoyl-4,5,6,7-tetrahydro-2H-isoindol-1-ylmethylene*)*-2-*oxo-2,3-dihydro-1*H*-indole-6-carboxylic acid |
| 115 | 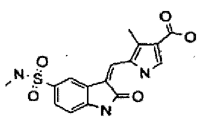 | 4-Methyl-5-(5-methylsulfamaoyl-2-oxo-1,2-dihydro-indal-3-ylidenemethyl)-1*H*-pyrrole-3-carboxylic acid |
| 116 | 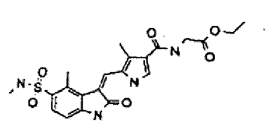 | {[4-Methyl-5-{4-methyl-5-methylsulfamoyl-2-oxo-1,2-dihydro-indol-3-ylidenemethyl)-1*H*-pyrrole-3-carbonyl]-amino}-acetic acid ethyl ester |
| 117 | 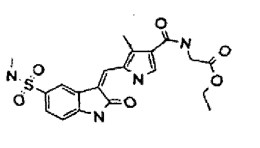 | {[4-Methyl-5-(5-methylsulfamoyl-2-oxo-1,2-dihydre-indol-3-ylidenemethyl)-1*H*-pyrrole-3-carbonyl]-amino}-acetic acid ethyl ester |

**[Table 3-15]**

| | | |
|---|---|---|
| 118 | 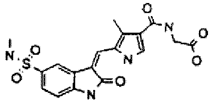 | {[4-Methyl-5-(5-melhylsulfamoyl-2-oxo-1,2-dihydro-indol-3-ylidenemethyl)-1*H*-pyrrole-3-carbonyl]-amino}-acetic acid |
| 119 | 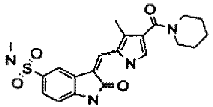 | 3-[3-Methyl4-(piperidine-1-carbonyl)-1*H*-pyrrol-2-ylmelhylene]-2-oxo-2,3-dihydro-1*H*-indole-5-sulfonic acid methylamide |
| 120 | 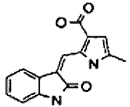 | 5-Methyl-2-(2-oxo-1,2-dihydro-indol-3-ylidenemethyl)-1H -pyrrole-3-carboxylic acid |
| 121 | 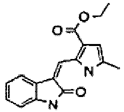 | 5-Methyl-2-(2-oxo-1,2-dihydro-indol-3-ylidenemethyl)-1*H*-pyrrole-3-carboxylic acid ethyl ester |
| 122 | 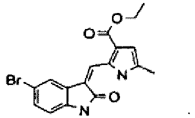 | 2-(5-Bromo-2-oxo-1,2-dihydro-indol-3-ylidenemethyl)-5-methyl-1*H* -pyrrole-3-carboxylic acid ethyl ester |
| 123 | 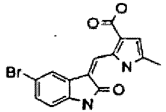 | 2-(5-Bromo-2-oxo-1,2-dihydro-indol-3-ylidenemethyl)-5-methyl-1*H*-pyrrole-3-carboxylic acid |
| 124 | 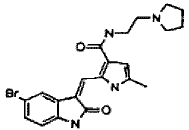 | 2-(5-Bromo-2-oxo-1,2-dihydro-indol-3-ylidenemethyl)-5-methyl-1H-pyrrole-3-carboxylic acid (2-pyrrolidin-1-yl-ethyl)-amide |
| 125 | 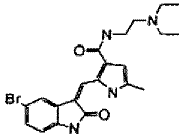 | 2-(5-Bromo-2-oxo-1,2-dihydro-indol-3-ylidenemethyl)-5-methyl-1H-pyrrole-3-carboxylic acid (2-diethylamino-ethyl)-amide |

**[Table 3-16]**

| | | | |
|---|---|---|---|
| 126 | 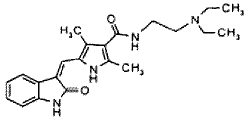 | 2,4-Dimethyl-5-(2-oxo-1,2-dihydro-indol-(3Z)-ylidenemethyl]-1*H*-pyrrole-3-carboxylic acid (2-diethylaminoethyl)-amide | 381 [M+1] |
| 127 | 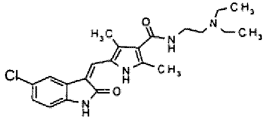 | 5-[5-Chloro-2-oxo-1,2-dihydroindol-(3Z)-ylidenemethyl]-2,4-dimethyl-1*H*-pyrrole-3-carboxylic acid (2-diethylaminoethyl)-amide | 415 [M+1] |
| 128 | 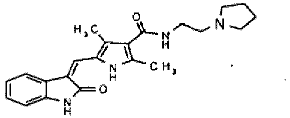 | 2,4-Dimethhyl-5-[2-oxo-1,2-dihydro-indol-(3Z)-ylidenemethyl]-2,4-dimethyl-1*H*-pyrrole-3-carboxylic acid (2-pyrrolidin-1-ylethyl)-amide | 379 [M+1] |
| 129 | 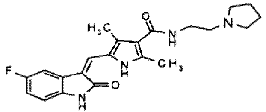 | 5-[5-Fluoro-2-oxo-1,2-dihydroindol-(3Z)-ylidenemethyl]-2,4-dimethyl-1*H*-pyrrole-3-carboxylic acid (2-pyrrolidin-1-ylethyl)-amide | 397 [M+1] |
| 130 | 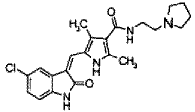 | 5-[5-Chloro-2-oxo-1,2-dihydroindol-(3Z)-ylidenemethyl]-1*H-*pyrrole-3-carboxylic acid (2-pyrrolidin-1-ylethyl)-amide | 413 [M+1] |
| 131 | 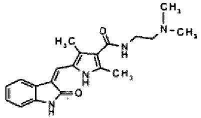 | 2,4-Dimethyl-6-[2-oxo-1,2-dihydro-indol-(3Z)-ylidenemethyl]-2,4-dimethyl-1*H*-pyrrole-3-carboxylic acid (2-dimethylaminoethyl)-amide | 353 [M+1] |
| 132 | 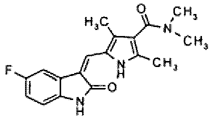 | 5-[5-Fluoro-2-oxo-1,2-dihydroindol-(3Z)-ylidenemethyl]-2,4-dimethyl-1*H*-pyrrole-3-carboxylic acid (2-dimethylaminoethyl)-amide | 371 [M+1] |

**[Table 3-17]**

| | | | |
|---|---|---|---|
| 133 | 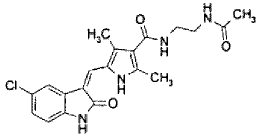 | 5-[5-Chloro-2-oxo-1,2-dihydro-indol-(3Z)-ylidenemethyl]-2,4-dimethyl-1*H*-pyrrole-3-carboxylic acid (2-acetylamino-ethyl)-amide | 399 [M-1] |
| 134 | 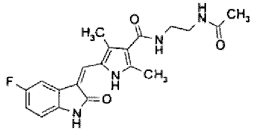 | 5-[5-Fluoro-2-oxo-1,2-dihydro-indol-(3Z)-ylidenemethyl]-2,4-dimethyl-1*H*-pyrrole-3-carboxylic acid (2-acetylamino-ethyl)-amide | 383 [M-1] |
| 135 | 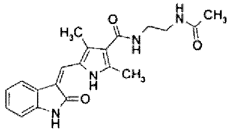 | 2,4-Dimethyl-5-[2-oxa-1,2-dihydro-indol-(3Z)-ylidenemethyl]-1*H*-pyrrole-3-carboxylic acid (2-acetylamino-ethyl)-amide | 365 [M-1] |
| 136 | 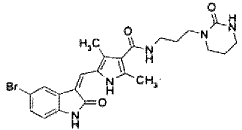 | 5-[5-Bromo-2-oxo-1,2-dihydro-indol-(3Z)-ylidenemethyl]-2,4-dimethyl-1*H*-pyrrole-3-carboxylic acid [3-(2-oxo-tetrahydropyrimidin-1-yl)-propyl]-amide | 500 [M+1] |
| | | | 502 [M+1] |
| 137 | 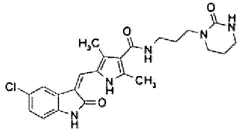 | 5-[5-Chloro-2-oxo-1,2-dihydro-indol-(3Z)-ylidenemethyl]-2,4-dimethyl-1*H*-pyrrole-3-carboxylic acid [3-(2-oxo-tetrahydropyrimidin-1-yl)-propyl]-amide | 454 [M-1] |
| 138 | 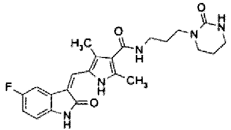 | 5-[5-Fluoro-2-oxo-1,2-dihydro-indol-(3Z)-ylidenemethyl]-2,4-dimethyl-1*H*-pyrrole-3-carboxylic acid [3-(2-oxo-tetrahydropyrimidin-1-yl)-propyl]-amide | 438 [M-1] |
| 139 | 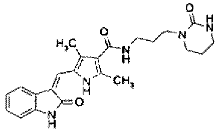 | 2,4-Dimethyl-5-[2-oxo-1,2-dihydro-indol-(3Z)-ylidenemethyl]-1*H*-pyrrole-3-carboxylic acid [3-(2-oxo-tetrahydropyrimidin-1-yl)-propyl]-amide | 422 [M+1] |
| 140 | 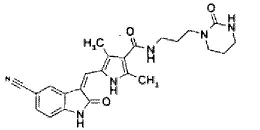 | 5-[5-Cyano-2-oxo-1,2-dihydro-indol-(3Z)-ylidenemethyl]-2,4-dimethyl-1*H*-pyrrole-3-carboxylic acid [3-(2-oxo-tetrahydropyrimidin-1-yl)-propyl]-amide | 447 [M+1] |
| 141 | 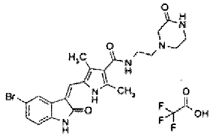 | Trifluoro-acetate4-[2-({5-[5-bromo-2-oxo-1,2-dihydro-indol-(3Z)-ylidenemethyl]-2,4 dimethyl-1*H*-pyrrole-3-carbonyl}-amino)-ethyl]-2-oxo-piperazin-1-ium; | 486 [M+1] |
| | | | 488 [M+1] |

**[Table 3-18]**

| | | | |
|---|---|---|---|
| 142 | 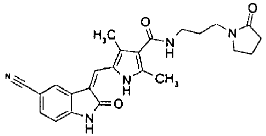 | 5-[5-Cyano-2-oxo-1,2-dihydro-indol-(3Z)-ylidenemethyl]-2,4-dimethyl-1*H* pyrrole-3-carboxylic acid [3-(2-oxopyrrolidin-1-yl)-propyl]-amide | 430 [M-1] |
| 143 | 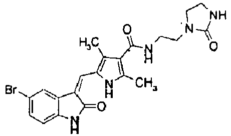 | 5-[5-Bromo-2-oxo-1,2-dihydro-indol-(3Z)-ylidenemethyl]-2,4-dimethyl-1*H* pyrrole-3-carboxylic acid [2-(2-oxo-imidazolidin-1-yl)-ethyl]-amide | 470 [M-1] |
| | | | 472 [M-1] |
| 144 | 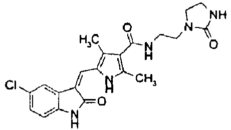 | 5-[5-Cyano-2-oxo-1,2-dihydro-indol-(3Z)-ylidenemethyl]-2,4-dimethyl-1*H* pyrrole-3-carboxylic acid [2-(2-oxo-imidazolidin-1-yl)-ethyl]-amide | 428 [M+1] |
| 145 | 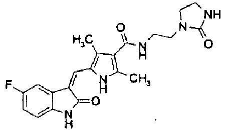 | 5-[5-Fluoro-2-oxo-1,2-dihydro-indol-(3Z)-ylidenemethyl]-2,4-dimethyl-1*H* pyrrole-3-carboxylic acid [2-(2-oxo-imidazolidin-1-yl)-ethyl]-amide | 412 [M+1] |
| 146 | 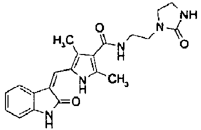 | 2,4-Dimethyl-5-[2-oxo-1,2-dihydroindol-(3Z)-ylidenemethyl]-1*H*-pyrrole 3-carboxylic acid [2-(2-oxo-imidazolidin-1-yl)-ethyl]-amide | 392 [M-1] |
| 147 | 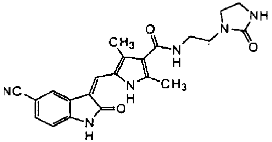 | 5-[5-Cyano-2-oxo-1,2-dihydro-indol-(3Z)-ylidenemethyl]-2,4-dimethyl-1*H* pyrrole-3-carboxylic acid [2-(2-oxo-imidazolidin-1-yl)-ethyl]-amide | 419 [M+1] |
| 148 | 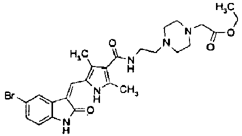 | {4-[2-({5-[5-Bromo-2-oxo-1,2-dihydro-indol-(3Z)-ylidenemethyl]-2,4-dimethyl-1*H*-pyrrole-3-carbonyl} amino)-ethyl]-piperazin-1-yl}-acetic acid ethyl ester | 558 [M+1] |
| | | | 560 [M+1] |
| 149 | 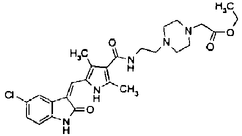 | {4-[2-({5-[5-Chloro-2-oxo-1,2-dihydro-indol-(3Z)-ylidenemethyl]-2,4-dimethyl-1*H*-pyrrole-3-carbonyl} amino)-ethyl]-piperazin-1-yl}-acetic acid ethyl ester | 514 [M+1] |
| 150 | 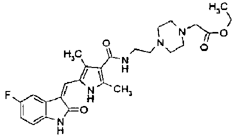 | {4-[2-({5-[5-Fluoro-2-oxo-1,2-dihydro indol-(3Z)-ylidenemethyl]-2,4-dimethyl-1*H*-pyrrole-3-carbonyl}-amino)-ethyl]-piperazin-1-yl}-acetic acid ethyl ester | 498 [M+1] |

**[Table 3-19]**

| | | | |
|---|---|---|---|
| 153 | 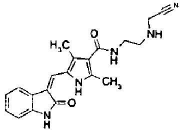 | 2,4-Dimethyl-5-[2-oxo-1,2-dihydro-indol-(3Z)-ylidenemethyl]-1*H*-pyrrole-3-carboxylic acid (2-(cyanomethyl-amino)-ethyl]-amide | 362 [M-1] |
| 154 | 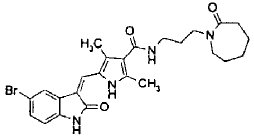 | 5-[5-Bromo-2-oxo-1,2-dihydro-indol-(3Z)-ylidenemethyl]-2,4-dimethyl-1*H*-pyrrole-3 carboxylic acid [3-(2-oxo-azepan-1-yl)-propyl]-amide | 511 [M-1] |
| | | | 513 [M-1] |
| 155 | 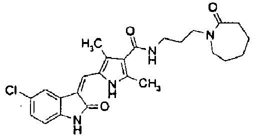 | 5-[5-Chloro-2-oxo-1,2-dihydro-indol-(3Z)-ylidenemethyl]-2,4-dimethyl-1*H*-pyrrole-3 carboxylic acid [3-(2-oxo-azepan-1-yl)-propyl]-amide | 469 [M+1] |
| 156 | 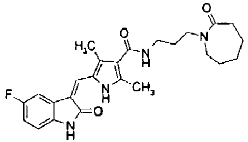 | 5-[5-Fluoro-2-oxo-1,2-dihydro-indol-(3Z)-ylidenemethyl]-2,4-dimethyl-1*H*-pyrrole-3 carboxylic acid [3-(2-oxo-azepan-1-yl)-propyl]-amide | 453 [M+1] |
| 157 | 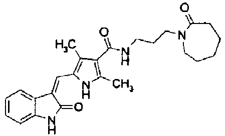 | 2,4-Dimethyl-5-[2-oxo-1,2-dihydro-indol-(3Z)-ylidenemethyl]-1*H*-pyrrole-3-carboxylic acid [3-(2-oxo-azepan-1-yl)-propyl]-amide | 435 [M+1] |
| 158 | 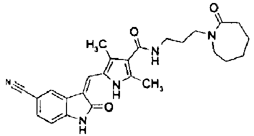 | 5-[5-Cyano-2-oxo-1,2-dihydro-indol-(3Z)-ylidenemethyl]-2,4-dimethyl-1*H*-pyrrole-3 carboxylic acid [3-(2-oxo-azepan-1-yl)-propyl]-amide | 460 [M+1] |
| 159 | 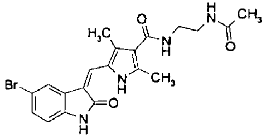 | 5-[5-Bromo-2-oxo-1,2-dihydro-indol-(3Z)-ylidenemethyl]-2,4-dimethyl-1*H*-pyrrole-3 carboxylic acid (2-acetylamino-ethyl)-amide | 443 [M-1] |
| | | | 445 [M-1] |

**[Table 3-20]**

| | | | |
|---|---|---|---|
| 160 | 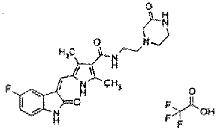 | Trifluoro-acetate4-[2-((5-[5-fluoro-2-oxo-1,2-dihydro-indol-(3Z)-ylidenemethyl]-2,4-dimethyl-1*H-*pyrrole-3-carbonyl)-amino)-ethyl]-2-oxo-piperazin-1-ium; | 426 [M+1] |
| 161 | 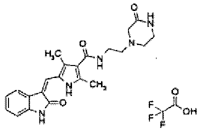 | Trifluoro-acetate4-[2-({2,4-dimethy-5-[2-oxo-1,2-dihydro-indol-(3Z)-ylidenemethyl]-1*H*-pyrrole-3-carbonyl}-amino)-ethyl]-2-oxo-piperazin-1-ium; | 408 [M+1] |
| 162 | 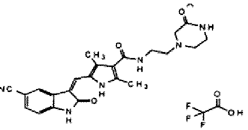 | Trifluoro-acetate4-[2-({5-[5-cyano-2-oxo-1,2-dihydro-indol-(3Z)-ylidenemethyl]-2,4-dimethyl-1*H-*pyrrole-3-carbonyl}-amino)-ethyl]-2-oxo-piperazin-1-ium; | 433 [M+1] |
| 163 | 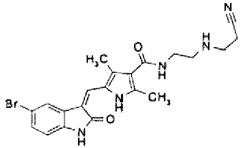 | 5-[5-Bromo-2-oxo-1,2-dihydro-indol-(3Z)-ylidenemethyl]-2,4-dimethyl-1*H-*pyrrole-3-carboxylic acid [2-(2-cyanoethylamino)-ethyl]-amide | 454 [M-1] |
| | | | 456 [M-1] |
| 164 | 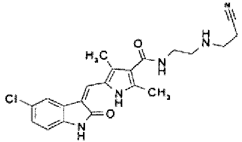 | 5-[5-Chloro-2-oxo-1,2-dihydro-indol-(3Z)-ylidenemethyl]-2,4-dimethyl-1*H-*pyrrole-3-carboxylic acid [2-(2-cyano-ethylamino)-ethyl]-amide | 410 [M-1] |
| 165 | 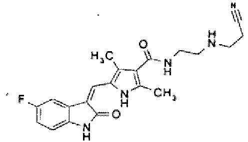 | 5-[5-Fluoro-2-oxo-1,2-dihydro-indol-(3Z)-ylidenemethyl]-2,4-dimethyl-1*H-*pyrrole-3-carboxylic acid [2-(2-cyano-ethylamino)-ethyl)-amide | 394 [M-1] |
| 166 | 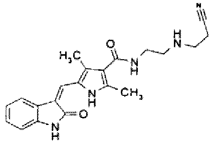 | 2,4-Dimethyl-5-[2-oxo-1,2-dihydroindol-(3Z)-ylidenemethyl]-1*H*-pyrrole-3 carboxylic acid [2-(2-cyano-ethylamino)-ethyl]-amide | 376 [M-1] |
| 167 | 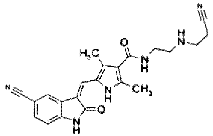 | 5-[5-Cyano-2-oxo-1,2-dihydro-indol-(3Z)-ylidenemethyf]-2,4-dimethyl-1*H-*pyrrole-3-carboxylic acid [2-(2-cyanoethylamino)-ethyl]-amide | 401 [M-1] |
| 168 | 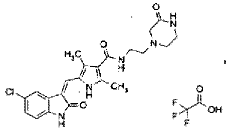 | Trifluoro-acetate4-[2-({5-[5-chloro-2-oxo-1,2-dihydro-indol-(3Z)-ylidenemethyl]-2,4-dimethyl-1*H-*pyrrole-3-carbonyl}-amino)-ethyl]-2-oxo-piperazin-1-ium; | 440 [M-1] |

**[Table 3-21]**

| | | | |
|---|---|---|---|
| 168 | 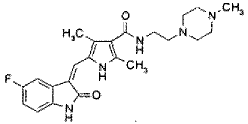 | 5-[5-Fluoro-2-oxo-1,2-dihydro-indol-(3Z)-ylidenemethyl]-2,4-dimethyl-1*H*-pyrrole-3-carboxylic acid [2-(4-methyl-piperazin-1-yl)-ethyl]-amide | 424 [M-1] |
| 169 | 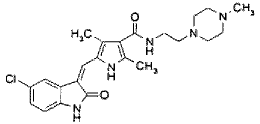 | 5-[5-Chloro-2-oxo-1,2-dihydro-indol-(3Z)-ylidenemethyl]-2,4-dimethyl-1H-pyrrole-3-carboxylic acid [2-(4-methyl-piperazin-1-yl)-ethyl]-amide | 440 [M-1] |
| 170 | 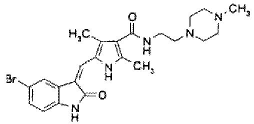 | 5-[5-Bromo-2-oxo-1,2-dihydro-indol-(3Z)-ylidenemethyl]-2,4-dimethyl-1*H*-pyrrole-3-carboxylic acid [2-(4-methyl-piperazin-1-yl)-ethyl]-amide | 484 [M-1] |
| | | | 486 [M-1] |
| 171 | 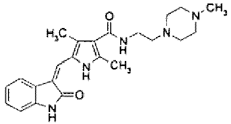 | 2,4-Dimethyl-5-[2-oxo-1,2-dihydro-indol-(3Z) ylidenemethyl]-1*H*-pyrrole-3-carboxylic acid [2-(4-methyl-piperazin-1-yl)-ethyl]-amide | 406 [M-1] |
| 172 | 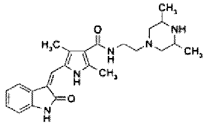 | 2,4-Dimethyl-5-[2-oxo-1,2-dihydro-indol-(3Z) ylidenemethyl]-1*H*-pyrrole-3-carboxylic acid [2-(3,5-dimethyl-piperazin-1-yl)-ethyl]-amide | 422 [M+1] |
| 173 | 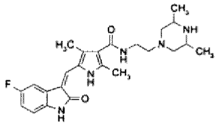 | 5-[5-Fluoro-2-oxo-1,2-dihydro-indol-(3Z) ylidenemethyl]-2,4-dimethyl-1*H*-pyrrole-3-carboxylic acid [2-(3,5-dimethyl-piperazin-1 yl)-ethyl]-amide | 438 [M-1] |
| 174 | 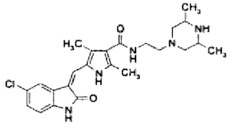 | 5-[5-Chloro-2-oxo-1,2-dihydro-indol-(3Z)-ylidenemethyl]-2,4-dimethyl-1*H*-pyrrole-3-carboxylic acid [2-(3,5-dimethyl-piperazin-1 yl)-ethyl]-amide | 456 [M+1] |
| 175 | 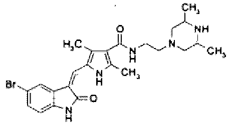 | 5-[5-Bromo-2-oxo-1,2-dihydro-indol-(3Z)-ylidenemethyl]-2,4-dimethyl-1*H*-pyrrole-3-carboxylic acid [2-(3,5-dimethyl-piperazin-1 yl)-ethyl]-amide | 498 [M-1] |
| | | | 500 [M-1] |

**[Table 3-22]**

| | | | |
|---|---|---|---|
| 176 | 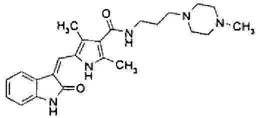 | 2,4-Dimethyl-6-[2-oxo-1,2-dihydro-indol-(3Z)-ylidenemethyl]-1*H*-pyrrole-3-carboxylic acid [3-(4-methyl-piperazin-1-yl)-propyl]-amide | 422 [M+1] |
| 177 | 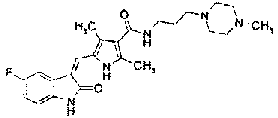 | 5-[5-Fluoro-2-oxo-1,2-dihydro-indol-(3Z)-ylidenemethyl]-2,4-dimethyl-1*H*-pyrrole-3-carboxylic acid [3-(4-methyl-piperazin-1-yl)-propyl]-amide | 438 [M-1] |
| 178 | 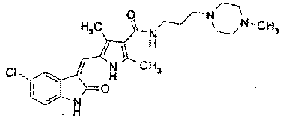 | 5-[5-Chloro-2-oxo-1,2-dihydroindol-(3Z)-ylidenemethyl]-2,4-dimethyl-1*H*-pyrrole-3-carboxylic acid [3-(4-methyl-piperazin-1-yl)-propyl]-amide | 454 [M-1] |
| 179 | 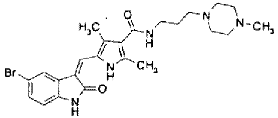 | 5-[5-Bromo-2-oxo-1,2-dihydroindol-(3Z)-ylidenemethyl]-2,4-dimethyl-1*H*-pyrrole-3-carboxylic acid [3-(4-methyl-piperazin-1-yl)-propyl]-amide | 498 [M-1] |
| | | | 500 [M-1] |
| 180 | 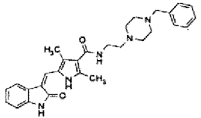 | 2,4-Dimethyl-5-[2-oxo-1,2-dihydro-indol-(3Z)-ylidenemethyl]-1*H*-pyrrole-3-carboxylic acid [2-(4-benzyl-piperazin-1-yl)-ethyl]-amide | 482 [M-1] |
| 181 | 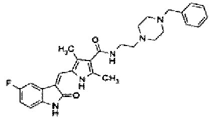 | 5-[5-Fluoro-2-oxo-1,2-dthydro-indol-(3Z)-ylidenemethyl]-2,4-dimethyl-1*H*-pyrrole-3-carboxylic acid [2-(4-benzyl-piperazin-1-yl)-ethyl]-amide | 500 [M-1] |
| 182 | 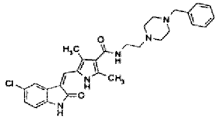 | 5-[5-Chloro-2-oxo-1,2-dihydro-indol-(3Z)-ylidenemethyl]-2,4-dimethyl-1*H*-pyrrole-3-carboxylic acid [2-(4-benzyl-piperazin-1-yl)-ethyl]-amide | 517 [M-1] |
| 183 | 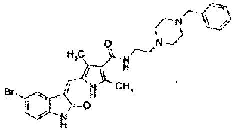 | 5-[5-Bromo-2-oxo-1,2-dihydro-indol-(3Z)-ylidenemethyl]-2,4-dimethyl-1*H*-pyrrole-3-carboxylic acid [2-(4-benzyl-piperazin-1-yl)-ethyl]-amide | 560 [M-1] |
| | | | 562 [M-1] |

**[Table 3-23]**

| | | | |
|---|---|---|---|
| 184 | 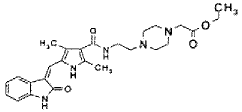 | 5-[5-Chloro-2-oxo-1,2-dihydro-indol-(3Z)-ylidenemethyl]-2,4-dimethyl-1*H*-pyrrole-3-carboxylic acid (3-pyrrolidin-1yl-2-one)-amide | 480 [M+1] |
| 185 | 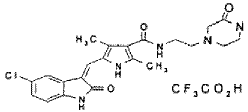 | Trifluoroacetate 4-[2-({5-[5-Chloro-2-oxo-1,2-dihydro-indol-(3Z)-ylidenemethyl]-2,4-dimethyl-1H-pyrrole-3-carbonyl}amino)-ethyl] 2-oxo-piperazin-1-ium | 440 [M-1] |
| 186 | 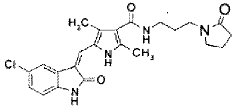 | 5-[5-Chloro-2-oxo-1,2-dihydro-indol-(3Z)-ylidenemethyl]-2,4-dimethyl-1*H*-pyrrole-3-carboxylic acid (3-pyrrolidin-1yl-2-one)-amide | |
| 187 | 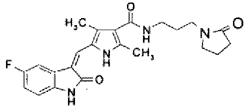 | 5-[5-Fluoro-2-oxo-1,2-dihydro-indol-(3Z)-ylidenemethyl]-2,4-dimethyl-1H-pyrrole-3-carboxylic acid (3-pyrrolidin-1yl-2-one)-amide | |
| 188 | 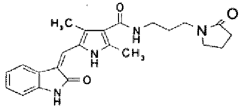 | 5-[2-oxo-1,2-dihydro-indol-(3Z)-ylidenemethyl]-2,4-dimethyl-1H-pyrrole-3-carboxylic acid (3-pyrrolidin-1 yl-2-one)-amide | |
| 189 | 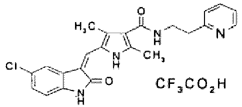 | 5-[5-Chloro-2-oxo-1,2-dihydro-indol-(3Z)-ylidenemethyl]-2,4-dimethyl-1*H*-pyrrole-3-carboxylic acid (2-pyridin-2-ylethyl)-amide | |
| 190 | 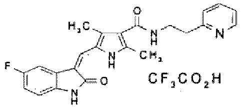 | 5-[5-Fluoro-2-oxo-1,2-dihydro-indol-(3Z)-ylidenemethyl]-2,4-dimethyl-1H-pyrrole-3-carboxylic acid (2-pyridin-2-ylethyl)-amide trifluroracetate salt | |
| 191 | 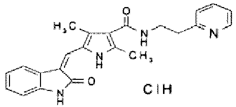 | 5-[2-oxo-1,2-dihydro-indol-(3Z)-ylidenemethyl]-2,4-dimethyl-1H-pyrrole-3-carboxylic acid (2-pyridin-2-ylethyl)-amide hydrochloride salt | |
| 192 | 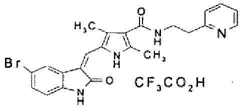 | 5-[5-Bromo-2-oxo-1,2-dihydro-indol-(3Z)-ylidenemethyl]-2,4-dimethyl-1H-pyrrole-3-carboxylic acid (2-pyridin-2-ylethyl)-amide trifluroracetate salt | |

**[Table 3-24]**

| | | |
|---|---|---|
| 193 | 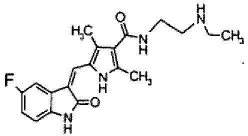 | 5-[5-Fluoro-2-oxo-1,2-dihydro-indol-(3Z)-ylidenemethyl]-2,4-dimethyl-1*H*-pyrrole-3-carboxylic acid (2-ethylaminoethyl)-amide |
| 194 | 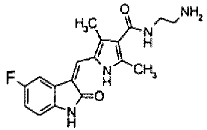 | 5-[5-Fluoro-2-oxo-1,2-dihydro-indol-(3Z)-ylidenemethyl]-2,4-dimethyl-1*H*-pyrrole-3-carboxylic acid (2-aminoethyl)-amide |
| 195 | 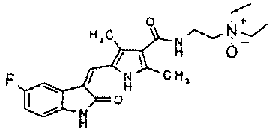 | 5-[5-Fluoro-2-oxo-1,2-dihydro-indol-(3Z)-ylidenemethyl]-2,4-dimethyl-2,4-dimethyl-1*H*-pyrrole-3-carboxylic acid (2-diethyl-N-oxoaminoethyl)-amide |
| 196 | 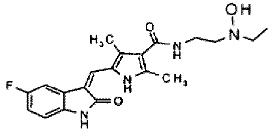 | 5-[5-Fluoro-2-oxo-1,2-dihydro-indol-(3Z)-ylidenemethyl]-2,4-dimethyl-1*H*-pyrrole-3-carboxylic acid (2-ethyl-N-hydroxy-aminoethyl)-amide |
| 197 | 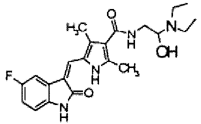 | 5-[5-Fluoro-2-oxo-1,2-dihydro-indol-(3Z)-ylidenerrethyl]-1*H*-pyrrole-3-carboxylic acid (2-diethylamino-2-hydroxyethyl)-amide |
| 198 | 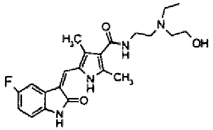 | 5-[5-Fluoro-2-oxo-1,2-dihydro-indol-(3Z)-ylidenemethyl]-2,4-dimethyl-1*H*-pyrrole-3-carboxylic acid [2-ethyl-2-(2-hydroxyethyl)aminoethyl]-amide |
| 199 | 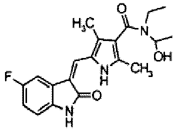 | 5-[5-Fluoro-2-oxo-1,2-dihydro-indol-(3Z)-ylidenemethyl]-2,4-dimethyl-1*H*-pyrrole-3-carboxylic acid [2-ethyl-2-(1-hydroxyethyl)aminoethyl]-amide |
| 200 | 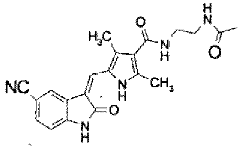 | 5-[5-Cyano-2-oxo-1,2-dihydro-indol-(3Z)-ylidenemethyl]-2,4-dimethyl-1*H*-pyrrole-3-carboxylic acid (2-N-acetylaminoethyl)-amide |
| 201 | 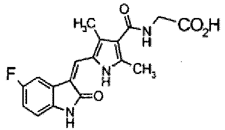 | 5-[5-Fluoro-2-oxo-1,2-dihydro-indol-(3Z)-ylidenemethyl]-2,4-dimethyl-1*H*-pyrrole-3-carboxylic acid (carboxymethyl)-amide |

**[Table 3-25]**

| | | |
|---|---|---|
| 202 | 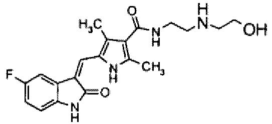 | 5-[5-Fluoro-2-oxo-1,2-dihydro-indol-(3Z)-ylidenemethyl]-1*H*-pyrrole-3-carboxylic acid [2-(2-hydroxethylamino)ethyl]-amide |
| 203 | 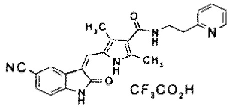 | 5-[5-Cyano-2-oxo-1,2-dihydro-indol-(3Z)-ylidenemethyl]-1*H*-pyrrole-3-carboxylic acid (2-pyridin-2-ylethyl)-amide trifluoroacetate |
| 204 | 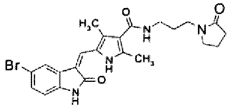 | 5-[5-Bromo-2-oxo-1,2-dihydro-indol-(3Z)-ylidenemethyl]-1*H*-pyrrole-3-carboxylic acid (3-pyrrolidin-1-yl-2-onepropyl)-amide trifluoroacetate |

Specific examples of the "analog of Axitinib" include a compound represented by the following formula (VIII): 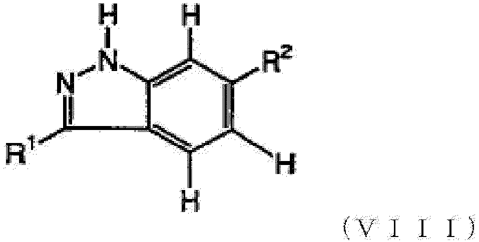 wherein
R¹ is a substituted or unsubstituted aryl group or heteroaryl group, or a group of the formula CH=CH-R³ or CH=N-R³ wherein R³ is a substituted or unsubstituted alkyl group, alkenyl group, cycloalkyl group, heterocycloalkyl group, aryl group or heteroaryl group;
R² is a substituted or unsubstituted aryl group or heteroaryl group, or a Y-X group wherein Y is O, S, C=CH₂, C=O, S=O, SO₂, an alkylidene group, NH or N-(C₁-C₈ alkyl) group, X is substituted or unsubstituted Ar, heteroaryl group, NH-(alkyl) group, NH-(cycloalkyl) group, NH-(heterocycloalkyl) group, NH(aryl) group, NH(heteroaryl group), NH-(alkoxyl) group or NH-(dialkylamide) group, Ar is substituted or unsubstituted aryl.

In the above-mentioned formula (VIII), when R² is a substituted aryl group, R² is preferably a group represented by the following formula: 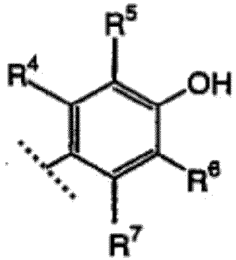 wherein
R⁴ and R⁷ are each independently hydrogen, OH, a halo group, a C₁-C₈ alkyl group, a C₁-C₈ alkoxyl group, a C₁-C₈ alkenyl group, an aryloxy group, a thioaryl group, CH₂-OH, a CH₂-O-(C₁-C₈ alkyl) group, CH₂-O-aryl, a CH₂-S-(C₁-C₈ alkyl) group, a CH₂-S-aryl group;
R⁵ and R⁶ are each independently hydrogen, OH, a halo group, a Z-alkyl group, a Z-aryl group, or Z-CH₂CH=CH₂ wherein Z is O, S, NH or CH₂, and alkyl group and aryl group of Z-alkyl group and Z-aryl group are each optionally substituted. In this case, more preferably, in the above-mentioned formula (V),
R¹ is substituted or unsubstituted bicyclic heteroaryl, or a group of the formula CH=CH-R³ wherein R³ is a substituted or unsubstituted aryl group or heteroaryl group;
R⁴ and R⁷ are each independently hydrogen or a C₁-C₈ alkyl group;
R⁵ and R⁶ are each independently a halo group, a Z-alkyl group or Z-CH₂CH=CH₂ wherein Z is O or S.

In another preferable embodiment, in the above-mentioned formula (VIII), R² is Y-Ar (Y, Ar are as defined above), more preferably, Ar is a group represented by the following formula: 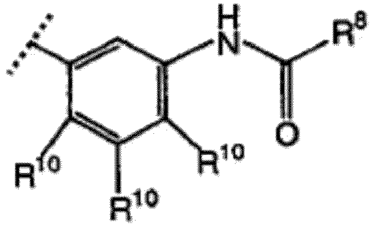 wherein
R⁸ is a substituted or unsubstituted alkyl group, alkenyl group, cycloalkyl group, heterocycloalkyl group, aryl group, heteroaryl group, alkoxyl group or aryloxy group;
R¹⁰ is independently selected from hydrogen, halogen and a lower alkyl group. In this case, more preferably, in the above-mentioned formula (V),
R¹ is a substituted or unsubstituted bicyclic heteroaryl group, or a group of the formula CH=CH-R³ wherein R³ is a substituted or unsubstituted aryl group or heteroaryl group; Y is O, S, C=CH₂, C=O, NH or N- (C₁-C₈ alkyl) group;
R⁸ is a substituted or unsubstituted aryl group, heteroaryl group, alkyl group, alkenyl group, more preferably, a substituted or unsubstituted aryl group, heteroaryl group, and each R¹⁰ is independently hydrogen or halogen, more preferably, all hydrogen.

In the above-mentioned formula (VIII), in another preferable embodiment wherein R² is Y-Ar (Y, Ar are as defined above), Ar is a group represented by the following formula: 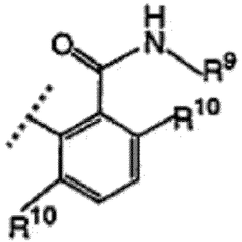 wherein
R⁹ is a substituted or unsubstituted alkyl group, cycloalkyl group, heterocycloalkyl group, aryl group, heteroaryl group, alkoxyl group, aryloxy group, cycloalkoxyl group, NH-(C₁-C₈ alkyl) group, NH-(aryl) group, NH-(heteroaryl) group, N=CH-(alkyl) group, NH(C=O)R¹¹ group or NH₂ wherein R¹¹ is independently selected from hydrogen, a substituted or unsubstituted alkyl group, cycloalkyl group, heterocycloalkyl group, aryl group and heteroaryl group);
R¹⁰ is independently selected from hydrogen, halogen and a lower alkyl group. In this case, more preferably, in the above-mentioned formula (V),
R¹ is a group of the formula CH=CH-R³ wherein R³ is a substituted or unsubstituted aryl group or heteroaryl group);
Y is S or NH;
R⁹ is a substituted or unsubstituted alkyl group, alkoxyl group or NH-(heteroaryl) group.

Each term described as higher concept in the explanation of each group in the above-mentioned formula (VIII) (e.g., "substituted or unsubstituted aryl group", "substituted or unsubstituted heteroaryl group", "substituted or unsubstituted alkyl group", "substituted or unsubstituted alkenyl group", "substituted or unsubstituted cycloalkyl group", "substituted or unsubstituted heterocycloalkyl group", "substituted or unsubstituted aryl group", "substituted or unsubstituted heteroaryl group" etc. for R¹) is as defined in WO 01/002369 (National Publication of International Patent Application No. 2003-503481).

Specific examples of the analog of Axitinib include the compounds represented by the following structural formulas.

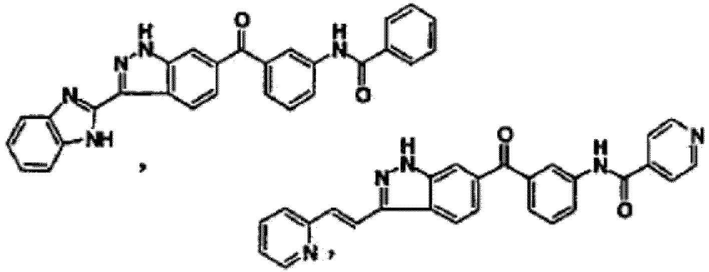

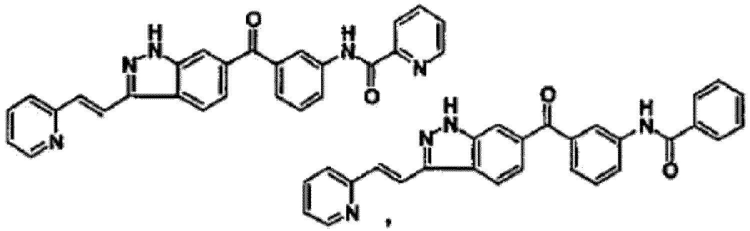

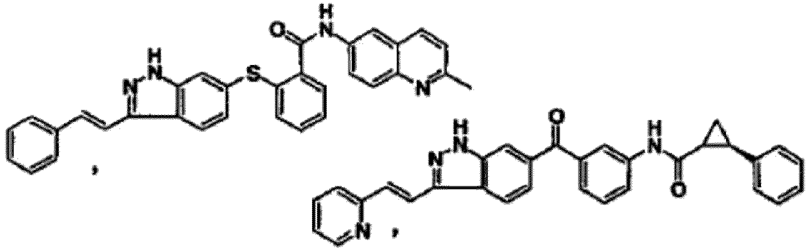

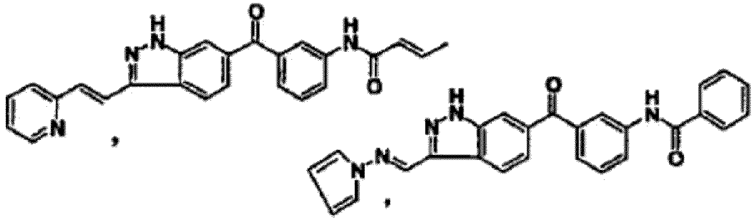

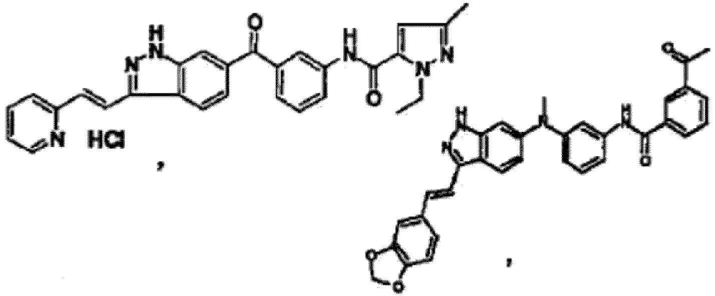

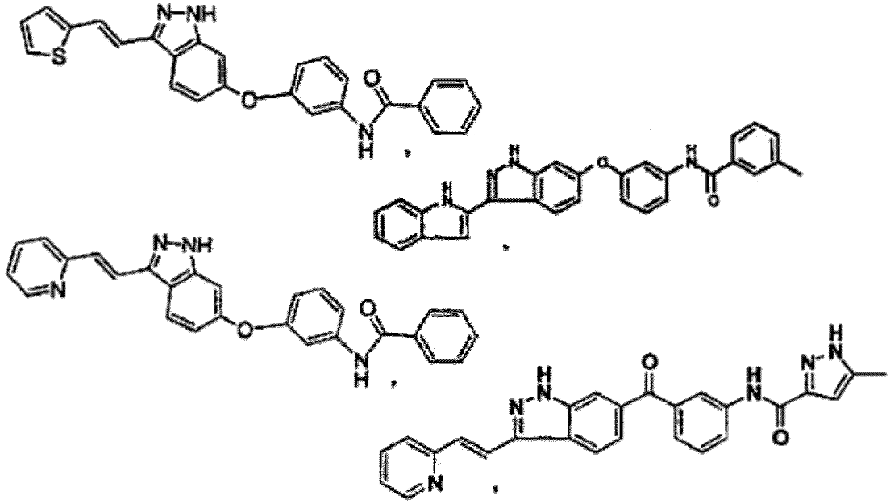

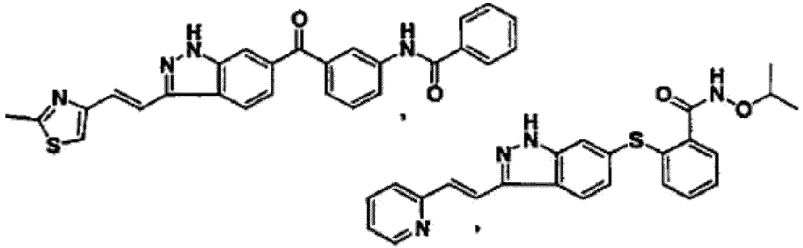

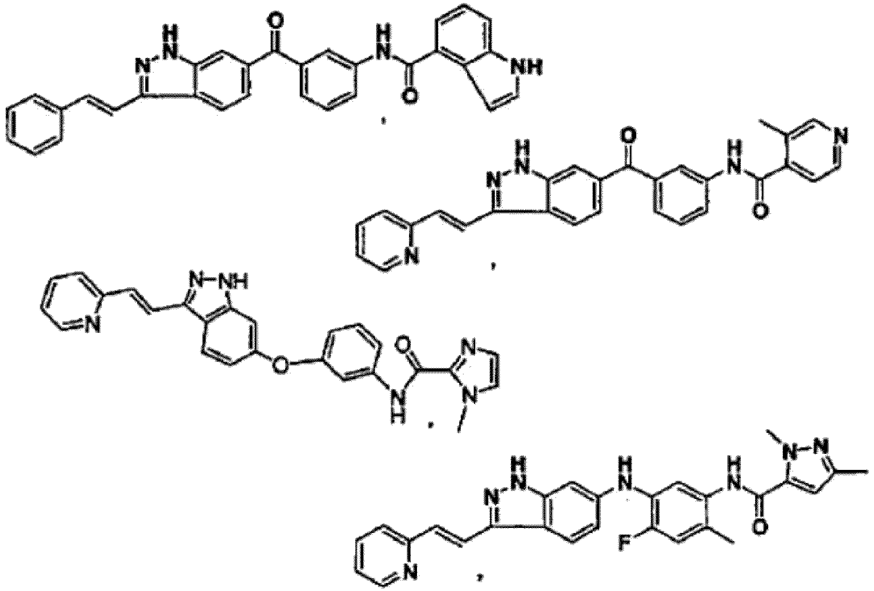

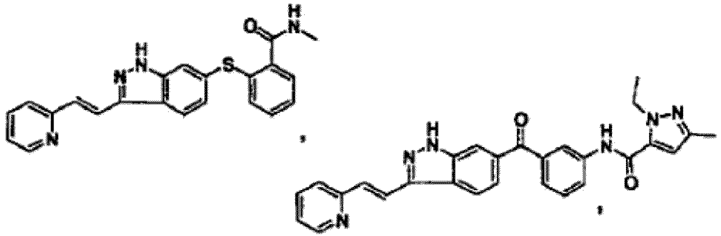

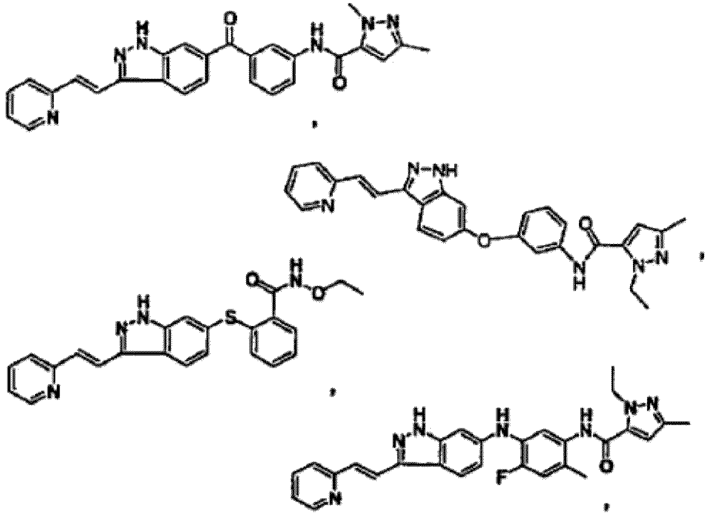

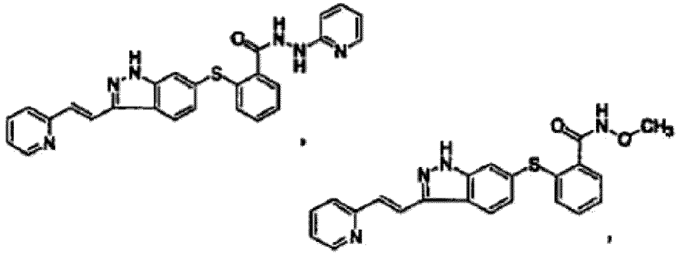

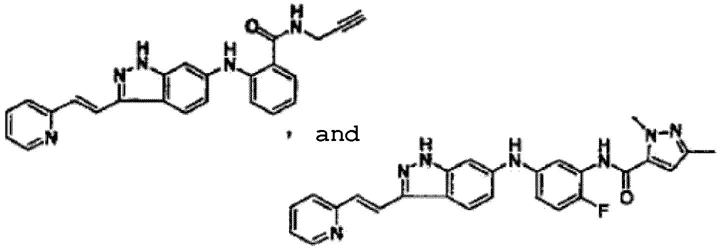

Examples of the analog of the above-mentioned "Bosutinib (SKI-606)" include a compound represented by the following formula (IX): 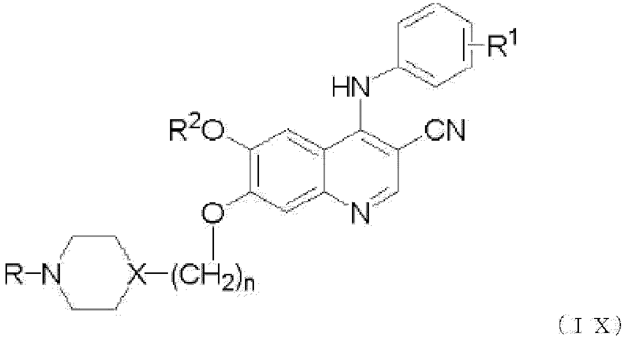 wherein
n is an integer of 1-3;
X is N, CH, provided when X is N, then n is 2 or 3;
R is alkyl having 1 to 3 carbon atoms;
R¹ is 2,4-dichloro and 5-methoxy, 2,4-dichloro, 3,4,5-trimethoxy, 2-chloro and 5-methoxy, 2-methyl and 5-methoxy, 2,4-dimethyl, 2,4-dimethyl and 5-methoxy, or 2,4-dichloro and 5-ethoxy;
R² is alkyl having 1 or 2 carbon atoms.

Each term described as higher concept in the explanation of each group in the above-mentioned formula (IX) ("alkyl having 1 to 3 carbon atoms" for R, "alkyl having 1 or 2 carbon atoms" for R²) is as defined in WO 2005/046693 (National Publication of International Patent Application No. 2007-533655).

Specific examples of the analog of "Bosutinib (SKI-606)" include the following compounds.
4-[(2,4-dichloro-5-methoxyphenyl)amino]-7-[3-(4-ethyl-1-piperazinyl)propoxy]-6-methoxy-3-quinolinecarbonitrile;
4-[(2,4-dichloro-5-methoxyphenyl)amino]-6-methoxy-7-[2-(4-methyl-1-piperazinyl)ethoxy]-3-quinolinecarbonitrile;
4-[(2,4-dichloro-5-methoxyphenyl)amino]-7-[2-(4-ethyl-1-piperazinyl)ethoxy]-6-methoxy-3-quinolinecarbonitrile;
4-[(2,4-dichloro-5-methoxyphenyl)amino]-6-methoxy-7-[(1-methylpiperidin-4-yl)methoxy]-3-quinolinecarbonitrile;
4-[(2,4-dichloro-5-methoxyphenyl)amino]-6-methoxy-7-[2-(1-methylpiperidin-4-yl)ethoxy]-3-quinolinecarbonitrile;
4-[(2,4-dichloro-5-methoxyphenyl)amino]-6-methoxy-7-[3-(1-methylpiperidin-4-yl)propoxy]quinoline-3-carbonitrile;
4-[(2,4-dichloro-5-methoxyphenyl)amino]-7-[(1-ethylpiperidin-4-yl)methoxy]-6-methoxyquinoline-3-carbonitrile;
4-[(2,4-dichloro-5-methoxyphenyl)amino]-6-ethoxy-7-[3-(4-methylpiperazin-1-yl)propoxy]quinoline-3-carbonitrile;
4-[(2,4-dichloro-5-methoxyphenyl)amino]-6-ethoxy-7-[(1-methylpiperidin-4-yl)methoxy]quinoline-3-carbonitrile;
4-[(2,4-dichloro-5-methoxyphenyl)amino]-6-ethoxy-7-[3-(4-ethylpiperazin-1-yl)propoxy]quinoline-3-carbonitrile;
4-[(2,4-dichloro-5-methoxyphenyl)amino]-6-ethoxy-7-[3-(1-methylpiperidin-4-yl)propoxy]quinoline-3-carbonitrile;
4-[(2,4-dichloro-5-methoxyphenyl)amino]-6-ethoxy-7-[2-(4-methyl-1-piperazinyl)ethoxy]quinoline-3-carbonitrile;
4-[(2,4-dichloro-5-methoxyphenyl)amino]-6-ethoxy-7-[2-(1-methylpiperidin-4-yl)ethoxy]quinoline-3-carbonitrile;
4-[(2,4-dichloro-5-methoxyphenyl)amino]-6-methoxy-7-[3-(4-propyl-1-piperazinyl)propoxy]-3-quinolinecarbonitrile;
4-[(2,4-dichlorophenyl)amino]-6-methoxy-7-[(1-methylpiperidin-4-yl)methoxy]-3-quinolinecarbonitrile;
6-methoxy-7-[(1-methylpiperidin-4-yl)methoxy]-4-[(3,4,5-trimethoxyphenyl)amino]quinoline-3-carbonitrile;
4-[(2-chloro-5-methoxyphenyl)amino]-6-methoxy-7-[(1-methylpiperidin-4-yl)methoxy]quinoline-3-carbonitrile;
6-methoxy-4-[(5-methoxy-2-methylphenyl)amino]-7-[(1-methylpiperidin-4-yl)methoxy]quinoline-3-carbonitrile;
4-[(2,4-dimethylphenyl)amino]-6-methoxy-7-[(1-methylpiperidin-4-yl)methoxy]quinoline-3-carbonitrile;
6-methoxy-4-[(5-methoxy-2,4-dimethylphenyl)amino]-7-[(1-methylpiperidin-4-yl)methoxy]quinoline-3-carbonitrile;
4-[(2,4-dichloro-5-ethoxyphenyl)amino]-6-methoxy-7-[(1-methylpiperidin-4-yl)methoxy]quinoline-3-carbonitrile

In one embodiment, EGFR inhibitor, FGFR inhibitor, Aurorakinase inhibitor, PKA inhibitor, PKC inhibitor, MEK inhibitor, Met inhibitor, JNK inhibitor, Syk inhibitor and JAK inhibitor, prostaglandin analogue, estrogen receptor antagonist, and the above-mentioned compounds which do not belong to these medicaments (hereinafter sometimes to be referred comprehensively to as "the compound of the present invention") as the active ingredient of the prophylactic or therapeutic agent for ALS of the present invention do not belong to platelet-derived growth factor receptor (PDGFR) inhibitor, vascular endothelial cell growth factor receptor (VEGFR) inhibitor, c-Kit inhibitor, Flt3 inhibitor, Cdk1 inhibitor, Cdk2 inhibitor, GSK-3β inhibitor and Src inhibitor.

As the compound of the present invention, a commercially available product may be used, or each compound can be produced by each method known per se.

For example, Pazopanib or an analog thereof can be produced according to the method described in, for example, WO 2002/059110 (National Publication of International Patent Application No. 2004-517925).

PF-2341066 (Crizotinib) or an analog thereof can be produced according to the method described in, for example, WO 2004/076412 (National Publication of International Patent Application No. 2006-519232).

Bimatoprost or an analog thereof can be produced according to the method described in, for example, US Patent No. 5,607,978.

Raloxifene or an analog thereof can be produced according to the method described in, for example, JP-A-52-53851.

Tivozanib or an analog thereof can be produced according to the method described in, for example, WO 02/088110.

Sunitinib or an analog thereof can be produced according to the method described in, for example, WO 01/060814 (National Publication of International Patent Application No. 2003-523340).

Axitinib or an analog thereof can be produced according to the method described in, for example, WO 01/002369 (National Publication of International Patent Application No. 2003-503481).

Bosutinib (SKI-606) or an analog thereof can be produced according to the methods described in, for example, US Patent Nos. 6,002,008 and 6,780,996, or Boschelli, D.H. et al., J. Med. Chem., 44, 3965 (2001), Boschelli, D.H. et al., J Med. Chem., 44, 822 (2001), Boschelli, D.H. et al., Bioorg. Med. Chem. Lett., 13, 3797 (2003), Boschelli, D.H. et al., J. Med. Chem., 47, 1599 (2004), and Ye, F. et al., 221th National Meeting of the American Chemical Society, San diego, California (April, 2001).

The compound of the present invention encompasses not only a free form but also a pharmacologically acceptable salt thereof. While the pharmacologically acceptable salt varies depending on the kind of the compound, examples thereof include base addition salts such as salts with inorganic base such as alkali metal salts (sodium salt, potassium salt etc.), alkaline earth metal salts (calcium salt, magnesium salt etc.), aluminum salt, ammonium salt and the like, and salts with organic base such as trimethylamine, triethylamine, pyridine, picoline, ethanolamine, diethanolamine, triethanolamine, dicyclohexylamine, N,N'-dibenzylethylenediamine and the like and the like, and acid addition salts such as salts with inorganic acid such as hydrochloride, hydrobromide, sulfate, hydroiodide, nitrate salt, phosphate and the like, and salts with organic acid such as citrate, oxalate, acetate, formate, propionate, benzoate, trifluoroacetate, maleate, tartrate, methanesulfonate, benzenesulfonate, paratoluenesulfonate and the like, and the like.

When the compound of the present invention contains isomers such as an optical isomer, a stereoisomer, a regioisomer or a rotamer, any one of the isomers and mixtures are also encompassed in the compound of the present invention. For example, when the compound of the present invention contains an optical isomer, an optical isomer resolved from racemate is also encompassed in the compound of the present invention. For example, while 3-hydroxybutyric acid contains an optical isomer, D-3-hydroxybutyric acid is preferably used as a prophylactic or therapeutic agent for ALS.

These isomers can be obtained as single products by a synthesis method, a separation method (e.g., concentration, solvent extraction, column chromatography, recrystallization etc.), an optical resolution method (e.g., fractional recrystallization, chiral column method, diastereomer method etc.) and the like each known per se.

The compound of the present invention may be a crystal, and is included in the compound of the present invention whether it is in a single crystal form or a crystal mixture. The crystal can be produced by crystallizing by applying a crystallization method known per se.

The compound of the present invention may be a solvate (e.g., hydrate etc.) or a non-solvate (e.g., non-hydrate etc.), both of which are encompassed in the compound of the present invention.

In addition, a compound labeled with an isotope (e.g., ³H, ¹⁴C, ³⁵S, ¹²⁵I etc.) is also encompassed in the compound of the present invention.

Even when the compound of the present invention is any of the above-mentioned kinase inhibitors, or prostaglandin analogue, estrogen receptor antagonist, or any of the above-mentioned compounds not belonging to these, it can show efficacy for any ALS. For example, when the compound of the present invention is an Met inhibitor (e.g., Crizotinib), its anti-ALS activity may be higher on ALS having mutation in SOD1 gene.

When the compound of the present invention is an FGFR inhibitor (e.g., Pazopanib), its anti-ALS activity may be higher on ALS having mutation in TDP-43 gene and/or C9orf72 gene.

When the compound of the present invention is Tivozanib, its anti-ALS activity may be higher on ALS having mutation in SOD1 gene and/or TDP-43 gene and sporadic ALS.

When the compound of the present invention is Bosutinib or Sunitinib, its anti-ALS activity may be higher on ALS having mutation in SOD1 gene and/or C9orf72 gene and sporadic ALS.

When the compound of the present invention is Axitinib, its anti-ALS activity may be higher on ALS having mutation in C9orf72 gene.

Thus, since the compound of the present invention has different therapeutic activity spectrum for various familial or sporadic ALS depending on the kind thereof, a prophylactic or therapeutic agent for ALS, which contains a compound having higher anti-ALS activity as an active ingredient, can be appropriately selected and used according to the kind of ALS of the subject in need of the prophylaxis and/or treatment. Alternatively, a prophylactic or therapeutic agent for ALS, which has a high anti-ALS activity against any ALS, can also be provided by combining two or more compounds of the present invention having different therapeutic activity spectra. In this case, two or more compounds of the present invention may be each formulated singly or produced as a combination agent. In the former case, each preparation can be administered to the same subject simultaneously or with time lag.

The prophylactic and/or therapeutic agent for ALS of the present invention can be administered orally or parenterally in the form of the active ingredient the compound of the present invention as it is alone, or as a pharmaceutical composition in an appropriate dosage form blended with a pharmacologically acceptable carrier, excipient, diluent and the like.

As the composition for oral administration, solid or liquid dosage forms, specifically tablets (including sugarcoated tablets and film-coated tablets), pills, granules, powders, capsules (including soft capsules), syrups, emulsions, suspensions and the like can be mentioned. Meanwhile, as examples of the composition for parenteral administration, injections, suppositories and the like are used; the injections may include dosage forms such as intravenous injections, subcutaneous injections, intracutaneous injections, intramuscular injections and drip infusion injections. These preparations are produced by a well-known method using additives, including excipients (e.g., organic excipients like sugar derivatives such as lactose, sucrose, glucose, mannitol, and sorbitol; starch derivatives such as cornstarch, potato starch, α starch, and dextrin; cellulose derivatives such as crystalline cellulose; gum arabic; dextran; and pullulan; and inorganic excipients like silicate derivatives such as light anhydrous silicic acid, synthetic aluminum silicate, calcium silicate, and magnesium metasilicoaluminate; phosphates such as calcium hydrogen phosphate; carbonates such as calcium carbonate; and sulfates such as calcium sulfate), lubricants (e.g., stearic acid, stearic acid metal salts such as calcium stearate and magnesium stearate; talc; colloidal silica; waxes such as beeswax and spermaceti; boric acid; adipic acid; sulfates such as sodium sulfate; glycol; fumaric acid; sodium benzoate; DL leucine; lauryl sulfates such as sodium lauryl sulfate and magnesium lauryl sulfate; silicates such as silicic anhydride and silicic hydrates; and the aforementioned starch derivatives), binders (e.g., hydroxypropylcellulose, hydroxypropylmethylcellulose, polyvinylpyrrolidone, macrogol, and the same compounds as the aforementioned excipients), disintegrants (e.g., cellulose derivatives such as lowsubstitutional hydroxypropylcellulose, carboxymethylcellulose, carboxymethylcellulose calcium, and internally crosslinked carboxymethylcellulose sodium; chemically modified starches and celluloses such as carboxymethylstarch, carboxymethylstarch sodium, and crosslinked polyvinylpyrrolidone), emulsifiers (e.g., colloidal clays such as bentonite and Veegum; metal hydroxides such as magnesium hydroxide and aluminum hydroxide; anionic surfactants such as sodium lauryl sulfate and calcium stearate; cationic surfactants such as benzalkonium chloride; and non-ionic surfactants such as polyoxyethylene alkyl ethers, polyoxyethylene sorbitan fatty acid ester of, and sucrose fatty acid ester), stabilizers (para-oxybenzoic acid esters such as methyl paraben and propyl paraben; alcohols such as chlorobutanol, benzyl alcohol, and phenylethyl alcohol; benzalkonium chloride; phenols such as phenol and cresol; thimerosal; dehydroacetic acid; and sorbic acid), taste/odor correctives (e.g., sweeteners, souring agents, and flavors in common use), and diluents.

The dose of the compound of the present invention as an active ingredient of the prophylactic and/or therapeutic agent for ALS in the present invention is variable according to various conditions such as the kind of compound, the patient's symptoms, age, weight, drug receptivity and the like. For oral administration, at least 0.1 mg (suitably 0.5 mg) to at most 1000 mg (suitably 500 mg) per dose for oral administration, or at least 0.01 mg (suitably 0.05 mg) to at most 100 mg (suitably 50 mg) per dose for parenteral administration, can be administered to an adult 1 to 6 times a day. The dose may be increased or reduced according to the symptoms. Particularly, when the compound of the present invention is already on the market as a pharmaceutical product for a disease other than ALS, an appropriately dose of each compound can be determined within the range confirmed to be safe.

Furthermore, the prophylactic and/or therapeutic agent for ALS of the present invention may be used in combination with other drugs, for example, glutamic acid action suppressants (e.g., riluzole and the like), neurotrophic factors [e.g., insulin-like growth factor-1, 5-HT_{1A} receptor agonists (e.g., xaliproden) and the like] and the like. The prophylactic and/or therapeutic agent for ALS of the present invention and these other drugs can be administered simultaneously, sequentially, or separately.

The present invention is hereinafter described in further detail by means of the following Example, to which, however, the invention is never limited.

### [Example 1]

iPS cells were produced according to Okita K, et al, Nat Methods. 2011, 8:409-12, by introducing SOX2, KLF4, OCT4, L-MYC, LIN28 and small hairpin RNA for p53 into fibroblast isolated from ALS patients (SOD1-L144FVX mutation) by using episomal vectors. The obtained iPS cells were cultured on SNL cells in a primate embryonic stem cell medium (ReproCELL) added with 4 ng/ml basic FGF (Wako Chemicals) and penicillin/streptomycin.

Motoneuron cells established from iPS cells derived from ALS patients related to SOD1 mutation (SOD1-L144FVX mutation) were immunostained, whereby accumulation of misfolded SOD1 (Fig. 1A) and fragility (decrease in the number of surviving cells) derived from mutation of SOD1 was confirmed (Fig. 1B). Immunostaining was performed by preparing samples according to a conventional method, and staining same with βIII tubulin (1:2000, Covance; 1:1000, abcam), HB9 (1:200, Developmental Studies Hybridoma Bank) and misfolded SOD1 (B8H10) (1:100, MEDIMABS). As the surviving cells, the cells stained with βIII tubulin were counted using IN Cell Analyzer 2000 (GE Healthcare), IN Cell Analyzer 6000 and IN CELL Developer toolbox software 1.9 (GE Healthcare). Induction into motoneuron cells was performed by the method described in WO 2014/148646. In brief, 3 transcription factors of LIM homeobox protein 3 (Lhx3), neurogenin 2 (Ngn2) and ISL LIM homeobox 1 (Isl1) were introduced into iPS cells by using piggyBac transposon, and were polycistronically expressed by a tetracycline responsive promoter on a Matrigel-coated culture dish, whereby iPS cells were directly induced into motoneuron cells. At 7 days after addition of doxycycline, induction of motoneuron cell was completed. The cells were cultured in DMEM/F12 (Life Technologies) added with 100 µg/ml apotransferrin (Sigma), 5 µg/ml insulin (Sigma), 30 nM selenite (Sigma), 20 nM progesterone (Sigma), 100 nM putrescine (Sigma), 1 µM RA, 1 µM Shh, 10 ng/ml BDNF, 10 ng/ml GDNF, 10 ng/ml NT-3 and 1 µg/ml doxycycline (Clontech). A known drug, riluzole, was added to the motoneuron cells derived from patients having SOD1-L144FVX mutation (at 7 days after addition of doxycycline), and the cells were cultured for 7 more days. Since the number of surviving cells was high, the motoneuron cell was confirmed to be useful for the evaluation of a therapeutic drug for ALS.

Subsequently, motoneuron cells derived from iPS cells having SOD1-L144FVX mutation, which were induced as mentioned above, were contacted with a medicament for 7 days, and a candidate therapeutic drug was screened for by using cell survival as an index. Screening was performed after calculating the Z factor from a 50 µM Kenpaullone (Tocris) (Cell Stem Cell, 12, 1-14, 2013) administration group having a known effect (positive control) and a DMSO administration group (negative control) (Zhang, J.H., et al., J. Biomol. Screen 4: 67-73, 1999), and confirming that the Z factor was not less than 0.5. The effect was studied with the libraries of 1760 compounds and medicaments considered to be effective for the treatment of ALS having an MN index (motoneuron cell survival number ratio as compared to negative control) of not less than 3SD were detected. The medicaments are shown in the following Tables 4-1 - 4-5. As the compound libraries, Microsource US Drugs (Microsource Discovery Systems), Microsource International Drugs (Microsource Discovery Systems), InhibitorSelect^{™} 96-Well Protein Kinase Inhibitor Library (EMD), InhibitorSelect^{™} 96-Well Protein Kinase Inhibitor Library II (EMD), InhibitorSelect^{™} 96-Well Protein Kinase Inhibitor Library III (EMD), Screen-Well(R) Kinase inhibitor library (ENZO) and Kinase Inhibitor Library (Selleck Chemicals LLC.) were used.

**[Table 4-1]**

| drug | structural formula | MN index | category |
|---|---|---|---|
| Tivozanib | 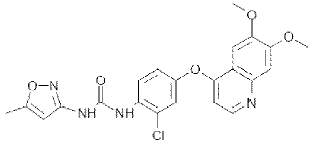 | 17.26 | none |
| Syk Inhibitor | 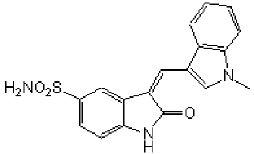 | 16.14 | Syk inhibitor |
| JAK Inhibitor I | 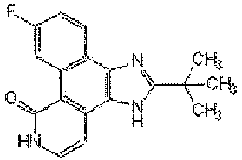 | 12.59 | JAK inhibitor |
| Aurora Kinase Inhibitor II | 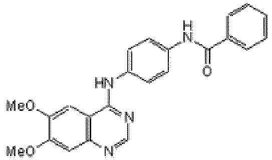 | 11.77 | Aurora kinase inhibitor |
| SB 216763 | 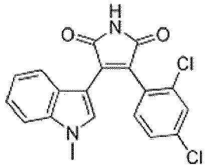 | 11.73 | none |
| Cdk2 Inhibitor II | 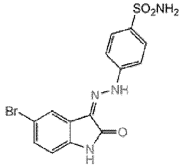 | 10.93 | none |
| ZM-447439 | 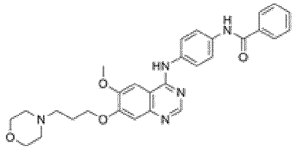 | 10.90 | Aurora kinase inhibitor |
| BUDESONIDE | 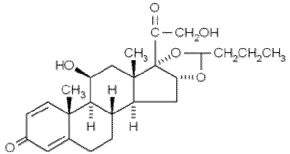 | 10.27 | none |

**[Table 4-2]**

| drug | structural formula | MN index | category |
|---|---|---|---|
| PDGF Receptor Tyrosine Kinase Inhibitor III | 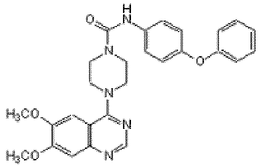 | 10.15 | EGFR inhibitor |
| | | | FGFR inhibitor |
| | | | PKA inhibitor |
| | | | PKC inhibitor |
| U0126-EtOH | 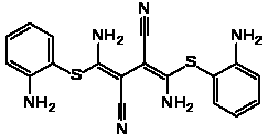 | 10.13 | MEK inhibitor |
| Enzastaurin | 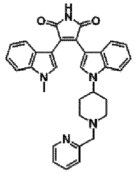 | 9.84 | PKC inhibitor |
| VX-680 | 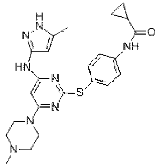 | 9.58 | Aurora kinase inhibitor |
| RIBOFLAVIN | 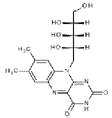 | 9.55 | none |
| Pazopanib Hydrochloride | 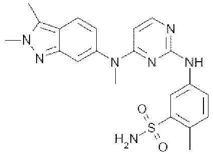 | 9.53 | FGFR inhibitor |
| alpha-TOCHOPHEROL | 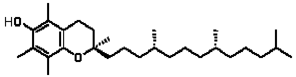 | 9.46 | none |
| AMODIAQUINE DIHYDROCHLORIDE | 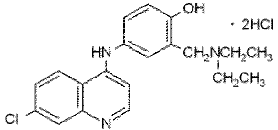 | 8.98 | none |

**[Table 4-3]**

| drug | structural formula | MN index | category |
|---|---|---|---|
| BMS 777607 | 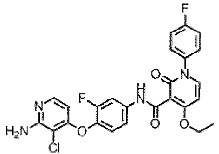 | 8.55 | Met inhibitor |
| SU9516 | 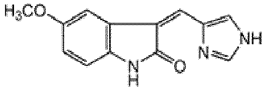 | 8.39 | none |
| SP600125 | 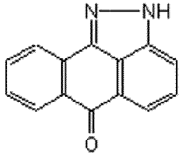 | 8.38 | JNK inhibitor |
| CYC116 | 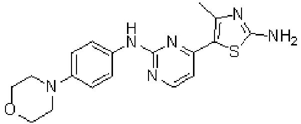 | 8.14 | Aurora kinase inhibitor |
| BPIQ-I | 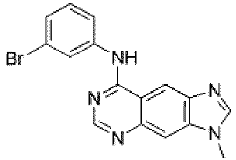 | 8.14 | FGFR inhibitor |
| Sunitinib Malate | 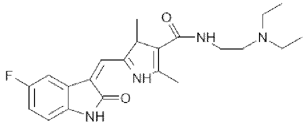 | 8.13 | none |
| GSK-3 Inhibitor XIII | 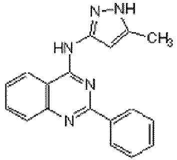 | 8.13 | none |
| Bisindolylmaleimide I | 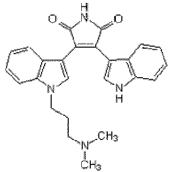 | 7.96 | none |

**[Table 4-4]**

| drug | structural formula | MN index | category |
|---|---|---|---|
| HYDROQUINONE | 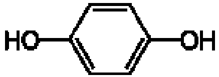 | 7.96 | none |
| FLUNISOLIDE | 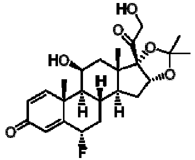 | 7.86 | none |
| KW 2449 | 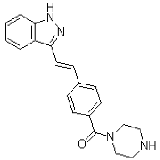 | 7.72 | Aurora kinase inhibitor |
| MGCD-265 | 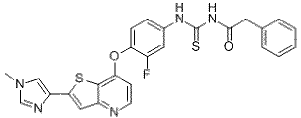 | 7.62 | none |
| Crizotinib (PF-2341066) | 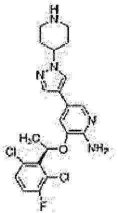 | 7.60 | Met inhibitor |
| Syk Inhibitor II | 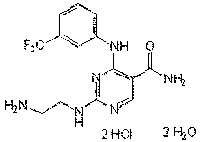 | 7.59 | Syk inhibitor |
| Indirubin-3'-monoxime | 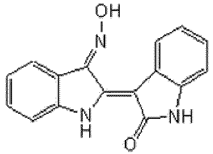 | 7.54 | none |
| HYDRASTINE (1R, 9S) | 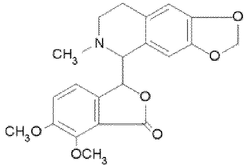 | 7.53 | none |

**[Table 4-5]**

| drug | structural formula | MN index | category |
|---|---|---|---|
| PIPERINE | 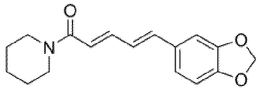 | 7.39 | none |
| BUTAMBEN | 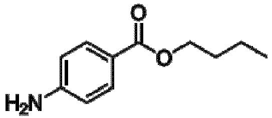 | 7.39 | none |
| Axitinib | 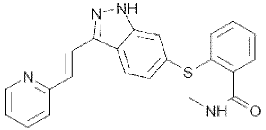 | 7.35 | none |
| APOMORPHINE HYDROCHLORIDE | 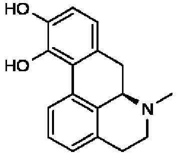 | 7.26 | none |
| FENBUFEN | 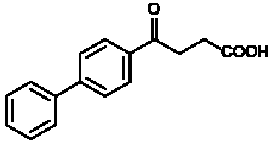 | 7.22 | none |
| Bosutinib (SKI-606) | 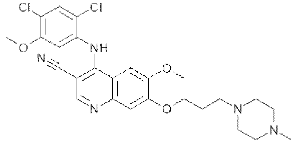 | 7.20 | none |
| Wee1 Inhibitor | 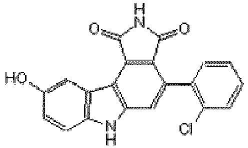 | 7.18 | none |
| Cdk2 Inhibitor IV, NU6140 | 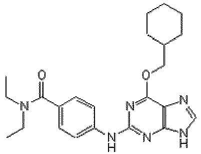 | 7.08 | none |

### [Example 2]

Bosutinib was purchased from Abcam, Sunitinib was purchased from SIGMA, Tivozanib and Crizotinib were purchased from LKT Laboratories, Axitinib was purchased from Selleck Chemicals LLC, and Pazopanib was purchased from AdooQ BioScience and used for this Example.

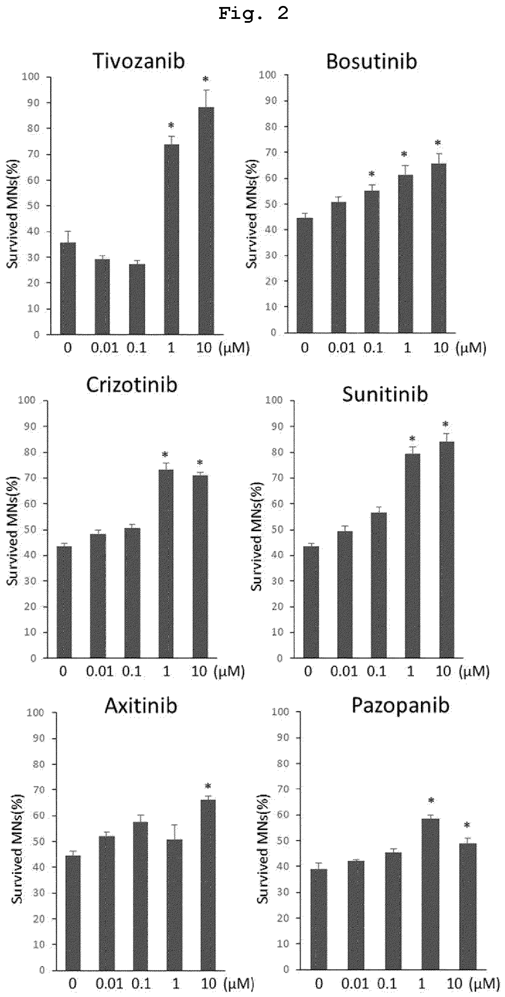

Tivozanib, Bosutinib, Sunitinib, Crizotinib, Axitinib and Pazopanib obtained in Example 1 as candidate drugs for ALS were studied for the dose correlation in the protection effect on motoneuron cell having SOD1 mutation. As a result, all medicaments were confirmed to show an increase in the effect in a dose-dependent manner (Fig. 2).

Tivozanib, Bosutinib and Sunitinib were studied for the effect on nerve cell derived from iPS cell having SOD1-L144FVX mutation. The nerve cells induced by 56 days of culture were subjected to a filter treatment with a 70 µm mesh, transferred to a 96 well plate, 1 µM of Tivozanib, Bosutinib, Sunitinib was added, the cells were cultured for 48 hr, and the length of neuritis was evaluated. As a result, elongation of neuritis was confirmed in the Tivozanib, Bosutinib and Sunitinib treatment groups. For induction of nerve cells, Wada, T. et al PLoS One, 4(8):e6722 2009, Chambers, S. M. et al Nat Biotechnol, 27(3):275-280, 2009 were used after modification. In detail, small clumps (diameter 40 - 100 µm) of iPS cell colonies were subjected to a filter treatment with Cell Strainer (BD Falcon), and cultured for 10 days in N2B27 medium [DMEM/F12, Neurobasal, N2 supplement, B27 supplement, L-Gln] added with 100 ng/ml human recombinant Noggin (R&D systems) and 1 µM SB431542 (Sigma) in a culture dish coated with poly-L-lysine (Sigma)/Laminin (BD Falcon) (PLL/LM). Then, the colonies were dissociated into small clumps with 200 U/ml collagenase added with CaCl₂, and transferred into a culture dish coated with PLL/ECL (Millipore). After 7 days of culture, the cells were dissociated with Accutase (Innovative Cell Technologies), and cultured in a culture dish coated with PLL/ECL for 7 days (24d). Finally, the cells dissociated with Accutase and selected by a 40 µm cell strainer (BD Falcon) were counted, cultured for 31 days (56d) in N2B27 medium added with 10 ng/ml BDNF, GDNF, and NT-3 (R&D systems) in a culture dish coated with PLL/LM/Fibronectin (Millipore) to give nerve cells from the iPS cells.

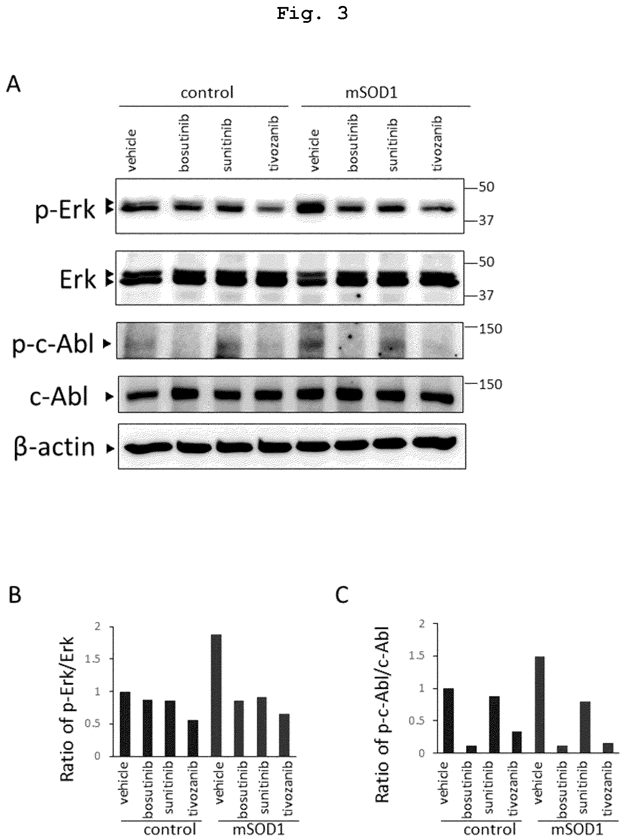

It is known that phosphorylation of Erk is promoted in nerve cells having mutation SOD1. To examine the mechanism of these candidate medicaments, phosphorylation of Erk was examined by Western blot method. The Western blot method included dissolving cells in RIPA buffer containing 0.1% SDS, 150 mM NaCl, 1% NP-40, 0.5% deoxycholate, 50 mM Tris-HCl (pH 8.0), protease inhibitor (Roche) and phosphatase inhibitor (Roche), centrifugation, measurement of protein concentration, addition of 20 µg amount of protein to each lane, and electrophoresis in 10-20% polyacrylamide gel. After blotting to Immobilon-P membrane (Millipore), the cells were stained with antibodies against Erk, phosphorylated Erk, c-Abl and phosphorylated c-Abl and detected. As a result, it was confirmed that phosphorylation of Erk decreased by culturing nerve cells after addition of 1 µM of Tivozanib, Bosutinib, Sunitinib. Similarly, it was confirmed that phosphorylation of c-abl also decreased by the treatment with the medicaments (Fig. 3). These results suggest that the medicaments suppress motoneuron cell death by inhibiting phosphorylation of Erk and c-abl.

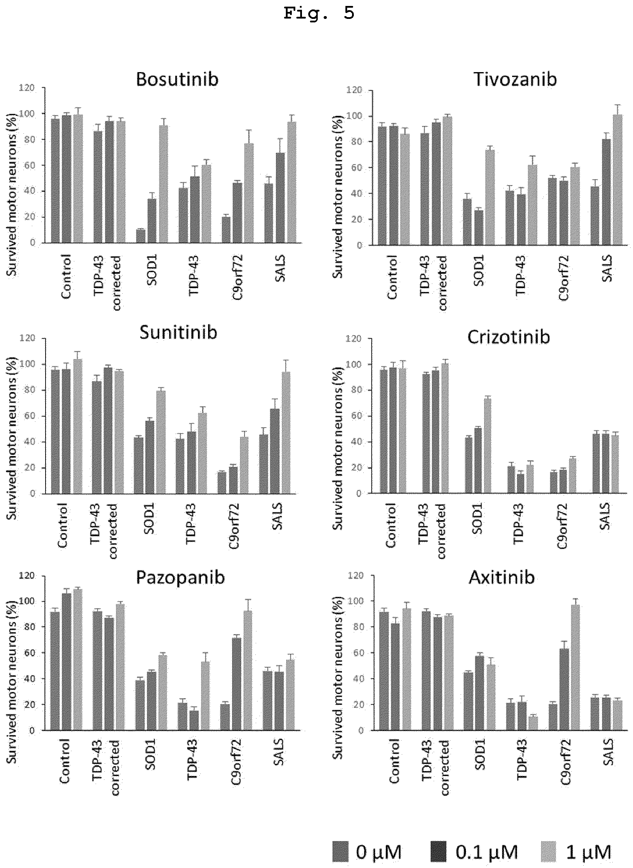

Lastly, the effect of candidate medicaments on the motoneuron cells derived from ALS patients was studied using iPS cells derived from ALS patients having other gene mutation (SOD1, TDP-43 and C9orf72) and iPS cells derived from sporadic ALS patients. The iPS cells were prepared from fibroblast isolated from ALS patients (SOD1-L144FVX mutation, TDP-43-M337V mutation and abnormal elongation of (GGGGCC)n repeat number in gene locus intron 1 of C9orf72) by a method similar to the above-mentioned method. iPS cells were cultured on SNL cells in a primate embryonic stem cell medium (ReproCELL) added with 4 ng/ml basic FGF (Wako Chemicals) and penicillin/streptomycin. Sporadic ALS was acknowledged by post mortem pathological test and exomeanalysis of fibroblast of patients. The number of survived motoneuron cells (on day 14 from the start of induction) induced from iPS cells derived from these ALS patients was counted, whereby it was confirmed that the number survived decreased in any ALS (Fig. 4). On day 7 from the start of induction from iPS cells, Tivozanib, Bosutinib, Sunitinib, Crizotinib, Pazopanib and Axitinib were added and the cells were cultured, and further cultured for 7 days, and the survived motoneuron cells were counted on day 14. It was confirmed that each medicament specifically shows the effect on ALS having particular mutation (Fig. 5). Those having a significant difference in the cell survival rate improving effect are shown in Table 5. In addition, those showing 20% improvement in the cell survival rate are considered to have a marked effect, and the analysis results thereof are shown in Table 6.

**[Table 5]**

| | SOD1 mutation | TDP-43 mutation | C9orf72 mutation | sporadic |
|---|---|---|---|---|
| Tivozanib | ○ | ○ | ○ | ○ |
| Bosutinib | ○ | ○ | ○ | ○ |
| Sunitinib | ○ | ○ | ○ | ○ |
| Crizotinib | ○ | x | ○ | x |
| Pazopanib | ○ | ○ | ○ | x |
| Axitinib | x | x | ○ | x |

| | | | | |
|---|---|---|---|---|
| ○: significant difference (p<0.05) x: no significant difference (p≥0.05) | | | | |

**[Table 6]**

| | SOD1 mutation | TDP-43 mutation | C9orf72 mutation | sporadic |
|---|---|---|---|---|
| Tivozanib | ○ | ○ | x | ○ |
| Bosutinib | ○ | x | ○ | ○ |
| Sunitinib | ○ | x | ○ | ○ |
| Crizotinib | ○ | x | x | x |
| Pazopanib | x | ○ | ○ | x |
| Axitinib | x | x | ○ | x |

| | | | | |
|---|---|---|---|---|
| ○**:** cell survival rate improvement not less than 20% x: cell survival rate improvement less than 20% | | | | |

From the above results, Tivozanib was confirmed to show higher efficacy for ALS having SOD1 mutation, ALS having TDP-43 mutation and sporadic ALS, Bosutinib was confirmed to show higher efficacy for ALS having SOD1 mutation, ALS having C9orf72 mutation and sporadic ALS, Sunitinib was confirmed to show higher efficacy for ALS having SOD1 mutation, ALS having C9orf72 mutation and sporadic ALS, Crizotinib was confirmed to show higher efficacy for ALS having SOD1 mutation, Pazopanib was confirmed to show higher efficacy for ALS having TDP-43 mutation and ALS having C9orf72 mutation, and Axitinib was confirmed to show higher efficacy for ALS having C9orf72 mutation.

### [Example 3]

Bimatoprost was purchased from SIGMA, Edaravone was purchased from TOCRIS, (+-) 3-hydroxybutyric acid was purchased from SIGMA, and Raloxifene was purchased from TOCRIS and they were used for this Example. The structural formulas of these compounds are shown in Table 7.

**[Table 7]**

| **drug** | **structural formula** | **category** |
|---|---|---|
| Bimatoprost | 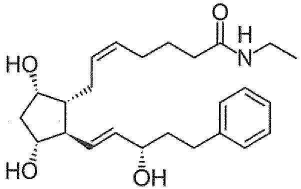 | prostaglandin analogue |
| Edaravone | 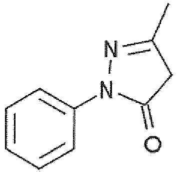 | |
| 3-hydroxy-butyric acid | 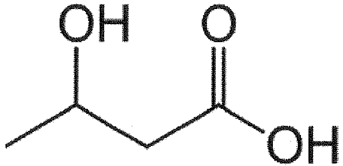 | |
| Raloxifene | 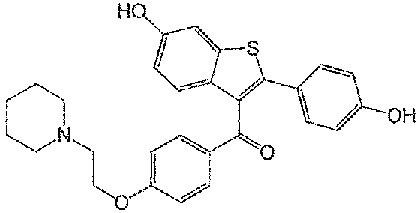 | estrogen receptor antagonist |

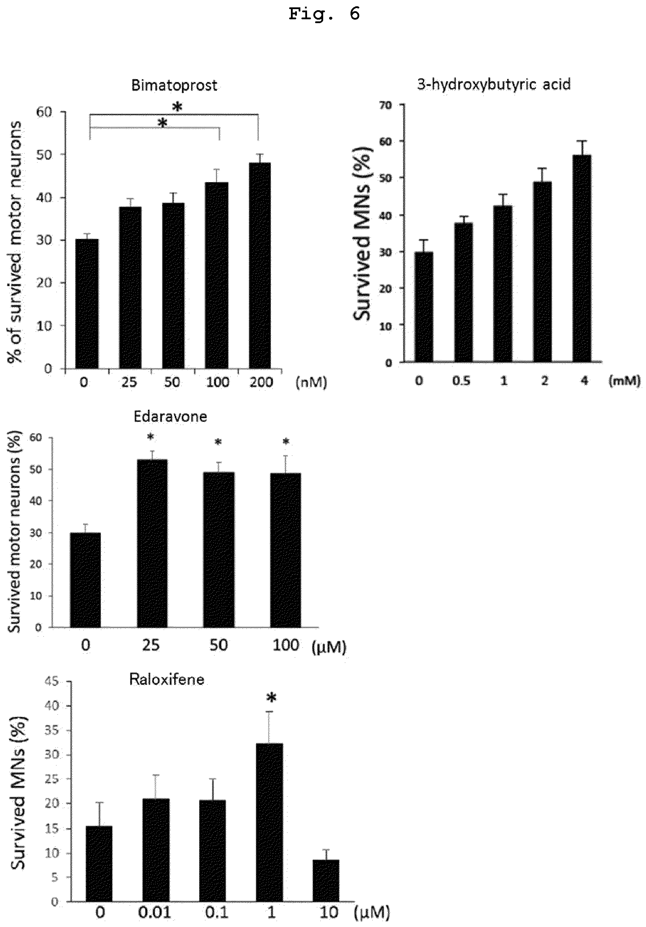

Bimatoprost, Edaravone, 3-hydroxybutyric acid and Raloxifene were studied for the dose correlation in the protection effect on motoneuron cell having the aforementioned SOD1-L144FVX mutation. As a result, all medicaments were confirmed to show an increase in the effect in a dose-dependent manner (Fig. 6). As described in Amyotroph Lateral Scler Frontotemporal Degener. 15:610-617, 2014, Edaravone is shown to be effective for ALS, and it was demonstrated that the medicaments found by screening in this Example are effective for ALS.

### [Example 4]

### Method

### 1. Medicament screening using motor neuron derived from iPS cell

Imatinib, Nilotinib, Rebastinib, AT9283, Bafetinib were purchased from Selleck. Using 0, 0.01, 0.1, 1, 10 µM of the above-mentioned reagents, screening was performed. The structural formulas of these compounds are shown in Table 8.

**[Table 8]**

| **drug** | **structural formula** | **category** |
|---|---|---|
| Imatinib | 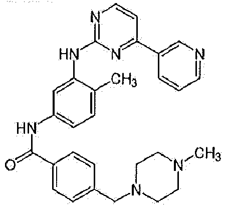 | |
| Nilotinib | 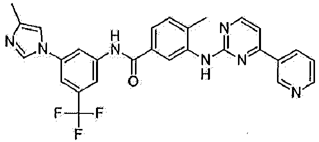 | |
| Rebastinib | 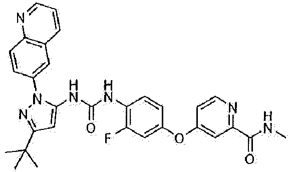 | |
| AT9283 | 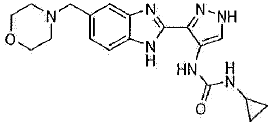 | Aurora kinase inhibitor |
| | | JAK inhibitor |
| Bafetinib | 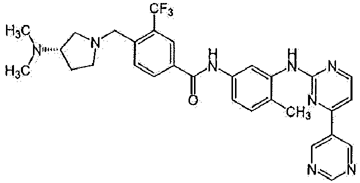 | |

The production method and screening method of motor neuron are similar to those in Example 1.

### 2. Western blotting

Vehicle (DMSO) or 1 µM Bosutinib was added to the motor neuron on day 7 of culture, and the mixture was cultured for 72 hr. PBS washing was performed once, and the cells were recovered with a scraper and dissolved in RIPA buffer (0.1% SDS, 150 mM NaCl, 1% NP-40, 0.5% deoxycholate, 50 mM Tris-HCl (pH 8.0), protease inhibitor (Roche), phosphatase inhibitor (Roche)). After sonication, the cells were centrifuged at 13,000 g for 10 min, and the supernatant was separated. 10 µg of a sample added with sample buffer (free of reducing agent, Nacalai) was electrophoresed on SDS-page, and blotted on Immobilon-P membrane (Millipore). The cells were blocked with 5% skim milk for 1 hr, primary antibody TDP-43 antibody (1:2000, protein tech) was added and the mixture was reacted at 4°C overnight. The secondary antibody Rb IgG-HRP (1:5000, GE healthcare) was reacted for 1 hr, ECL prime (GE healthcare) was added over 5 min, and the cells were detected by LAS4000 (GE healthcare).

### Results

### 1. Screening by familial ALS (mSOD1 ALS) motoneuron cells

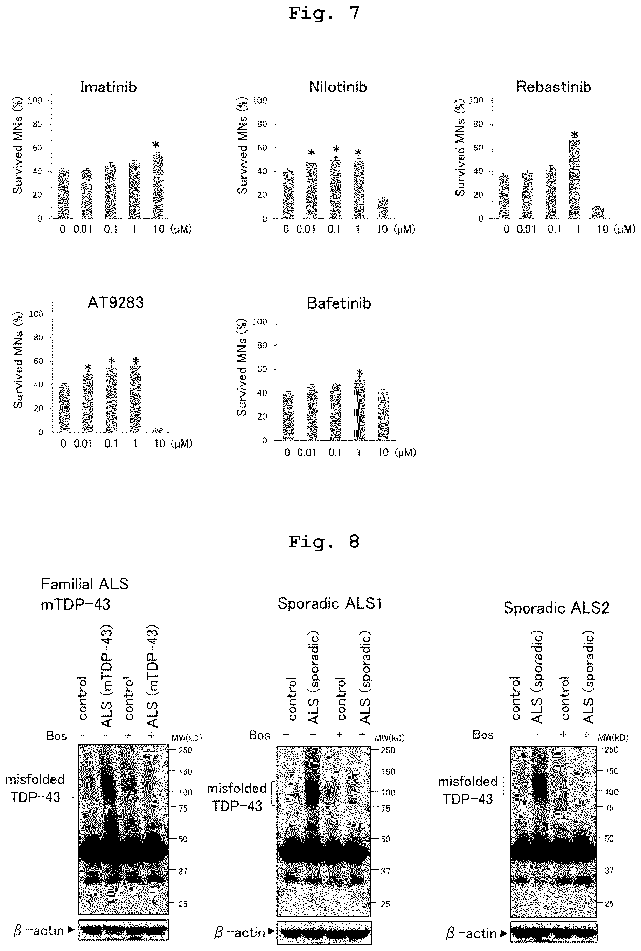

Imatinib, Nilotinib, Rebastinib, AT9283, and Bafetinib increased survival of motor neuron (n=6, one-way ANOVA; p<0.05, post hoc test p<0.05) (Fig. 7).

### 2. Effect of Bosutinib on ALS other than mSOD1 ALS

In familial ALS having TDP-43 mutation and sporadic ALS, accumulation of 40-200 kD misfolded TDP-43 was found.
Misfolded TDP43 decreased by the addition of Bosutinib (Fig. 8).

### [Industrial Applicability]

The compound of the present invention is useful for the prophylaxis and/or treatment of ALS. Particularly, since clinical and nonclinical data of safety and the like of medicaments already on the market as pharmaceutical products for other diseases have been accumulated, and the libraries of surrounding compounds already exist, it is expected that a pharmaceutical product capable of preventing and/or treating ALS can be developed rapidly at a low cost. In addition, an effective prophylaxis and/or treatment of ALS, which is suitable for individual patients or a preliminary group thereof, can be available by properly using the compound of the present invention according to the causative gene of ALS.

All patents, patent specifications and all publications including scientific documents described herein are incorporated in their entireties by reference to the extent that they have been disclosed in the present specification.

This application is based on patent application Nos. 2015-004589 filed in Japan (filing date: January 13, 2015) and 2015-137909 filed in Japan (filing date: July 9, 2015), the contents of which are incorporated in full herein.

### Aspects

1. A prophylactic and/or therapeutic agent for amyotrophic lateral sclerosis (ALS), which comprises one or more kinase inhibitors selected from the group consisting of an epithelial cell growth factor receptor (EGFR) inhibitor, a fibroblast growth factor receptor (FGFR) inhibitor, an Aurorakinase inhibitor, a protein kinase A (PKA) inhibitor, a protein kinase C (PKC) inhibitor, an MEK inhibitor, an Met inhibitor, a JNK inhibitor, a Syk inhibitor and a JAK inhibitor, and/or a prostaglandin analogue and/or an estrogen receptor antagonist.
2. The agent according to aspect 1, wherein the aforementioned EGFR inhibitor is a PDGF Receptor Tyrosine Kinase Inhibitor III or BPIQ-I.
3. The agent according to aspect 1, wherein the aforementioned FGFR inhibitor is Pazopanib or an analog thereof or PDGF Receptor Tyrosine Kinase Inhibitor III.
4. The agent according to aspect 1, wherein the aforementioned Aurorakinase inhibitor is ZM-447439, VX-680, Aurora Kinase Inhibitor II, CYC116, KW2449, or AT9283.
5. The agent according to aspect 1, wherein the aforementioned PKA inhibitor is PDGF Receptor Tyrosine Kinase Inhibitor III.
6. The agent according to aspect 1, wherein the aforementioned PKC inhibitor is PDGF Receptor Tyrosine Kinase inhibitor III or Enzastaurin.
7. The agent according to aspect 1, wherein the aforementioned MEK inhibitor is U0126-EtOH.
8. The agent according to aspect 1, wherein the aforementioned Met inhibitor is MGCD-265, PF-2341066 (Crizotinib) or an analog thereof or BMS 777607.
9. The agent according to aspect 1, wherein the aforementioned JNK inhibitor is SP600125.
10. The agent according to aspect 1, wherein the aforementioned Syk inhibitor is a Syk Inhibitor.
11. The agent according to aspect 1, wherein the aforementioned JAK inhibitor is JAK Inhibitor I or AT9283.
12. The agent according to aspect 1, wherein the aforementioned prostaglandin analogue is Bimatoprost.
13. The agent according to aspect 1, wherein the aforementioned estrogen receptor antagonist is Raloxifene.
14. A prophylactic and/or therapeutic agent for ALS, comprising one or more compounds selected from the group consisting of Tivozanib and an analog thereof, SB 216763, Cdk2 Inhibitor II, BUDESONIDE, RIBOFLAVIN, alpha-TOCHOPHEROL, AMODIAQUINE, SU9516, Sunitinib and an analog thereof, GSK-3 Inhibitor XIII, Bisindolylmaleimide I, HYDROQUINONE, FLUNISOLIDE, MGCD-265, Indirubin-3'-monoxime, HYDRASTINE (1R, 9S), PIPERINE, BUTAMBEN, Axitinib and an analog thereof, APOMORPHINE, FENBUFEN, Bosutinib (SKI-606) and an analog thereof, Wee1 Inhibitor, Cdk2 Inhibitor IV, NU6140, 3-hydroxybutyric acid, Imatinib, Nilotinib, Rebastinib, and Bafetinib.
15. A method for the prophylaxis and/or treatment of ALS, comprising administering an effective amount of one or more kinase inhibitors selected from the group consisting of an EGFR inhibitor, an FGFR inhibitor, an Aurorakinase inhibitor, a PKA inhibitor, a PKC inhibitor, an MEK inhibitor, an Met inhibitor, a JNK inhibitor, a Syk inhibitor and a JAK inhibitor, and/or a prostaglandin analogue and/or an estrogen receptor antagonist.
16. A method for the prophylaxis and/or treatment of ALS, comprising administering an effective amount of one or more kinase inhibitors selected from the group consisting of Tivozanib and an analog thereof, SB 216763, Cdk2 Inhibitor II, BUDESONIDE, RIBOFLAVIN, alpha-TOCHOPHEROL, AMODIAQUINE, SU9516, Sunitinib and an analog thereof, GSK-3 Inhibitor XIII, Bisindolylmaleimide I, HYDROQUINONE, FLUNISOLIDE, MGCD-265, Indirubin-3'-monoxime, HYDRASTINE (1R,9S), PIPERINE, BUTAMBEN, Axitinib and an analog thereof, APOMORPHINE, FENBUFEN, Bosutinib (SKI-606) and an analog thereof, a Wee1 Inhibitor, Cdk2 Inhibitor IV, NU6140, 3-hydroxybutyric acid, Imatinib, Nilotinib, Rebastinib, and Bafetinib.
17. One or more kinase inhibitors selected from the group consisting of an EGFR inhibitor, an FGFR inhibitor, an Aurorakinase inhibitor, a PKA inhibitor, a PKC inhibitor, an MEK inhibitor, an Met inhibitor, a JNK inhibitor, a Syk inhibitor and a JAK inhibitor, and/or a prostaglandin analogue and/or an estrogen receptor for the prophylaxis and/or treatment of ALS.
18. One or more kinase inhibitors selected from the group consisting of Tivozanib and an analog thereof, SB 216763, Cdk2 Inhibitor II, BUDESONIDE, RIBOFLAVIN, alpha-TOCHOPHEROL, AMODIAQUINE, SU9516, Sunitinib and an analog thereof, GSK-3 Inhibitor XIII, Bisindolylmaleimide I, HYDROQUINONE, FLUNISOLIDE, MGCD-265, Indirubin-3'-monoxime, HYDRASTINE (1R,9S), PIPERINE, BUTAMBEN, Axitinib and an analog thereof, APOMORPHINE, FENBUFEN, Bosutinib (SKI-606) and an analog thereof, a Wee1 Inhibitor, Cdk2 Inhibitor IV, NU6140, 3-hydroxybutyric acid, Imatinib, Nilotinib, Rebastinib, and Bafetinib for the prophylaxis and/or treatment of ALS.

## Claims

1. A compound for use in the prophylaxis and/or treatment of amyotrophic lateral sclerosis (ALS):
wherein the compound is bosutinib and the ALS is sporadic ALS, ALS having SOD1 mutation, ALS having TDP-43 mutation and/or ALS having C9orf72 mutation.

2. The compound for use according to claim 1, wherein the ALS is sporadic ALS.

3. The compound for use according to claim 1, wherein the ALS is ALS having SOD1 mutation.

4. The compound for use according to claim 1, wherein the ALS is ALS having TDP-43 mutation.

5. The compound for use according to claim 1, wherein the ALS is ALS having C9orf72 mutation.
